# EUROPEAN PATENT SPECIFICATION

(11) **EP 3 027 656 B1**
(45) Date of publication and mention of the grant of the patent: **26.06.2019**
(21) Application number: 14761423.4
(22) Date of filing: 30.07.2014
(51) Int. Cl.: C07K 16/40, A61P 37/00, A61K 39/395

(54) **MEDICAMENT COMPRISING ANTI-PHOSPHOLIPASE D4 ANTIBODY**
ARZNEIMITTEL MIT ANTI-PHOSPHOLIPASE-D4-ANTIKÖRPER
MÉDICAMENT COMPRENANT UN ANTICORPS ANTI-PHOSPHOLIPASE D4

(30) Priority: 30.07.2013 JP 2013158258
(43) Date of publication of application: 08.06.2016
(73) Proprietor: SBI Biotech Co., Ltd., Minato-ku Tokyo 106-6015 (JP)
(72) Inventor: YAMAZAKI, Tomohide, Tokyo 106-6015 (JP); ENDO, Mayuki, Tokyo 106-6015 (JP); ISHIDA, Koji, Tokyo 106-6015 (JP)
(74) Representative: J A Kemp
(86) International application number: PCT/JP2014/070661
(87) International publication number: WO 2015/016386

(56) References cited:
- WO-A1-00/11015
- WO-A2-02/45570
- WO-A2-2013/115410
- YOSHINORI OTANI ET AL: "PLD4 Is Involved in Phagocytosis of Microglia: Expression and Localization Changes of PLD4 Are Correlated with Activation State of Microglia", PLOS ONE, vol. 6, no. 11, 15 November 2011 (2011-11-15), page e27544, XP055061737, DOI: 10.1371/journal.pone.0027544 & DATABASE UniProt [Online] 16 May 2006 (2006-05-16), "RecName: Full=Phospholipase D4; Short=PLD 4; EC=3.1.4.4; AltName: Full=Choline phosphatase 4; AltName: Full=Phosphatidylcholine-hydrolyzing phospholipase D4;", retrieved from EBI accession no. UNIPROT:Q96BZ4 Database accession no. Q96BZ4
- FUMIO YOSHIKAWA ET AL: "Phospholipase D Family Member 4, a Transmembrane Glycoprotein with No Phospholipase D Activity, Expression in Spleen and Early Postnatal Microglia", PLOS ONE, vol. 5, no. 11, 11 November 2010 (2010-11-11), page e13932, XP055061704, DOI: 10.1371/journal.pone.0013932
- DAVIES J ET AL: "Affinity improvement of single antibody VH domains: residues in all three hypervariable regions affect antigen binding", IMMUNOTECHNOLOGY, ELSEVIER SCIENCE PUBLISHERS BV, NL, vol. 2, no. 3, 1 September 1996 (1996-09-01), pages 169-179, XP004070292, ISSN: 1380-2933, DOI: 10.1016/S1380-2933(96)00045-0
- YUKINORI OKADA ET AL: "Meta-analysis identifies nine new loci associated with rheumatoid arthritis in the Japanese population", NATURE GENETICS, vol. 44, no. 5, 25 March 2012 (2012-03-25) , pages 511-516, XP055153331, ISSN: 1061-4036, DOI: 10.1038/ng.2231
- CHIKASHI TERAO ET AL: "PLD4 as a novel susceptibility gene for systemic sclerosis in a Japanese population", ARTHRITIS & RHEUMATISM, vol. 65, no. 2, 28 February 2013 (2013-02-28), pages 472-480, XP055153327, ISSN: 0004-3591, DOI: 10.1002/art.37777

### [Sequence Listing]

### SEQUENCE LISTING

<110> SBI BIOTECH C0., LTD.
<120> MEDICAMENT COMPRISING ANTI PHOSPHOLIPASE D4 ANTIBODY
<130> W7064-00
<150> JP 2013-158258
   <151> 2013-07-30
<160> 132
<170> PatentIn version 3.4
<210> 1
   <211> 506
   <212> PRT
   <213> Homo sapiens
<220>
   <221> PEPTIDE
   <222> (1).. (506)
<400> 1
   Met Leu Lys Pro Leu Trp Lys Ala Ala Val Ala Pro Thr Trp Pro Cys
   1 5 10 15
   Ser Met Pro Pro Arg Arg Pro Trp Asp Arg Glu Ala Gly Thr Leu Gln
   20 25 30
   Val Leu Gly Ala Leu Ala Val Leu Trp Leu Gly Ser Val Ala Leu Ile
   35 40 45
   Cys Leu Leu Trp Gln Val Pro Arg Pro Pro Thr Trp Gly Gln Val Gln
   50 55 60
   Pro Lys Asp Val Pro Arg Ser Trp Glu His Gly Ser Ser Pro Ala Trp
   65 70 75 80
   Glu Pro Leu Glu Ala Glu Ala Arg Gln Gln Arg Asp Ser Cys Gln Leu
   85 90 95
   Val Leu Val Glu Ser Ile Pro Gln Asp Leu Pro Ser Ala Ala Gly Ser
   100 105 110
   Pro Ser Ala Gln Pro Leu Gly Gln Ala Trp Leu Gln Leu Leu Asp Thr
   115 120 125
   Ala Gln Glu Ser Val His Val Ala Ser Tyr Tyr Trp Ser Leu Thr Gly
   130 135 140
   Pro Asp Ile Gly Val Asn Asp Ser Ser Ser Gln Leu Gly Glu Ala Leu
   145 150 155 160
   Leu Gln Lys Leu Gln Gln Leu Leu Gly Arg Asn Ile Ser Leu Ala Val
   165 170 175
   Ala Thr Ser Ser Pro Thr Leu Ala Arg Thr Ser Thr Asp Leu Gln Val
   180 185 190
   Leu Ala Ala Arg Gly Ala His Val Arg Gln Val Pro Met Gly Arg Leu
   195 200 205
   Thr Arg Gly Val Leu His Ser Lys Phe Trp Val Val Asp Gly Arg His
   210 215 220
   Ile Tyr Met Gly Ser Ala Asn Met Asp Trp Arg Ser Leu Thr Gln Val
   225 230 235 240
   Lys Glu Leu Gly Ala Val Ile Tyr Asn Cys Ser His Leu Ala Gln Asp
   245 250 255
   Leu Glu Lys Thr Phe Gln Thr Tyr Trp Val Leu Gly Val Pro Lys Ala
   260 265 270
   Val Leu Pro Lys Thr Trp Pro Gln Asn Phe Ser Ser His Phe Asn Arg
   275 280 285
   Phe Gln Pro Phe His Gly Leu Phe Asp Gly Val Pro Thr Thr Ala Tyr
   290 295 300
   Phe Ser Ala Ser Pro Pro Ala Leu Cys Pro Gln Gly Arg Thr Arg Asp
   305 310 315 320
   Leu Glu Ala Leu Leu Ala Val Met Gly Ser Ala Gln Glu Phe Ile Tyr
   325 330 335
   Ala Ser Val Met Glu Tyr Phe Pro Thr Thr Arg Phe Ser His Pro Pro
   340 345 350
   Arg Tyr Trp Pro Val Leu Asp Asn Ala Leu Arg Ala Ala Ala Phe Gly
   355 360 365
   Lys Gly Val Arg Val Arg Leu Leu Val Gly Cys Gly Leu Asn Thr Asp
   370 375 380
   Pro Thr Met Phe Pro Tyr Leu Arg Ser Leu Gln Ala Leu Ser Asn Pro
   385 390 395 400
   Ala Ala Asn Val Ser Val Asp Val Lys Val Phe Ile Val Pro Val Gly
   405 410 415
   Asn His Ser Asn Ile Pro Phe Ser Arg Val Asn His Ser Lys Phe Met
   420 425 430
   Val Thr Glu Lys Ala Ala Tyr Ile Gly Thr Ser Asn Trp Ser Glu Asp
   435 440 445
   Tyr Phe Ser Ser Thr Ala Gly Val Gly Leu Val Val Thr Gln Ser Pro
   450 455 460
   Gly Ala Gln Pro Ala Gly Ala Thr Val Gln Glu Gln Leu Arg Gln Leu
   465 470 475 480
   Phe Glu Arg Asp Trp Ser Ser Arg Tyr Ala Val Gly Leu Asp Gly Gln
   485 490 495
   Ala Pro Gly Gln Asp Cys Val Trp Gln Gly
   500 505
<210> 2
   <211> 5
   <212> PRT
   <213> Homo sapiens
<400> 2
   Ser Tyr Trp Met His
   1 5
<210> 3
   <211> 17
   <212> PRT
   <213> Homo sapiens
<400> 3
   Asp Ile Tyr Pro Gly Ser Asp Ser Thr Asn Tyr Asn Glu Lys Phe Lys
   1 5 10 15
   Ser
<210> 4
   <211> 9
   <212> PRT
   <213> Homo sapiens
<400> 4
   Gly Gly Trp Leu Asp Ala Met Asp Tyr
   1 5
<210> 5
   <211> 11
   <212> PRT
   <213> Homo sapiens
<400> 5
   Arg Ala Ser Gln Asp Ile Ser Asn Tyr Leu Asn
   1 5 10
<210> 6
   <211> 7
   <212> PRT
   <213> Homo sapiens
<400> 6
   Tyr Thr Ser Arg Leu His Ser
   1 5
<210> 7
   <211> 8
   <212> PRT
   <213> Homo sapiens
<400> 7
   Gln Gln Gly Asn Thr Leu Pro Trp
   1               5
<210> 8
   <211> 5
   <212> PRT
   <213> Homo sapiens
<400> 8
   Thr Tyr Trp Met His
   1               5
<210> 9
   <211> 17
   <212> PRT
   <213> Homo sapiens
<400> 9
   Ala Ile Tyr Pro Gly Asn Ser Glu Thr Ser Tyr Asn Gln Lys Phe Lys
   1               5                   10                  15
   Gly
<210> 10
   <211> 7
   <212> PRT
   <213> Homo sapiens
<400> 10
   Gly Tyr Ser Asp Phe Asp Tyr
   1               5
<210> 11
   <211> 11
   <212> PRT
   <213> Homo sapiens
<400> 11
   His Ala Ser Gln Gly Ile Arg Ser Asn Ile Gly
   1               5                   10
<210> 12
   <211> 7
   <212> PRT
   <213> Homo sapiens
<400> 12
   His Gly Thr Asn Leu Glu Asp
   1               5
<210> 13
   <211> 7
   <212> PRT
   <213> Homo sapiens
<400> 13
   Val Gln Tyr Val Gln Phe Pro
   1               5
<210> 14
   <211> 5
   <212> PRT
   <213> Homo sapiens
<400> 14
   Asp Tyr Asn Leu His
   1               5
<210> 15
   <211> 17
   <212> PRT
   <213> Homo sapiens
<400> 15
   Tyr Ile Tyr Pro Tyr Asn Gly Asn Thr Gly Tyr Asn Gln Lys Phe Lys
   1               5                   10                  15
   Arg
<210> 16
   <211> 14
   <212> PRT
   <213> Homo sapiens
<400> 16
   Gly Gly Ile Tyr Asp Asp Tyr Tyr Asp Tyr Ala Ile Asp Tyr
   1 5 10
<210> 17
   <211> 11
   <212> PRT
   <213> Homo sapiens
<400> 17
   Arg Ala Ser Glu Asn Ile Tyr Ser His Ile Ala
   1 5 10
<210> 18
   <211> 7
   <212> PRT
   <213> Homo sapiens
<400> 18
   Gly Ala Thr Asn Leu Ala His
   1 5
<210> 19
   <211> 7
   <212> PRT
   <213> Homo sapiens
<400> 19
   Gln His Phe Trp Gly Thr Pro
   1 5
<210> 20
   <211> 5
   <212> PRT
   <213> Homo sapiens
<400> 20
   Ser Tyr Tyr Leu Tyr
   1 5
<210> 21
   <211> 17
   <212> PRT
   <213> Homo sapiens
<400> 21
   Leu Ile Asn Pro Thr Asn Ser Asp Thr Ile Phe Asn Glu Lys Phe Lys
   1               5                   10                  15
   Ser
<210> 22
   <211> 11
   <212> PRT
   <213> Homo sapiens
<400> 22
   Glu Gly Gly Tyr Gly Tyr Gly Pro Phe Ala Tyr
   1               5                   10
<210> 23
   <211> 16
   <212> PRT
   <213> Homo sapiens
<400> 23
   Thr Ser Ser Gln Thr Leu Val His Ser Asn Gly Asn Thr Tyr Leu His
   1               5                   10                  15
<210> 24
   <211> 7
   <212> PRT
   <213> Homo sapiens
<400> 24
   Lys Val Ser Asn Arg Phe Ser
   1               5
<210> 25
   <211> 6
   <212> PRT
   <213> Homo sapiens
<400> 25
   His Ser Thr His Val Pro
   1 5
<210> 26
   <211> 5
   <212> PRT
   <213> Homo sapiens
<400> 26
   Ser Tyr Gly Met Ser
   1 5
<210> 27
   <211> 17
   <212> PRT
   <213> Homo sapiens
<400> 27
   Thr Ile Ser Ser Gly Gly Ser Tyr Ile Tyr Tyr Pro Glu Ser Val Lys
   1 5 10 15
   Gly
<210> 28
   <211> 12
   <212> PRT
   <213> Homo sapiens
<400> 28
   Leu Tyr Gly Gly Arg Arg Gly Tyr Gly Leu Asp Tyr
   1 5 10
<210> 29
   <211> 16
   <212> PRT
   <213> Homo sapiens
<400> 29
   Arg Ser Ser Lys Ser Leu Leu His Ser Asp Gly Ile Thr Tyr Leu Tyr
   1 5 10 15
<210> 30
   <211> 7
   <212> PRT
   <213> Homo sapiens
<400> 30
   Gln Met Ser Asn Leu Ala Ser
   1               5
<210> 31
   <211> 6
   <212> PRT
   <213> Homo sapiens
<400> 31
   Ala Gln Asn Leu Glu Leu
   1               5
<210> 32
   <211> 6
   <212> PRT
   <213> Homo sapiens
<400> 32
   Ser His Tyr Tyr Trp Thr
   1               5
<210> 33
   <211> 16
   <212> PRT
   <213> Homo sapiens
<400> 33
   Tyr Ile Ser Tyr Asp Gly Ser Asn Asn Tyr Asn Pro Ser Leu Lys Asn
   1               5                   10                  15
<210> 34
   <211> 15
   <212> PRT
   <213> Homo sapiens
<400> 34
   Glu Gly Pro Leu Tyr Tyr Gly Asn Pro Tyr Trp Tyr Phe Asp Val
   1 5 10 15
<210> 35
   <211> 11
   <212> PRT
   <213> Homo sapiens
<400> 35
   Arg Ala Ser Gln Asp Ile Asp Asn Tyr Leu Asn
   1 5 10
<210> 36
   <211> 7
   <212> PRT
   <213> Homo sapiens
<400> 36
   Tyr Thr Ser Arg Leu His Ser
   1 5
<210> 37
   <211> 7
   <212> PRT
   <213> Homo sapiens
<400> 37
   Gln Gln Phe Asn Thr Leu Pro
   1 5
<210> 38
   <211> 6
   <212> PRT
   <213> Homo sapiens
<400> 38
   Ser His Tyr Tyr Trp Ser
   1 5
<210> 39
   <211> 16
   <212> PRT
   <213> Homo sapiens
<400> 39
   Tyr Ile Ser Tyr Asp Gly Ser Asn Asn Tyr Asn Pro Ser Leu Lys Asn
   1 5 10 15
<210> 40
   <211> 15
   <212> PRT
   <213> Homo sapiens
<400> 40
   Glu Gly Pro Leu Tyr Tyr Gly Asn Pro Tyr Trp Tyr Phe Asp Val
   1 5 10 15
<210> 41
   <211> 11
   <212> PRT
   <213> Homo sapiens
<400> 41
   Arg Ala Ser Gln Asp Ile Asp Asn Tyr Leu Asn
   1 5 10
<210> 42
   <211> 7
   <212> PRT
   <213> Homo sapiens
<400> 42
   Tyr Thr Ser Arg Leu His Ser
   1 5
<210> 43
   <211> 7
   <212> PRT
   <213> Homo sapiens
<400> 43
   Gln Gln Phe Asn Thr Leu Pro
   1 5
<210> 44
   <211> 1521
   <212> DNA
   <213> Homo sapiens
<400> 44
   atgctgaagc ctctttggaa agcagcagtg gcccccacat ggccatgctc catgccgccc      60
   cgccgcccgt gggacagaga ggctggcacg ttgcaggtcc tgggagcgct ggctgtgctg     120
   tggctgggct ccgtggctct tatctgcctc ctgtggcaag tgccccgtcc tcccacctgg 180
   ggccaggtgc agcccaagga cgtgcccagg tcctgggagc atggctccag cccagcttgg 240
   gagcccctgg aagcagaggc caggcagcag agggactcct gccagcttgt ccttgtggaa 300
   agcatccccc aggacctgcc atctgcagcc ggcagcccct ctgcccagcc tctgggccag 360
   gcctggctgc agctgctgga cactgcccag gagagcgtcc acgtggcttc atactactgg 420
   tccctcacag ggcctgacat cggggtcaac gactcgtctt cccagctggg agaggctctt 480
   ctgcagaagc tgcagcagct gctgggcagg aacatttccc tggctgtggc caccagcagc 540
   ccgacactgg ccaggacatc caccgacctg caggttctgg ctgcccgagg tgcccatgta 600
   cgacaggtgc ccatggggcg gctcaccagg ggtgttttgc actccaaatt ctgggttgtg 660
   gatggacggc acatatacat gggcagtgcc aacatggact ggcggtctct gacgcaggtg 720
   aaggagcttg gcgctgtcat ctataactgc agccacctgg cccaagacct ggagaagacc 780
   ttccagacct actgggtact gggggtgccc aaggctgtcc tccccaaaac ctggcctcag 840
   aacttctcat ctcacttcaa ccgtttccag cccttccacg gcctctttga tggggtgccc 900
   accactgcct acttctcagc gtcgccacca gcactctgtc cccagggccg cacccgggac 960
   ctggaggcgc tgctggcggt gatggggagc gcccaggagt tcatctatgc ctccgtgatg    1020
   gagtatttcc ccaccacgcg cttcagccac cccccgaggt actggccggt gctggacaac    1080
   gcgctgcggg cggcagcctt cggcaagggc gtgcgcgtgc gcctgctggt cggctgcgga 1140
   ctcaacacgg accccaccat gttcccctac ctgcggtccc tgcaggcgct cagcaacccc 1200
   gcggccaacg tctctgtgga cgtgaaagtc ttcatcgtgc cggtggggaa ccattccaac 1260
   atcccattca gcagggtgaa ccacagcaag ttcatggtca cggagaaggc agcctacata 1320
   ggcacctcca actggtcgga ggattacttc agcagcacgg cgggggtggg cttggtggtc 1380
   acccagagcc ctggcgcgca gcccgcgggg gccacggtgc aggagcagct gcggcagctc 1440
   tttgagcggg actggagttc gcgctacgcc gtcggcctgg acggacaggc tccgggccag 1500
   gactgcgttt ggcagggctg a 1521
<210> 45
   <211> 18
   <212> DNA
   <213> Artificial
<220>
   <223> forward primer
<400> 45
   atggactggc ggtctctg 18
<210> 46
   <211> 20
   <212> DNA
   <213> Artificial
<220>
   <223> reverse primer
<400> 46
   tggaaggtct tctccaggtc 20
<210> 47
   <211> 19
   <212> DNA
   <213> Artificial
<220>
   <223> forward primer
<400> 47
   agccacatcg ctcagacac 19
<210> 48
   <211> 19
   <212> DNA
   <213> Artificial
<220>
   <223> reverse primer
<400> 48
   gcccaatacg accaaatcc 19
<210> 49
   <211> 18
   <212> DNA
   <213> Artificial
<220>
   <223> forward primer
<400> 49
   atggactggc ggtctctg 18
<210> 50
   <211> 20
   <212> DNA
   <213> Artificial
<220>
   <223> reverse primer
<400> 50
   tggaaggtct tctccaggtc 20
<210> 51
   <211> 19
   <212> DNA
   <213> Artificial
<220>
   <223> forward primer
<400> 51
   agccacatcg ctcagacac 19
<210> 52
   <211> 19
   <212> DNA
   <213> Artificial
<220>
   <223> reverse primer
<400> 52
   gcccaatacg accaaatcc 19
<210> 53
   <211> 30
   <212> DNA
   <213> Artificial
<220>
   <223> forward primer
<400> 53
   tttgaattcg ccgccaccat gctgaagcct 30
<210> 54
   <211> 34
   <212> DNA
   <213> Artificial
<220>
   <223> reverse primer
<400> 54
   aaagcggccg ctcagccctg ccaaacgcag tcct 34
<210> 55
   <211> 1473
   <212> DNA
   <213> Homo sapiens
<220>
   <221> gene
   <222> (1).. (1473)
<400> 55
   atgaagccta aactgatgta ccaggagctg aaggtgcctg cagaggagcc cgccaatgag 60
   ctgcccatga atgagattga ggcgtggaag gctgcggaaa agaaagcccg ctgggtcctg 120
   ctggtcctca ttctggcggt tgtgggcttc ggagccctga tgactcagct gtttctatgg 180
   gaatacggcg acttgcatct ctttgggccc aaccagcgcc cagccccctg ctatgaccct 240
   tgcgaagcag tgctggtgga aagcattcct gagggcctgg acttccccaa tgcctccacg 300
   gggaaccctt ccaccagcca ggcctggctg ggcctgctcg ccggtgcgca cagcagcctg 360
   gacatcgcct ccttctactg gaccctcacc aacaatgaca cccacacgca ggagccctct 420
   gcccagcagg gtgaggaggt cctccggcag ctgcagaccc tggcaccaaa gggcgtgaac 480
   gtccgcatcg ctgtgagcaa gcccagcggg ccccagccac aggcggacct gcaggctctg 540
   ctgcagagcg gtgcccaggt ccgcatggtg gacatgcaga agctgaccca tggcgtcctg 600
   cataccaagt tctgggtggt ggaccagacc cacttctacc tgggcagtgc caacatggac 660
   tggcgttcac tgacccaggt caaggagctg ggcgtggtca tgtacaactg cagctgcctg 720
   gctcgagacc tgaccaagat ctttgaggcc tactggttcc tgggccaggc aggcagctcc 780
   atcccatcaa cttggccccg gttctatgac acccgctaca accaagagac accaatggag 840
   atctgcctca atggaacccc tgctctggcc tacctggcga gtgcgccccc acccctgtgt 900
   ccaagtggcc gcactccaga cctgaaggct ctactcaacg tggtggacaa tgcccggagt 960
   ttcatctacg tcgctgtcat gaactacctg cccactctgg agttctccca ccctcacagg 1020
   ttctggcctg ccattgacga tgggctgcgg cgggccacct acgagcgtgg cgtcaaggtg 1080
   cgcctgctca tcagctgctg gggacactcg gagccatcca tgcgggcctt cctgctctct 1140
   ctggctgccc tgcgtgacaa ccatacccac tctgacatcc aggtgaaact ctttgtggtc 1200
   cccgcggatg aggcccaggc tcgaatccca tatgcccgtg tcaaccacaa caagtacatg 1260
   gtgactgaac gcgccaccta catcggaacc tccaactggt ctggcaacta cttcacggag 1320
   acggcgggca cctcgctgct ggtgacgcag aatgggaggg gcggcctgcg gagccagctg 1380
   gaggccattt tcctgaggga ctgggactcc ccttacagcc atgaccttga cacctcagct 1440
   gacagcgtgg gcaacgcctg ccgcctgctc tga 1473
<210> 56
   <211> 1338
   <212> DNA
   <213> Homo sapiens
<220>
   <221> gene
   <222> (1).. (1338)
<400> 56
   atgggagagg atgaggatgg actctcagaa aaaaattgcc aaaataaatg tcgaattgcc 60
   ctggtggaaa atattcctga aggccttaac tattcagaaa atgcaccatt tcacttatca 120
   cttttccaag gctggatgaa tttactcaac atggccaaaa agtctgttga catagtgtct 180
   tcccattggg atctcaacca cactcatcca tcagcatgtc agggtcaacg tctttttgaa 240
   aagttgctcc agctgacttc gcaaaatatt gaaatcaagc tagtgagtga tgtaacagct 300
   gattcaaagg tattagaagc cttgaaatta aagggagccg aggtgacgta catgaacatg 360
   accgcttaca acaagggccg gctgcagtcc tccttctgga tcgtggacaa acagcacgtg 420
   tatatcggca gtgccggttt ggactggcaa tccctgggac agatgaaaga actcggtgtc 480
   atcttctaca actgcagctg cctggtccta gatttacaaa ggatatttgc tctatatagt 540
   tcattaaaat tcaaaagcag agtgcctcaa acctggtcca aaagactcta tggagtctat 600
   gacaatgaaa agaaattgca acttcagttg aatgaaacca aatctcaagc atttgtatcg 660
   aattctccaa aactcttttg ccctaaaaac agaagttttg acatagatgc catctacagt 720
   gtgatagatg atgccaagca gtatgtgtac atcgctgtca tggactacct gcctatctcc 780
   agcacaagca ccaaaaggac ttactggcca gacttggatg caaaaataag agaagcatta 840
   gttttacgaa gcgttagagt tcgactcctt ttaagcttct ggaaggaaac tgatcccctt 900
   acgtttaact ttatttcatc tcttaaagcg atttgcactg aaatagccaa ctgcagtttg 960
   aaagttaaat tttttgatct ggaaagagag aatgcttgtg ctacaaaaga acaaaagaat 1020
   cacacctttc ctaggttaaa tcgcaacaag tacatggtga cagatggagc agcttatatt 1080
   ggaaattttg attgggtagg gaatgatttc actcagaatg ctggcacggg ccttgttatc 1140
   aaccaggcag atgtgaggaa caacagaagc atcattaagc aacttaaaga tgtgtttgaa 1200
   agggactggt attcaccgta tgccaaaacc ttacagccaa ccaaacagcc gaactgctca 1260
   agcctgttca aactcaaacc cctctccaac aaaactgcca cagacgacac aggcggaaag 1320
   gatccccgga acgtatga 1338
<210> 57
   <211> 39
   <212> DNA
   <213> Artificial
<220>
   <223> forward primer
<400> 57
   tttaagcttg ccgccaccat gaagcctaaa ctgatgtac 39
<210> 58
   <211> 57
   <212> DNA
   <213> Artificial
<220>
   <223> reverse primer
<400> 58
   tttgaattct cacttatcgt cgtcatcctt gtaatcgagc aggcggcagg cgttgcc 57
<210> 59
   <211> 39
   <212> DNA
   <213> Artificial
<220>
   <223> forward primer
<400> 59
   tttaagcttg ccgccaccat gggagaggat gaggatgga 39
<210> 60
   <211> 57
   <212> DNA
   <213> Artificial
<220>
   <223> reverse primer
<400> 60
   tttgaattct cacttatcgt cgtcatcctt gtaatctacg ttccggggat cctttcc 57
<210> 61
   <211> 26
   <212> DNA
   <213> Artificial
<220>
   <223> forward primer
<400> 61
   agatgctgaa gcctcttcgg agagcg 26
<210> 62
   <211> 25
   <212> DNA
   <213> Artificial
<220>
   <223> reverse primer
<400> 62
   tcagccctgc caaacgcagt cctgg 25
<210> 63
   <211> 1521
   <212> DNA
   <213> Macaca fascicularis
<220>
   <221> gene
   <222> (1).. (1521)
<400> 63
   atgctgaagc ctcttcggag agcggcagtg acccccatgt ggccgtgctc catgctgccc 60
   cgccgcctgt gggacagaga ggctggcacg ttgcaggtcc tgggagtgct ggctatgctg 120
   tggctgggct ccatggctct tacctacctc ctgtggcaag tgcgccgtcc tcccacctgg 180
   ggccaggtgc agcccaagga cgtgcccagg tcctgggggc atggttccag cccagctctg 240
   gagcccctgg aagcggaggt caggaagcag agggactcct gccagcttgt ccttgtggaa 300
   agcatccccc aggacctgcc atttgcagcc ggcagcctct ccgcccagcc tctgggccag 360
   gcctggctgc agctgctgga cactgcccag gagagcgtcc acgtggcttc atactactgg 420
   tccctcacag ggcccgacat tggggtcaac gactcatctt cccagctggg agaggccctt 480
   ctgcagaagc tgcagcagct gctgggcagg aacatttcct tggctgtggc caccagcagt 540
   ccaacactgg ccaggaagtc caccgacctg caggtcctgg ctgcccgagg tgcccaggta 600
   cgacgggtgc ccatggggcg gctcaccagg ggcgttttgc actccaaatt ctgggttgtg 660
   gatggacggc acatatacat gggcagtgcc aacatggact ggcggtccct gacgcaggtg 720
   aaggagcttg gcgctgtcat ctataactgc agccacctgg cccaagacct ggagaagacc 780
   ttccagacct actgggtgct gggggtgccc aaggctgtcc tccccaaaac ctggcctcag 840
   aacttctcat ctcacatcaa ccgtttccag cccttccagg gcctctttga tggggtgccc 900
   accactgcct acttctcagc atcgccaccc gcactctgtc cccagggccg cacccctgac 960
   ctggaggcgc tgttggcggt gatggggagc gcccaggagt tcatctatgc ctccgtgatg 1020
   gagtatttcc ctaccacgcg cttcagccac ccccgcaggt actggccggt gctggacaac 1080
   gcgctgcggg cggcagcctt cagcaagggt gtgcgcgtgc gcctgctggt cagctgcgga 1140
   ctcaacacgg accccaccat gttcccctat ctgcggtccc tgcaggcgct cagcaacccc 1200
   gcggccaacg tctctgtgga cgtgaaagtc ttcatcgtgc cggtggggaa tcattccaac 1260
   atcccgttca gcagggtgaa ccacagcaag ttcatggtca cggagaaggc agcctacata 1320
   ggcacctcca actggtcgga ggattacttc agcagcacga cgggggtggg cctggtggtc 1380
   acccagagcc ccggcgcgca gcccgcgggg gccacggtac aggagcagct gcggcagctc 1440
   tttgagcggg actggagttc gcgctacgcc gtcggcctgg acggacaggc tccgggccag 1500
   gactgcgttt ggcagggctg a 1521
<210> 64
   <211> 36
   <212> DNA
   <213> Artificial
<220>
   <223> primer
<400> 64
   ccaggagagt gggagaggct cttctcagta tggtgg 36
<210> 65
   <211> 32
   <212> DNA
   <213> Artificial
<220>
   <223> primer
<400> 65
   ggctcaggga aatagccctt gaccaggcat cc 32
<210> 66
   <211> 24
   <212> DNA
   <213> Artificial
<220>
   <223> primer
<400> 66
   tccagagttc caggtcactg tcac 24
<210> 67
   <211> 24
   <212> DNA
   <213> Artificial
<220>
   <223> primer
<400> 67
   aggggccagt ggatagacag atgg 24
<210> 68
   <211> 24
   <212> DNA
   <213> Artificial
<220>
   <223> primer
<400> 68
   tccagagttc caagtcacag tcac 24
<210> 69
   <211> 24
   <212> DNA
   <213> Artificial
<220>
   <223> primer
<400> 69
   aggggccagt ggatagactg atgg 24
<210> 70
   <211> 36
   <212> DNA
   <213> Artificial
<220>
   <223> anchor primer
<220>
   <221> misc_feature
   <222> (24).. (25)
   <223> n is deoxyisosine.
<220>
   <221> misc_feature
   <222> (29).. (30)
   <223> n is deoxyisosine.
<220>
   <221> misc_feature
   <222> (34).. (35)
   <223> n is deoxyisosine.
<400> 70
   ggccacgcgt cgactagtac gggnngggnn gggnng 36
<210> 71
   <211> 20
   <212> DNA
   <213> Artificial
<220>
   <223> AUAP primer
<400> 71
   ggccacgcgt cgactagtac 20
<210> 72
   <211> 31
   <212> DNA
   <213> Artificial
<220>
   <223> primer
<400> 72
   cactacttcc tgttgaagct cttgacgatg g 31
<210> 73
   <211> 23
   <212> DNA
   <213> Artificial
<220>
   <223> primer
<400> 73
   gtgagtggcc tcacaggtat agc 23
<210> 74
   <211> 504
   <212> DNA
   <213> Mus musculus
<400> 74
   atgagatcac agttctctat acagttactg agcacacaga acctcacctt gggatggagc 60
   tgtatcatcc tcttcttggt agcaacagct acaggtgtcc actcccaggt ccaactgcag 120
   cagcctgggg ctgaactggt gaagcctggg acttcagtga aaatgtcctg caaggcttct 180
   ggctacacct tcaccagcta ctggatgcac tgggtgaagc agaggccggg acaaggcctt 240
   gagtggattg gagatattta tcctggtagt gatagtacta actacaatga gaagttcaag 300
   agcaaggcca cactgactgt agacacatcc tccagcacag cctacatgca actcagcagc 360
   ctgacatctg aggactctgc ggtctattac tgtgcaagag gagggtggtt ggatgctatg 420
   gactactggg gtcaaggaac ctcagtcacc gtctcctcag ccaaaacaac acccccatca 480
   gtctatccac tggcccctaa gggc 504
<210> 75
   <211> 168
   <212> PRT
   <213> Mus musculus
<400> 75
   Met Arg Ser Gln Phe Ser Ile Gln Leu Leu Ser Thr Gln Asn Leu Thr
   1               5                   10                  15
   Leu Gly Trp Ser Cys Ile Ile Leu Phe Leu Val Ala Thr Ala Thr Gly
               20                  25                  30
   Val His Ser Gln Val Gln Leu Gln Gln Pro Gly Ala Glu Leu Val Lys
           35                  40                  45
   Pro Gly Thr Ser Val Lys Met Ser Cys Lys Ala Ser Gly Tyr Thr Phe
       50                  55                  60
   Thr Ser Tyr Trp Met His Trp Val Lys Gln Arg Pro Gly Gln Gly Leu
   65                  70                  75                  80
   Glu Trp Ile Gly Asp Ile Tyr Pro Gly Ser Asp Ser Thr Asn Tyr Asn
                   85                  90                  95
   Glu Lys Phe Lys Ser Lys Ala Thr Leu Thr Val Asp Thr Ser Ser Ser
               100                 105                 110
   Thr Ala Tyr Met Gln Leu Ser Ser Leu Thr Ser Glu Asp Ser Ala Val
           115                 120                 125
   Tyr Tyr Cys Ala Arg Gly Gly Trp Leu Asp Ala Met Asp Tyr Trp Gly
       130                 135                 140
   Gln Gly Thr Ser Val Thr Val Ser Ser Ala Lys Thr Thr Pro Pro Ser
   145                 150                 155                 160
   Val Tyr Pro Leu Ala Pro Lys Gly
                   165
<210> 76
   <211> 437
   <212> DNA
   <213> Mus musculus
<400> 76
   atggaatgta actggatact tccttttatt ctgtcggtaa tttcaggggt ctcctcagag 60
   gttcagctcc agcagtctgg gactgtgctg tcaaggcctg gggcttccgt gacgatgtcc 120
   tgcaaggctt ctggcgacag ctttaccacc tactggatgc actgggtaaa acagaggcct 180
   ggacagggtc tagaatggat tggtgctatc tatcctggaa atagtgaaac tagctacaac 240
   cagaagttca agggcaaggc caaactgact gcagtcacat ccgccagcac tgcctatatg 300
   gagttcacta gcctgacaaa tgaggactct gcggtctatt actgtacggg gggttattcc 360
   gactttgact actggggcca aggcaccact ctcacagtct cctcagccaa aacgacaccc 420
   ccatctgtct atccact 437
<210> 77
   <211> 145
   <212> PRT
   <213> Mus musculus
<400> 77
   Met Glu Cys Asn Trp Ile Leu Pro Phe Ile Leu Ser Val Ile Ser Gly
   1 5 10 15
   Val Ser Ser Glu Val Gln Leu Gln Gln Ser Gly Thr Val Leu Ser Arg
   20 25 30
   Pro Gly Ala Ser Val Thr Met Ser Cys Lys Ala Ser Gly Asp Ser Phe
   35 40 45
   Thr Thr Tyr Trp Met His Trp Val Lys Gln Arg Pro Gly Gln Gly Leu
   50 55 60
   Glu Trp Ile Gly Ala Ile Tyr Pro Gly Asn Ser Glu Thr Ser Tyr Asn
   65 70 75 80
   Gln Lys Phe Lys Gly Lys Ala Lys Leu Thr Ala Val Thr Ser Ala Ser
   85 90 95
   Thr Ala Tyr Met Glu Phe Thr Ser Leu Thr Asn Glu Asp Ser Ala Val
   100 105 110
   Tyr Tyr Cys Thr Gly Gly Tyr Ser Asp Phe Asp Tyr Trp Gly Gln Gly
   115 120 125
   Thr Thr Leu Thr Val Ser Ser Ala Lys Thr Thr Pro Pro Ser Val Tyr
   130 135 140
   Pro
   145
<210> 78
   <211> 475
   <212> DNA
   <213> Mus musculus
<400> 78
   atgggatgga gctggatctt tctcttcctc ctgtcaggaa ctgcaggcgt ccactctgag 60
   gtccagcttc agcagtcagg acctgaactg gtgaaacctg gggcctcagt gaagatatcc 120
   tgcaaggctt ctggatacac attcactgac tacaacttgc actgggtgaa gcagagccat 180
   ggaaagagcc ttgagtggat tggatatatt tatccttaca atggtaatac tggctacaac 240
   cagaagttca agaggaaggc cacattgact gtagacaatt cctccggcac agtctacatg 300
   gagctccgca gcctgacatc tgaggactct gcagtctatt actgtgcaag aggagggatc 360
   tatgatgatt actacgacta tgctatcgac tattggggtc aaggaacctc agtcaccgtc 420
   tcctcagcca aaacaacacc cccatcagtc tatccactgg cccctaaggg cgaat 475
<210> 79
   <211> 158
   <212> PRT
   <213> Mus musculus
<400> 79
   Met Gly Trp Ser Trp Ile Phe Leu Phe Leu Leu Ser Gly Thr Ala Gly
   1               5                   10                  15
   Val His Ser Glu Val Gln Leu Gln Gln Ser Gly Pro Glu Leu Val Lys
               20                  25                  30
   Pro Gly Ala Ser Val Lys Ile Ser Cys Lys Ala Ser Gly Tyr Thr Phe
           35                  40                  45
   Thr Asp Tyr Asn Leu His Trp Val Lys Gln Ser His Gly Lys Ser Leu
       50                  55                  60
   Glu Trp Ile Gly Tyr Ile Tyr Pro Tyr Asn Gly Asn Thr Gly Tyr Asn
   65                  70                  75                  80
   Gln Lys Phe Lys Arg Lys Ala Thr Leu Thr Val Asp Asn Ser Ser Gly
                   85                  90                  95
   Thr Val Tyr Met Glu Leu Arg Ser Leu Thr Ser Glu Asp Ser Ala Val
               100                 105                 110
   Tyr Tyr Cys Ala Arg Gly Gly Ile Tyr Asp Asp Tyr Tyr Asp Tyr Ala
           115                 120                 125
   Ile Asp Tyr Trp Gly Gln Gly Thr Ser Val Thr Val Ser Ser Ala Lys
       130                 135                 140
   Thr Thr Pro Pro Ser Val Tyr Pro Leu Ala Pro Lys Gly Glu
   145                 150                 155
<210> 80
   <211> 470
   <212> DNA
   <213> Mus musculus
<400> 80
   atgggatgga gctggatctt tctcttcctc ctgtcaggaa ctgcaggcgt ccactctgag 60
   gtccagcttc agcagtcagg acctgaactg gtgaaacctg gggcctcagt gaagatatcc 120
   tgcaaggctt ctggatacac attcactgac tacaacttgc actgggtgaa gcagagccat 180
   ggaaagagcc ttgagtggat tggatatatt tatccttaca atggtaatac tggctacaac 240
   cagaagttca agaggaaggc cacattgact gtagacaatt cctccggcac agtctacatg 300
   gagctccgca gcctgacatc tgaggactct gcagtctatt actgtgcaag aggagggatc 360
   tatgatgatt actacgacta tgctatcgac tattggggtc aaggaacctc agtcaccgtc 420
   tcctcagcca aaacaacacc cccatcagtc tatccactgg cccctaaggg 470
<210> 81
   <211> 156
   <212> PRT
   <213> Mus musculus
<400> 81
   Met Gly Trp Ser Trp Ile Phe Leu Phe Leu Leu Ser Gly Thr Ala Gly
   1 5 10 15
   Val His Ser Glu Val Gln Leu Gln Gln Ser Gly Pro Glu Leu Val Lys
   20 25 30
   Pro Gly Ala Ser Val Lys Ile Ser Cys Lys Ala Ser Gly Tyr Thr Phe
   35 40 45
   Thr Asp Tyr Asn Leu His Trp Val Lys Gln Ser His Gly Lys Ser Leu
   50 55 60
   Glu Trp Ile Gly Tyr Ile Tyr Pro Tyr Asn Gly Asn Thr Gly Tyr Asn
   65 70 75 80
   Gln Lys Phe Lys Arg Lys Ala Thr Leu Thr Val Asp Asn Ser Ser Gly
   85 90 95
   Thr Val Tyr Met Glu Leu Arg Ser Leu Thr Ser Glu Asp Ser Ala Val
   100 105 110
   Tyr Tyr Cys Ala Arg Gly Gly Ile Tyr Asp Asp Tyr Tyr Asp Tyr Ala
   115 120 125
   Ile Asp Tyr Trp Gly Gln Gly Thr Ser Val Thr Val Ser Ser Ala Lys
   130 135 140
   Thr Thr Pro Pro Ser Val Tyr Pro Leu Ala Pro Lys
   145 150 155
<210> 82
   <211> 462
   <212> DNA
   <213> Mus musculus
<400> 82
   atgggatgga gctatatcat cctctttttg gtagcaacag caacaggggt ccactcccag 60
   gtccaactgc agcagtcggg ggctgaactg gtgaagcctg gggcttcagt gaagttgtcc 120
   tgcaaggctt ctggctacac cttcaccagc tactatttgt actgggtgag gcagaggcct 180
   ggacaaggcc ttgagtggat tggactgatt aatcctacca atagtgatac tatcttcaat 240
   gagaagttca agagcaaggc cacactgact gtagacaaat cctccagcac agcatacatg 300
   caactcagca gcctgacatc tgaggactct gcggtctatt actgtacacg agagggggga 360
   tatggttacg gcccgtttgc ttactggggc caagggactc tggtcactgt ctctgcagcc 420
   aaaacaacac ccccatcagt ctatccactg gcccctaagg gc 462
<210> 83
   <211> 154
   <212> PRT
   <213> Mus musculus
<400> 83
   Met Gly Trp Ser Tyr Ile Ile Leu Phe Leu Val Ala Thr Ala Thr Gly
   1 5 10 15
   Val His Ser Gln Val Gln Leu Gln Gln Ser Gly Ala Glu Leu Val Lys
   20 25 30
   Pro Gly Ala Ser Val Lys Leu Ser Cys Lys Ala Ser Gly Tyr Thr Phe
   35 40 45
   Thr Ser Tyr Tyr Leu Tyr Trp Val Arg Gln Arg Pro Gly Gln Gly Leu
   50 55 60
   Glu Trp Ile Gly Leu Ile Asn Pro Thr Asn Ser Asp Thr Ile Phe Asn
   65 70 75 80
   Glu Lys Phe Lys Ser Lys Ala Thr Leu Thr Val Asp Lys Ser Ser Ser
   85 90 95
   Thr Ala Tyr Met Gln Leu Ser Ser Leu Thr Ser Glu Asp Ser Ala Val
   100 105 110
   Tyr Tyr Cys Thr Arg Glu Gly Gly Tyr Gly Tyr Gly Pro Phe Ala Tyr
   115 120 125
   Trp Gly Gln Gly Thr Leu Val Thr Val Ser Ala Ala Lys Thr Thr Pro
   130 135 140
   Pro Ser Val Tyr Pro Leu Ala Pro Lys Gly
   145 150
<210> 84
   <211> 450
   <212> DNA
   <213> Mus musculus
<400> 84
   atgaacttcg ggctcagctt gattttcctt gccctcattt taaaaggtgt ccagtgtgag 60
   gtgcagctgg tggagtctgg gggagactta gtgaggcctg gagggtccct gaaactctcc 120
   tgtgcagcct ctggattcag tttcagtagc tatggcatgt cttggtttcg ccagactcca 180
   gacaagaggc tggagtgggt cgcaaccatt agtagtggtg gtagttacat ctactatcca 240
   gaaagtgtga aggggcgatt caccatctcc agagacaatg ccaggaacat cctgtacctg 300
   caaatgagca gtctgaagtc tgaggacaca gccatgtatt attgtgtaag actctacggt 360
   ggtaggagag gctatggttt ggactactgg ggtcaaggaa cctcagtcac cgtctcctca 420
   gccaaaacaa cagccccatc ggtctatcca 450
<210> 85
   <211> 150
   <212> PRT
   <213> Mus musculus
<400> 85
   Met Asn Phe Gly Leu Ser Leu Ile Phe Leu Ala Leu Ile Leu Lys Gly
   1               5                   10                  15
   Val Gln Cys Glu Val Gln Leu Val Glu Ser Gly Gly Asp Leu Val Arg
               20                  25                  30
   Pro Gly Gly Ser Leu Lys Leu Ser Cys Ala Ala Ser Gly Phe Ser Phe
           35                  40                  45
   Ser Ser Tyr Gly Met Ser Trp Phe Arg Gln Thr Pro Asp Lys Arg Leu
       50                  55                  60
   Glu Trp Val Ala Thr Ile Ser Ser Gly Gly Ser Tyr Ile Tyr Tyr Pro
   65                  70                  75                  80
   Glu Ser Val Lys Gly Arg Phe Thr Ile Ser Arg Asp Asn Ala Arg Asn
                   85                  90                  95
   Ile Leu Tyr Leu Gln Met Ser Ser Leu Lys Ser Glu Asp Thr Ala Met
               100                 105                 110
   Tyr Tyr Cys Val Arg Leu Tyr Gly Gly Arg Arg Gly Tyr Gly Leu Asp
           115                 120                 125
   Tyr Trp Gly Gln Gly Thr Ser Val Thr Val Ser Ser Ala Lys Thr Thr
       130                 135                 140
   Ala Pro Ser Val Tyr Pro
   145                 150
<210> 86
   <211> 450
   <212> DNA
   <213> Mus musculus
<400> 86
   atgaacttcg ggctcagctt gattttcctt gccctcattt taaaaggtgt ccagtgtgag 60
   gtgcagctgg tggagtctgg gggagactta gtgaggcctg gagggtccct gaaactctcc 120
   tgtgcagcct ctggattcag tttcagtagc tatggcatgt cttggtttcg ccagactcca 180
   gacaagaggc tggagtgggt cgcaaccatt agtagtggtg gtagttacat ctactatcca 240
   gaaagtgtga aggggcgatt caccatctcc agagacaatg ccaggaacat cctgtacctg 300
   caaatgagca gtctgaagtc tgaggacaca gccatgtatt attgtgtaag actctacggt 360
   ggtaggagag gctatggttt ggactactgg ggtcaaggaa cctcagtcac cgtctcctca 420
   gccaaaacaa cacccccatc agtctatcca 450
<210> 87
   <211> 150
   <212> PRT
   <213> Mus musculus
<400> 87
   Met Asn Phe Gly Leu Ser Leu Ile Phe Leu Ala Leu Ile Leu Lys Gly
   1 5 10 15
   Val Gln Cys Glu Val Gln Leu Val Glu Ser Gly Gly Asp Leu Val Arg
   20 25 30
   Pro Gly Gly Ser Leu Lys Leu Ser Cys Ala Ala Ser Gly Phe Ser Phe
   35 40 45
   Ser Ser Tyr Gly Met Ser Trp Phe Arg Gln Thr Pro Asp Lys Arg Leu
   50 55 60
   Glu Trp Val Ala Thr Ile Ser Ser Gly Gly Ser Tyr Ile Tyr Tyr Pro
   65 70 75 80
   Glu Ser Val Lys Gly Arg Phe Thr Ile Ser Arg Asp Asn Ala Arg Asn
   85 90 95
   Ile Leu Tyr Leu Gln Met Ser Ser Leu Lys Ser Glu Asp Thr Ala Met
   100 105 110
   Tyr Tyr Cys Val Arg Leu Tyr Gly Gly Arg Arg Gly Tyr Gly Leu Asp
   115 120 125
   Tyr Trp Gly Gln Gly Thr Ser Val Thr Val Ser Ser Ala Lys Thr Thr
   130 135 140
   Pro Pro Ser Val Tyr Pro
   145 150
<210> 88
   <211> 472
   <212> DNA
   <213> Mus musculus
<400> 88
   atgaaagtgt tgagtctgtt gtacctgttg acagccattc ctggtatcct gtctgatgta 60
   cagcttcagg agtcaggacc tggcctcgtg aaaccttctc aatctctgtc tctcacctgc 120
   tctgtcactg gctactccat caccagtcat tattactgga cctggatccg gcagtttcca 180
   ggaaacaaac tggaatggat gggctacata agctacgacg gtagcaataa ctacaaccca 240
   tctctcaaaa atcgaatctc catcactcgt gacacatcta agaaccagtt tttcctgaag 300
   ttgaattctg tgactactga ggacacagct acatataact gtgcaagaga gggcccgctc 360
   tactatggta acccctactg gtatttcgat gtctggggcg cagggaccac ggtcaccgtc 420
   tcctcagcca aaacaacacc cccatcagtc tatccactgg cccctaaggg cg 472
<210> 89
   <211> 157
   <212> PRT
   <213> Mus musculus
<400> 89
   Met Lys Val Leu Ser Leu Leu Tyr Leu Leu Thr Ala Ile Pro Gly Ile
   1               5                   10                  15
   Leu Ser Asp Val Gln Leu Gln Glu Ser Gly Pro Gly Leu Val Lys Pro
               20                  25                  30
   Ser Gln Ser Leu Ser Leu Thr Cys Ser Val Thr Gly Tyr Ser Ile Thr
           35                  40                  45
   Ser His Tyr Tyr Trp Thr Trp Ile Arg Gln Phe Pro Gly Asn Lys Leu
       50                  55                  60
   Glu Trp Met Gly Tyr Ile Ser Tyr Asp Gly Ser Asn Asn Tyr Asn Pro
   65                  70                  75                  80
   Ser Leu Lys Asn Arg Ile Ser Ile Thr Arg Asp Thr Ser Lys Asn Gln
                   85                  90                  95
   Phe Phe Leu Lys Leu Asn Ser Val Thr Thr Glu Asp Thr Ala Thr Tyr
               100                 105                 110
   Asn Cys Ala Arg Glu Gly Pro Leu Tyr Tyr Gly Asn Pro Tyr Trp Tyr
           115                 120                 125
   Phe Asp Val Trp Gly Ala Gly Thr Thr Val Thr Val Ser Ser Ala Lys
       130                 135                 140
   Thr Thr Pro Pro Ser Val Tyr Pro Leu Ala Pro Lys Gly
   145                 150                 155
<210> 90
   <211> 471
   <212> DNA
   <213> Mus musculus
<400> 90
   atgaaagtgt tgagtctgtt gtacctgttg acagccattc ctggtatcct gtctgatgta 60
   cagcttcagg agtcaggacc tggcctcgtg aaaccttctc agtctctgtc tctcacctgc 120
   tctgtcactg gctactccat ctccagtcat tattactgga gttggatccg gcagtttcca 180
   ggaaacagac tggaatggat gggctacata agctacgacg gtagcaataa ctacaaccca 240
   tctctcaaaa atcgaatctc catcactcgt gacacatcta agaaccagtt tttcctgaag 300
   ttgaattctg tgactactga ggacacagct acatataact gtgcaagaga gggcccgctc 360
   tactatggta acccctactg gtatttcgat gtctggggcg cagggaccac ggtcaccgtc 420
   tcctcagcca aaacaacacc cccatcagtc tatccactgg cccctaaggg c 471
<210> 91
   <211> 157
   <212> PRT
   <213> Mus musculus
<400> 91
   Met Lys Val Leu Ser Leu Leu Tyr Leu Leu Thr Ala Ile Pro Gly Ile
   1               5                   10                  15
   Leu Ser Asp Val Gln Leu Gln Glu Ser Gly Pro Gly Leu Val Lys Pro
               20                  25                  30
   Ser Gln Ser Leu Ser Leu Thr Cys Ser Val Thr Gly Tyr Ser Ile Ser
           35                  40                  45
   Ser His Tyr Tyr Trp Ser Trp Ile Arg Gln Phe Pro Gly Asn Arg Leu
       50                  55                  60
   Glu Trp Met Gly Tyr Ile Ser Tyr Asp Gly Ser Asn Asn Tyr Asn Pro
   65                  70                  75                  80
   Ser Leu Lys Asn Arg Ile Ser Ile Thr Arg Asp Thr Ser Lys Asn Gln
                   85                  90                  95
   Phe Phe Leu Lys Leu Asn Ser Val Thr Thr Glu Asp Thr Ala Thr Tyr
               100                 105                 110
   Asn Cys Ala Arg Glu Gly Pro Leu Tyr Tyr Gly Asn Pro Tyr Trp Tyr
           115                 120                 125
   Phe Asp Val Trp Gly Ala Gly Thr Thr Val Thr Val Ser Ser Ala Lys
       130                 135                 140
   Thr Thr Pro Pro Ser Val Tyr Pro Leu Ala Pro Lys Gly
   145                 150                 155
<210> 92
   <211> 470
   <212> DNA
   <213> Mus musculus
<400> 92
   atgaaagtgt tgagtctgtt gtacctgttg acagccattc ctggtatcct gtctgatgta 60
   cagcttcagg agtcaggacc tggcctcgtg aaaccttctc agtctctgtc tctcacctgc 120
   tctgtcactg gctactccat ctccagtcat tattactgga gttggatccg gcagtttcca 180
   ggaaacagac tggaatggat gggctacata agctacgacg gtagcaataa ctacaaccca     240
   tctctcaaaa atcgaatctc catcactcgt gacacatcta agaaccagtt tttcctgaag     300
   ttgaattctg tgactactga ggacacagct acatataact gtgcaagaga gggcccgctc     360
   tactatggta acccctactg gtatttcgat gtctggggcg cagggaccac ggtcaccgtc     420
   tcctcagcca aaacgacacc cccatctgtc tatccactgg cccctaaggg               470
<210> 93
   <211> 156
   <212> PRT
   <213> Mus musculus
<400> 93
   Met Lys Val Leu Ser Leu Leu Tyr Leu Leu Thr Ala Ile Pro Gly Ile
   1               5                   10                  15
   Leu Ser Asp Val Gln Leu Gln Glu Ser Gly Pro Gly Leu Val Lys Pro
               20                  25                  30
   Ser Gln Ser Leu Ser Leu Thr Cys Ser Val Thr Gly Tyr Ser Ile Ser
           35                  40                  45
   Ser His Tyr Tyr Trp Ser Trp Ile Arg Gln Phe Pro Gly Asn Arg Leu
       50                  55                  60
   Glu Trp Met Gly Tyr Ile Ser Tyr Asp Gly Ser Asn Asn Tyr Asn Pro
   65                  70                  75                  80
   Ser Leu Lys Asn Arg Ile Ser Ile Thr Arg Asp Thr Ser Lys Asn Gln
                   85                  90                  95
   Phe Phe Leu Lys Leu Asn Ser Val Thr Thr Glu Asp Thr Ala Thr Tyr
               100                 105                 110
   Asn Cys Ala Arg Glu Gly Pro Leu Tyr Tyr Gly Asn Pro Tyr Trp Tyr
           115                 120                 125
   Phe Asp Val Trp Gly Ala Gly Thr Thr Val Thr Val Ser Ser Ala Lys
       130                 135                 140
   Thr Thr Pro Pro Ser Val Tyr Pro Leu Ala Pro Lys
   145                 150                 155
<210> 94
   <211> 421
   <212> DNA
   <213> Mus musculus
<400> 94
   atgatgtcct ctgctcagtt ccttggtctc ctgttgctct gttttcaagg taccagatgt      60
   gatatccaga tgacacagac tacatcctcc ctgtctgcct ctctgggaga cagagtcacc     120
   atcagttgca gggcaagtca ggacattagc aattatttaa actggtatca gcagaaacca     180
   gatggaactg ttaaactcct gatctactac acatcaagat tacactcagg agtcccatca     240
   aggttcagtg gcagtgggtc tggaacagat tattctctca ccattagcaa cctggagcaa     300
   gaagatattg ccacttactt ttgccaacag ggtaatacgc ttccgtggac gttcggtgga     360
   ggcaccaagc tggaaatcaa acgggctgat gctgcaccaa ctgtatccat caagggcgaa     420
   t                                                                     421
<210> 95
   <211> 140
   <212> PRT
   <213> Mus musculus
<400> 95
   Met Met Ser Ser Ala Gln Phe Leu Gly Leu Leu Leu Leu Cys Phe Gln
   1               5                   10                  15
   Gly Thr Arg Cys Asp Ile Gln Met Thr Gln Thr Thr Ser Ser Leu Ser
               20                  25                  30
   Ala Ser Leu Gly Asp Arg Val Thr Ile Ser Cys Arg Ala Ser Gln Asp
           35                  40                  45
   Ile Ser Asn Tyr Leu Asn Trp Tyr Gln Gln Lys Pro Asp Gly Thr Val
       50                  55                  60
   Lys Leu Leu Ile Tyr Tyr Thr Ser Arg Leu His Ser Gly Val Pro Ser
   65                  70                  75                  80
   Arg Phe Ser Gly Ser Gly Ser Gly Thr Asp Tyr Ser Leu Thr Ile Ser
                   85                  90                  95
   Asn Leu Glu Gln Glu Asp Ile Ala Thr Tyr Phe Cys Gln Gln Gly Asn
               100                 105                 110
   Thr Leu Pro Trp Thr Phe Gly Gly Gly Thr Lys Leu Glu Ile Lys Arg
   115 120 125
   Ala Asp Ala Ala Pro Thr Val Ser Ile Lys Gly Glu
   130 135 140
<210> 96
   <211> 459
   <212> DNA
   <213> Mus musculus
<400> 96
   atgatggtcc ttgctcagtt tcttgcattc ttgttgcttt ggtttccagg tgcaggatgt 60
   gacatcctga tgacccaatc tccatcctcc atgtctgtat ctctgggaga cacagtcagc 120
   atcacttgcc atgcaagtca gggcattaga agtaatatag ggtggttgca gcagaaacca 180
   gggaaatcat ttaagggcct gatctttcat ggaaccaact tggaagatgg agttccatca 240
   aggttcagtg gcagaggatc tggagcagat tattctctca ccatcaacag cctggaatct 300
   gaagattttg cagactatta ctgtgtacag tatgttcagt ttcctccaac gttcggctcg 360
   gggacaaagt tggaaataag acgggctgat gctgcaccaa ctgtatccat cttcccacca 420
   tccagtgagc agttaacatc tggaggtgcc tcagtcgtg 459
<210> 97
   <211> 153
   <212> PRT
   <213> Mus musculus
<400> 97
   Met Met Val Leu Ala Gln Phe Leu Ala Phe Leu Leu Leu Trp Phe Pro
   1               5                   10                  15
   Gly Ala Gly Cys Asp Ile Leu Met Thr Gln Ser Pro Ser Ser Met Ser
               20                  25                  30
   Val Ser Leu Gly Asp Thr Val Ser Ile Thr Cys His Ala Ser Gln Gly
           35                  40                  45
   Ile Arg Ser Asn Ile Gly Trp Leu Gln Gln Lys Pro Gly Lys Ser Phe
       50                  55                  60
   Lys Gly Leu Ile Phe His Gly Thr Asn Leu Glu Asp Gly Val Pro Ser
   65                  70                  75                  80
   Arg Phe Ser Gly Arg Gly Ser Gly Ala Asp Tyr Ser Leu Thr Ile Asn
                   85                  90                  95
   Ser Leu Glu Ser Glu Asp Phe Ala Asp Tyr Tyr Cys Val Gln Tyr Val
               100                 105                 110
   Gln Phe Pro Pro Thr Phe Gly Ser Gly Thr Lys Leu Glu Ile Arg Arg
           115                 120                 125
   Ala Asp Ala Ala Pro Thr Val Ser Ile Phe Pro Pro Ser Ser Glu Gln
       130                 135                 140
   Leu Thr Ser Gly Gly Ala Ser Val Val
   145                 150
<210> 98
   <211> 467
   <212> DNA
   <213> Mus musculus
<400> 98
   atgagtgtgc ccactcaggt cctggggttg ctgctgctgt ggcttacaga tgccagatgt    60
   gacatccaga tgactcagtc tccagcctcc ctatctgtat ctgtgggaga aactgtcgcc   120
   atcacatgtc gagcaagtga gaatatttac agtcatatag catggtatca gcagaaagag   180
   ggaaaatctc ctcagcgcct ggtctatggt gcaacaaact tagcacatgg tgtgccatca   240
   aggttcagtg gcagtggatc aggcacacag tattccctca agatcaacag ccttcagtct   300
   gaagattttg ggagttatta ctgtcaacat ttttggggta ctccgtggac gttcggtgga   360
   ggcaccaagc tggaaatcaa acgggctgat gctgcaccaa ctgtatccat cttcccacca   420
   tccagtgagc agttaacatc tggaggtgcc tcagtcgtgt gcttctt                 467
<210> 99
   <211> 155
   <212> PRT
   <213> Mus musculus
<400> 99
   Met Ser Val Pro Thr Gln Val Leu Gly Leu Leu Leu Leu Trp Leu Thr
   1               5                   10                  15
   Asp Ala Arg Cys Asp Ile Gln Met Thr Gln Ser Pro Ala Ser Leu Ser
               20                  25                  30
   Val Ser Val Gly Glu Thr Val Ala Ile Thr Cys Arg Ala Ser Glu Asn
   35 40 45
   Ile Tyr Ser His Ile Ala Trp Tyr Gln Gln Lys Glu Gly Lys Ser Pro
   50 55 60
   Gln Arg Leu Val Tyr Gly Ala Thr Asn Leu Ala His Gly Val Pro Ser
   65 70 75 80
   Arg Phe Ser Gly Ser Gly Ser Gly Thr Gln Tyr Ser Leu Lys Ile Asn
   85 90 95
   Ser Leu Gln Ser Glu Asp Phe Gly Ser Tyr Tyr Cys Gln His Phe Trp
   100 105 110
   Gly Thr Pro Trp Thr Phe Gly Gly Gly Thr Lys Leu Glu Ile Lys Arg
   115 120 125
   Ala Asp Ala Ala Pro Thr Val Ser Ile Phe Pro Pro Ser Ser Glu Gln
   130 135 140
   Leu Thr Ser Gly Gly Ala Ser Val Val Cys Phe
   145 150 155
<210> 100
   <211> 454
   <212> DNA
   <213> Mus musculus
<400> 100
   atgagtgtgc ccactcaggt cctggggttg ctgctgctgt ggcttacaga tgccagatgt 60
   gacatccaga tgactcagtc tccagcctcc ctatctgtat ctgtgggaga aactgtcgcc 120
   atcacatgtc gagcaagtga gaatatttac agtcatatag catggtatca gcagaaagag 180
   ggaaaatctc ctcagcgcct ggtctatggt gcaacaaact tagcacatgg tgtgccatca 240
   aggttcagtg gcagtggatc aggcacacag tattccctca agatcaacag ccttcagtct 300
   gaagattttg ggagttatta ctgtcaacat ttttggggta ctccgtggac gttcggtgga 360
   ggcaccaagc tggaaatcaa acgggctgat gctgcaccaa ctgtatccat cttcccacca 420
   tccagtgagc agttaacatc tggaggtgcc tcag 454
<210> 101
   <211> 151
   <212> PRT
   <213> Mus musculus
<400> 101
   Met Ser Val Pro Thr Gln Val Leu Gly Leu Leu Leu Leu Trp Leu Thr
   1               5                   10                  15
   Asp Ala Arg Cys Asp Ile Gln Met Thr Gln Ser Pro Ala Ser Leu Ser
               20                  25                  30
   Val Ser Val Gly Glu Thr Val Ala Ile Thr Cys Arg Ala Ser Glu Asn
           35                  40                  45
   Ile Tyr Ser His Ile Ala Trp Tyr Gln Gln Lys Glu Gly Lys Ser Pro
       50                  55                  60
   Gln Arg Leu Val Tyr Gly Ala Thr Asn Leu Ala His Gly Val Pro Ser
   65                  70                  75                  80
   Arg Phe Ser Gly Ser Gly Ser Gly Thr Gln Tyr Ser Leu Lys Ile Asn
                   85                  90                  95
   Ser Leu Gln Ser Glu Asp Phe Gly Ser Tyr Tyr Cys Gln His Phe Trp
               100                 105                 110
   Gly Thr Pro Trp Thr Phe Gly Gly Gly Thr Lys Leu Glu Ile Lys Arg
           115                 120                 125
   Ala Asp Ala Ala Pro Thr Val Ser Ile Phe Pro Pro Ser Ser Glu Gln
       130                 135                 140
   Leu Thr Ser Gly Gly Ala Ser
   145                 150
<210> 102
   <211> 457
   <212> DNA
   <213> Mus musculus
<400> 102
   atgaagttgc ctgttaggct gttggtgctg atgttctgga ttcctgcttc cagcagtgat 60
   gttgtgatga cccaaactcc actctccctg cctgtcagtc ttggagatca agcctccatc 120
   tcttgcacat ctagtcagac ccttgtacac agtaatggaa acacctattt acattggtac 180
   ctgcagaagc caggccagtc tccaaagctc ctgatctaca aagtttccaa ccgattttct 240
   ggggtcccag acaggttcag tggcagtgga tcagggacag atttcacact caagatcagc 300
   agagtggagg ctgaggatct gggagtttat ttctgctctc acagtacaca tgttccattc 360
   acgttcggct cggggacaaa gttggaaata aaacgggctg atgctgcacc aactgtatcc 420
   atcttcccac catccagtga gcagttaaca tctggag 457
<210> 103
   <211> 152
   <212> PRT
   <213> Mus musculus
<400> 103
   Met Lys Leu Pro Val Arg Leu Leu Val Leu Met Phe Trp Ile Pro Ala
   1               5                   10                  15
   Ser Ser Ser Asp Val Val Met Thr Gln Thr Pro Leu Ser Leu Pro Val
               20                  25                  30
   Ser Leu Gly Asp Gln Ala Ser Ile Ser Cys Thr Ser Ser Gln Thr Leu
           35                  40                  45
   Val His Ser Asn Gly Asn Thr Tyr Leu His Trp Tyr Leu Gln Lys Pro
       50                  55                  60
   Gly Gln Ser Pro Lys Leu Leu Ile Tyr Lys Val Ser Asn Arg Phe Ser
   65                  70                  75                  80
   Gly Val Pro Asp Arg Phe Ser Gly Ser Gly Ser Gly Thr Asp Phe Thr
                   85                  90                  95
   Leu Lys Ile Ser Arg Val Glu Ala Glu Asp Leu Gly Val Tyr Phe Cys
               100                 105                 110
   Ser His Ser Thr His Val Pro Phe Thr Phe Gly Ser Gly Thr Lys Leu
           115                 120                 125
   Glu Ile Lys Arg Ala Asp Ala Ala Pro Thr Val Ser Ile Phe Pro Pro
       130                 135                 140
   Ser Ser Glu Gln Leu Thr Ser Gly
   145                 150
<210> 104
   <211> 423
   <212> DNA
   <213> Mus musculus
<400> 104
   atgaggttct ctgctcagct tctggggctg cttgtgctct ggatccctgg atccactgcg 60
   gaaattgtga tgacgcaggc tgcattctcc aatccagtca ctcttggaac atcagcttcc 120
   atctcctgca ggtctagtaa gagtctccta catagtgatg gcatcactta tttgtattgg 180
   tatctgcaga agccaggcca gtctcctcag ctcctgattt atcagatgtc caaccttgcc 240
   tcaggagtcc cagacaggtt cagtagcagt gggtcaggaa ctgatttcac actgagaatc 300
   agcagagtgg aggctgagga tgtgggtgtt tattactgtg ctcaaaatct agaactttac 360
   acgttcggag gggggaccaa gctggaaata aaacgggctg atgctgcacc aactgtatcc 420
   atc 423
<210> 105
   <211> 141
   <212> PRT
   <213> Mus musculus
<400> 105
   Met Arg Phe Ser Ala Gln Leu Leu Gly Leu Leu Val Leu Trp Ile Pro
   1               5                   10                  15
   Gly Ser Thr Ala Glu Ile Val Met Thr Gln Ala Ala Phe Ser Asn Pro
               20                  25                  30
   Val Thr Leu Gly Thr Ser Ala Ser Ile Ser Cys Arg Ser Ser Lys Ser
           35                  40                  45
   Leu Leu His Ser Asp Gly Ile Thr Tyr Leu Tyr Trp Tyr Leu Gln Lys
       50                  55                  60
   Pro Gly Gln Ser Pro Gln Leu Leu Ile Tyr Gln Met Ser Asn Leu Ala
   65                  70                  75                  80
   Ser Gly Val Pro Asp Arg Phe Ser Ser Ser Gly Ser Gly Thr Asp Phe
                   85                  90                  95
   Thr Leu Arg Ile Ser Arg Val Glu Ala Glu Asp Val Gly Val Tyr Tyr
               100                 105                 110
   Cys Ala Gln Asn Leu Glu Leu Tyr Thr Phe Gly Gly Gly Thr Lys Leu
           115                 120                 125
   Glu Ile Lys Arg Ala Asp Ala Ala Pro Thr Val Ser Ile
       130                 135                 140
<210> 106
   <211> 423
   <212> DNA
   <213> Mus musculus
<400> 106
   atgaggttct ctgctcagct tctggggctg cttgtgctct ggatccctgg atccactgcg 60
   gaaattgtga tgacgcaggc tgcattctcc aatccagtca ctcttggaac atcagcttcc 120
   atctcctgca ggtctagtaa gagtctccta catagtgatg gcatcactta tttgtattgg 180
   tatctgcaga agccaggcca gtctcctcag ctcctgattt atcagatgtc caaccttgcc 240
   tcaggagtcc cagacaggtt cagtagcagt gggtcaggaa ctgatttcac actgagaatc 300
   agcagagtgg aggctgagga tgtgggtgtt tattactgtg ctcaaaatct agaactttac 360
   acgttcggag gggggaccaa gctggaaata aaacgggctg atgctgcacc aactgtatcc 420
   atc 423
<210> 107
   <211> 141
   <212> PRT
   <213> Mus musculus
<400> 107
   Met Arg Phe Ser Ala Gln Leu Leu Gly Leu Leu Val Leu Trp Ile Pro
   1               5                   10                  15
   Gly Ser Thr Ala Glu Ile Val Met Thr Gln Ala Ala Phe Ser Asn Pro
               20                  25                  30
   Val Thr Leu Gly Thr Ser Ala Ser Ile Ser Cys Arg Ser Ser Lys Ser
           35                  40                  45
   Leu Leu His Ser Asp Gly Ile Thr Tyr Leu Tyr Trp Tyr Leu Gln Lys
       50                  55                  60
   Pro Gly Gln Ser Pro Gln Leu Leu Ile Tyr Gln Met Ser Asn Leu Ala
   65                  70                  75                  80
   Ser Gly Val Pro Asp Arg Phe Ser Ser Ser Gly Ser Gly Thr Asp Phe
                   85                  90                  95
   Thr Leu Arg Ile Ser Arg Val Glu Ala Glu Asp Val Gly Val Tyr Tyr
   100 105 110
   Cys Ala Gln Asn Leu Glu Leu Tyr Thr Phe Gly Gly Gly Thr Lys Leu
   115 120 125
   Glu Ile Lys Arg Ala Asp Ala Ala Pro Thr Val Ser Ile
   130 135 140
<210> 108
   <211> 404
   <212> DNA
   <213> Mus musculus
<400> 108
   atgatgtcct ctgctcagtt ccttggtctc ctgttgctct gttttcaagg taccagatgt 60
   gatatccaga tgacacagac tacatcctcc ctgtctgcct ctctggggga cagagtcacc 120
   atcagttgca gggcaagtca ggacattgac aattatttaa actggtatca gcagaaacca 180
   gatggaactg ttaaactcct gatctactac acatcaagat tacactcagg agtcccatca 240
   aggttcagtg gcagtgggtc tggaacagat tattctctca ccattagcaa cctggagcaa 300
   gaagatgttg ccacttactt ttgccagcag tttaatacgc ttcctcggac gttcggtgga 360
   ggcaccaaac tggaaatcaa acgggctgat gctgcaccaa ctgt 404
<210> 109
   <211> 134
   <212> PRT
   <213> Mus musculus
<400> 109
   Met Met Ser Ser Ala Gln Phe Leu Gly Leu Leu Leu Leu Cys Phe Gln
   1               5                   10                  15
   Gly Thr Arg Cys Asp Ile Gln Met Thr Gln Thr Thr Ser Ser Leu Ser
               20                  25                  30
   Ala Ser Leu Gly Asp Arg Val Thr Ile Ser Cys Arg Ala Ser Gln Asp
           35                  40                  45
   Ile Asp Asn Tyr Leu Asn Trp Tyr Gln Gln Lys Pro Asp Gly Thr Val
       50                  55                  60
   Lys Leu Leu Ile Tyr Tyr Thr Ser Arg Leu His Ser Gly Val Pro Ser
   65                  70                  75                  80
   Arg Phe Ser Gly Ser Gly Ser Gly Thr Asp Tyr Ser Leu Thr Ile Ser
                   85                  90                  95
   Asn Leu Glu Gln Glu Asp Val Ala Thr Tyr Phe Cys Gln Gln Phe Asn
               100                 105                 110
   Thr Leu Pro Arg Thr Phe Gly Gly Gly Thr Lys Leu Glu Ile Lys Arg
           115                 120                 125
   Ala Asp Ala Ala Pro Thr
       130
<210> 110
   <211> 414
   <212> DNA
   <213> Mus musculus
<400> 110
   atgatgtcct ctgctcagtt ccttggtctc ctgttgctct gttttcaagg taccagatgt 60
   gatatccaga tgacacagac tacatcctcc ctgtctgcct ctctgggggg cagcgtcacc 120
   atcagttgca gggcaagtca ggacattgac aattatttaa actggtatca gcaaaaacca 180
   gatggaactg ttaaactcct gatctactac acatcaagat tacactcagg agtcccatca 240
   aggttcagtg gcagtgggtc tggaacagat tattctctca ccattagcaa cctggaacaa 300
   gaagatattg ccacttactt ttgccaacag tttaatacgc ttcctcggac gttcggtgga 360
   ggcaccaagc tggaaatcaa acgggctgat gctgcaccaa ctgtatccat cttc 414
<210> 111
   <211> 138
   <212> PRT
   <213> Mus musculus
<400> 111
   Met Met Ser Ser Ala Gln Phe Leu Gly Leu Leu Leu Leu Cys Phe Gln
   1 5 10 15
   Gly Thr Arg Cys Asp Ile Gln Met Thr Gln Thr Thr Ser Ser Leu Ser
   20 25 30
   Ala Ser Leu Gly Gly Ser Val Thr Ile Ser Cys Arg Ala Ser Gln Asp
   35 40 45
   Ile Asp Asn Tyr Leu Asn Trp Tyr Gln Gln Lys Pro Asp Gly Thr Val
   50 55 60
   Lys Leu Leu Ile Tyr Tyr Thr Ser Arg Leu His Ser Gly Val Pro Ser
   65 70 75 80
   Arg Phe Ser Gly Ser Gly Ser Gly Thr Asp Tyr Ser Leu Thr Ile Ser
   85 90 95
   Asn Leu Glu Gln Glu Asp Ile Ala Thr Tyr Phe Cys Gln Gln Phe Asn
   100 105 110
   Thr Leu Pro Arg Thr Phe Gly Gly Gly Thr Lys Leu Glu Ile Lys Arg
   115 120 125
   Ala Asp Ala Ala Pro Thr Val Ser Ile Phe
   130 135
<210> 112
   <211> 465
   <212> DNA
   <213> Mus musculus
<400> 112
   atgatgtcct ctgctcagtt ccttggtctc ctgttgctct gttttcaagg taccagatgt 60
   gatatccaga tgacacagac tacatcctcc ctgtctgcct ctctgggggg cagcgtcacc 120
   atcagttgca gggcaagtca ggacattgac aattatttaa actggtatca gcaaaaacca 180
   gatggaactg ttaaactcct gatctactac acatcaagat tacactcagg agtcccatca 240
   aggttcagtg gcagtgggtc tggaacagat tattctctca ccattagcaa cctggaacaa 300
   gaagatattg ccacttactt ttgccaacag tttaatacgc ttcctcggac gttcggtgga 360
   ggcaccaagc tggaaatcaa acgggctgat gctgcaccaa ctgtatccat cttcccacca 420
   tccagtgagc agttaacatc tggaggtgcc tcagtcgtgt gcttc 465
<210> 113
   <211> 155
   <212> PRT
   <213> Mus musculus
<400> 113
   Met Met Ser Ser Ala Gln Phe Leu Gly Leu Leu Leu Leu Cys Phe Gln
   1               5                   10                  15
   Gly Thr Arg Cys Asp Ile Gln Met Thr Gln Thr Thr Ser Ser Leu Ser
               20                  25                  30
   Ala Ser Leu Gly Gly Ser Val Thr Ile Ser Cys Arg Ala Ser Gln Asp
           35                  40                  45
   Ile Asp Asn Tyr Leu Asn Trp Tyr Gln Gln Lys Pro Asp Gly Thr Val
       50                  55                  60
   Lys Leu Leu Ile Tyr Tyr Thr Ser Arg Leu His Ser Gly Val Pro Ser
   65                  70                  75                  80
   Arg Phe Ser Gly Ser Gly Ser Gly Thr Asp Tyr Ser Leu Thr Ile Ser
                   85                  90                  95
   Asn Leu Glu Gln Glu Asp Ile Ala Thr Tyr Phe Cys Gln Gln Phe Asn
               100                 105                 110
   Thr Leu Pro Arg Thr Phe Gly Gly Gly Thr Lys Leu Glu Ile Lys Arg
           115                 120                 125
   Ala Asp Ala Ala Pro Thr Val Ser Ile Phe Pro Pro Ser Ser Glu Gln
       130                 135                 140
   Leu Thr Ser Gly Gly Ala Ser Val Val Cys Phe
   145                 150                 155
<210> 114
   <211> 93
   <212> DNA
   <213> Artificial
<220>
   <223> primer
<400> 114
   accaagcttg ccgccaccat gaaagtgttg agtctgttgt acctgttgac agccattcct 60
   ggtatcctgt ctcaggtcca actgcagcag cct 93
<210> 115
   <211> 42
   <212> DNA
   <213> Artificial
<220>
   <223> primer
<400> 115
   cgatgggccc ttggtgctag ctgaggagac ggtgactgag gt 42
<210> 116
   <211> 39
   <212> DNA
   <213> Artificial
<220>
   <223> primer
<400> 116
   accaagcttg ccgccaccat gatgtcctct gctcagttc 39
<210> 117
   <211> 33
   <212> DNA
   <213> Artificial
<220>
   <223> primer
<400> 117
   agccacagtt cgtttgattt ccagcttggt gcc 33
<210> 118
   <211> 33
   <212> DNA
   <213> Artificial
<220>
   <223> primer
<400> 118
   ctggaaatca aacgaactgt ggctgcacca tct 33
<210> 119
   <211> 30
   <212> DNA
   <213> Artificial
<220>
   <223> primer
<400> 119
   aaagaattcc tagcactctc ccctgttgaa 30
<210> 120
   <211> 1401
   <212> DNA
   <213> Chimaera sp.
<400> 120
   atgaaagtgt tgagtctgtt gtacctgttg acagccattc ctggtatcct gtctcaggtc 60
   caactgcagc agcctggggc tgaactggtg aagcctggga cttcagtgaa aatgtcctgc 120
   aaggcttctg gctacacctt caccagctac tggatgcact gggtgaagca gaggccggga 180
   caaggccttg agtggattgg agatatttat cctggtagtg atagtactaa ctacaatgag 240
   aagttcaaga gcaaggccac actgactgta gacacatcct ccagcacagc ctacatgcaa 300
   ctcagcagcc tgacatctga ggactctgcg gtctattact gtgcaagagg agggtggttg 360
   gatgctatgg actactgggg tcaaggaacc tcagtcaccg tctcctcagc tagcaccaag 420
   ggcccatcgg tcttccccct ggcaccctcc tccaagagca cctctggggg cacagcggcc 480
   ctgggctgcc tggtcaagga ctacttcccc gaaccggtga cggtgtcgtg gaactcaggc 540
   gccctgacca gcggcgtgca caccttcccg gctgtcctac agtcctcagg actctactcc 600
   ctcagcagcg tggtgaccgt gccctccagc agcttgggca cccagaccta catctgcaac 660
   gtgaatcaca agcccagcaa caccaaggtg gacaagaaag ttgagcccaa atcttgtgac 720
   aaaactcaca catgcccacc gtgcccagca cctgaactcc tggggggacc gtcagtcttc 780
   ctcttccccc caaaacccaa ggacaccctc atgatctccc ggacccctga ggtcacatgc 840
   gtggtggtgg acgtgagcca cgaagaccct gaggtcaagt tcaactggta cgtggacggc 900
   gtggaggtgc ataatgccaa gacaaagccg cgggaggagc agtacaacag cacgtaccgt 960
   gtggtcagcg tcctcaccgt cctgcaccag gactggctga atggcaagga gtacaagtgc 1020
   aaggtctcca acaaagccct cccagccccc atcgagaaaa ccatctccaa agccaaaggg 1080
   cagccccgag aaccacaggt gtacaccctg cccccatccc gggatgagct gaccaagaac 1140
   caggtcagcc tgacctgcct ggtcaaaggc ttctatccca gcgacatcgc cgtggagtgg 1200
   gagagcaatg ggcagccgga gaacaactac aagaccacgc ctcccgtgct ggactccgac 1260
   ggctccttct tcctctacag caagctcacc gtggacaaga gcaggtggca gcaggggaac 1320
   gtcttctcat gctccgtgat gcatgaggct ctgcacaacc actacacgca gaagagcctc 1380
   tccctgtctc cgggtaaatg a 1401
<210> 121
   <211> 466
   <212> PRT
   <213> Chimaera sp.
<400> 121
   Met Lys Val Leu Ser Leu Leu Tyr Leu Leu Thr Ala Ile Pro Gly Ile
   1               5                   10                  15
   Leu Ser Gln Val Gln Leu Gln Gln Pro Gly Ala Glu Leu Val Lys Pro
               20                  25                  30
   Gly Thr Ser Val Lys Met Ser Cys Lys Ala Ser Gly Tyr Thr Phe Thr
           35                  40                  45
   Ser Tyr Trp Met His Trp Val Lys Gln Arg Pro Gly Gln Gly Leu Glu
       50                  55                  60
   Trp Ile Gly Asp Ile Tyr Pro Gly Ser Asp Ser Thr Asn Tyr Asn Glu
   65                  70                  75                  80
   Lys Phe Lys Ser Lys Ala Thr Leu Thr Val Asp Thr Ser Ser Ser Thr
                   85                  90                  95
   Ala Tyr Met Gln Leu Ser Ser Leu Thr Ser Glu Asp Ser Ala Val Tyr
               100                 105                 110
   Tyr Cys Ala Arg Gly Gly Trp Leu Asp Ala Met Asp Tyr Trp Gly Gln
           115                 120                 125
   Gly Thr Ser Val Thr Val Ser Ser Ala Ser Thr Lys Gly Pro Ser Val
       130                 135                 140
   Phe Pro Leu Ala Pro Ser Ser Lys Ser Thr Ser Gly Gly Thr Ala Ala
   145                 150                 155                 160
   Leu Gly Cys Leu Val Lys Asp Tyr Phe Pro Glu Pro Val Thr Val Ser
                   165                 170                 175
   Trp Asn Ser Gly Ala Leu Thr Ser Gly Val His Thr Phe Pro Ala Val
               180                 185                 190
   Leu Gln Ser Ser Gly Leu Tyr Ser Leu Ser Ser Val Val Thr Val Pro
           195                 200                 205
   Ser Ser Ser Leu Gly Thr Gln Thr Tyr Ile Cys Asn Val Asn His Lys
       210                 215                 220
   Pro Ser Asn Thr Lys Val Asp Lys Lys Val Glu Pro Lys Ser Cys Asp
   225                 230                 235                 240
   Lys Thr His Thr Cys Pro Pro Cys Pro Ala Pro Glu Leu Leu Gly Gly
                   245                 250                 255
   Pro Ser Val Phe Leu Phe Pro Pro Lys Pro Lys Asp Thr Leu Met Ile
               260                 265                 270
   Ser Arg Thr Pro Glu Val Thr Cys Val Val Val Asp Val Ser His Glu
           275                 280                 285
   Asp Pro Glu Val Lys Phe Asn Trp Tyr Val Asp Gly Val Glu Val His
       290                 295                 300
   Asn Ala Lys Thr Lys Pro Arg Glu Glu Gln Tyr Asn Ser Thr Tyr Arg
   305                 310                 315                 320
   Val Val Ser Val Leu Thr Val Leu His Gln Asp Trp Leu Asn Gly Lys
                   325                 330                 335
   Glu Tyr Lys Cys Lys Val Ser Asn Lys Ala Leu Pro Ala Pro Ile Glu
               340                 345                 350
   Lys Thr Ile Ser Lys Ala Lys Gly Gln Pro Arg Glu Pro Gln Val Tyr
           355                 360                 365
   Thr Leu Pro Pro Ser Arg Asp Glu Leu Thr Lys Asn Gln Val Ser Leu
       370                 375                 380
   Thr Cys Leu Val Lys Gly Phe Tyr Pro Ser Asp Ile Ala Val Glu Trp
   385                 390                 395                 400
   Glu Ser Asn Gly Gln Pro Glu Asn Asn Tyr Lys Thr Thr Pro Pro Val
                   405                 410                 415
   Leu Asp Ser Asp Gly Ser Phe Phe Leu Tyr Ser Lys Leu Thr Val Asp
               420                 425                 430
   Lys Ser Arg Trp Gln Gln Gly Asn Val Phe Ser Cys Ser Val Met His
           435                 440                 445
   Glu Ala Leu His Asn His Tyr Thr Gln Lys Ser Leu Ser Leu Ser Pro
       450                 455                 460
   Gly Lys
   465
<210> 122
   <211> 705
   <212> DNA
   <213> Chimaera sp.
<400> 122
   atgatgtcct ctgctcagtt ccttggtctc ctgttgctct gttttcaagg taccagatgt 60
   gatatccaga tgacacagac tacatcctcc ctgtctgcct ctctgggaga cagagtcacc 120
   atcagttgca gggcaagtca ggacattagc aattatttaa actggtatca gcagaaacca 180
   gatggaactg ttaaactcct gatctactac acatcaagat tacactcagg agtcccatca 240
   aggttcagtg gcagtgggtc tggaacagat tattctctca ccattagcaa cctggagcaa 300
   gaagatattg ccacttactt ttgccaacag ggtaatacgc ttccgtggac gttcggtgga 360
   ggcaccaagc tggaaatcaa acgaactgtg gctgcaccat ctgtcttcat cttcccgcca 420
   tctgatgagc agttgaaatc tggaactgcc tctgttgtgt gcctgctgaa taacttctat 480
   cccagagagg ccaaagtaca gtggaaggtg gataacgccc tccaatcggg taactcccag 540
   gagagtgtca cagagcagga cagcaaggac agcacctaca gcctcagcag caccctgacg 600
   ctgagcaaag cagactacga gaaacacaaa gtctacgcct gcgaagtcac ccatcagggc 660
   ctgagctcgc ccgtcacaaa gagcttcaac aggggagagt gctag 705
<210> 123
   <211> 234
   <212> PRT
   <213> Chimaera sp.
<400> 123
   Met Met Ser Ser Ala Gln Phe Leu Gly Leu Leu Leu Leu Cys Phe Gln
   1 5 10 15
   Gly Thr Arg Cys Asp Ile Gln Met Thr Gln Thr Thr Ser Ser Leu Ser
   20 25 30
   Ala Ser Leu Gly Asp Arg Val Thr Ile Ser Cys Arg Ala Ser Gln Asp
   35 40 45
   Ile Ser Asn Tyr Leu Asn Trp Tyr Gln Gln Lys Pro Asp Gly Thr Val
   50 55 60
   Lys Leu Leu Ile Tyr Tyr Thr Ser Arg Leu His Ser Gly Val Pro Ser
   65 70 75 80
   Arg Phe Ser Gly Ser Gly Ser Gly Thr Asp Tyr Ser Leu Thr Ile Ser
   85 90 95
   Asn Leu Glu Gln Glu Asp Ile Ala Thr Tyr Phe Cys Gln Gln Gly Asn
   100 105 110
   Thr Leu Pro Trp Thr Phe Gly Gly Gly Thr Lys Leu Glu Ile Lys Arg
   115 120 125
   Thr Val Ala Ala Pro Ser Val Phe Ile Phe Pro Pro Ser Asp Glu Gln
   130 135 140
   Leu Lys Ser Gly Thr Ala Ser Val Val Cys Leu Leu Asn Asn Phe Tyr
   145 150 155 160
   Pro Arg Glu Ala Lys Val Gln Trp Lys Val Asp Asn Ala Leu Gln Ser
   165 170 175
   Gly Asn Ser Gln Glu Ser Val Thr Glu Gln Asp Ser Lys Asp Ser Thr
   180 185 190
   Tyr Ser Leu Ser Ser Thr Leu Thr Leu Ser Lys Ala Asp Tyr Glu Lys
   195 200 205
   His Lys Val Tyr Ala Cys Glu Val Thr His Gln Gly Leu Ser Ser Pro
   210 215 220
   Val Thr Lys Ser Phe Asn Arg Gly Glu Cys
   225 230
<210> 124
   <211> 1521
   <212> DNA
   <213> Macaca mulatta
<220>
   <221> gene
   <222> (1).. (1521)
<400> 124
   atgctgaagc ctcttcggag agcggcagtg acccccatgt ggccgtgctc catgctgccc 60
   cgccgcctgt gggacagaga ggctggcacg ttgcaggtcc tgggagtgct ggctatgctg 120
   tggctgggct ccatggctct tacctacctc ctgtggcaag tgcgctgtcc tcccacctgg 180
   ggccaggtgc agcccaggga cgtgcccagg tcctgggggc atggttccag cctagctctg 240
   gagcccctgg aagcggaggt caggaagcag agggactcct gccagcttgt ccttgtggaa 300
   agcatccccc aggacctgcc atttgcagcc ggcagcctct ccgcccagcc tctgggccag 360
   gcctggctgc agctgctgga cactgcccag gagagcgtcc acgtggcttc atactactgg 420
   tccctcacag ggcccgacat tggggtcaac gactcatctt cccagctggg agaggcccct 480
   ctgcagaagc tgcagcagct gctgggcagg aacatttcct tggctgtggc caccagcagt 540
   ccaacactgg ccaggaagtc caccgacctg caggtcctgg ctgcccgagg tgcccaggta 600
   cgacgggtgc ccatggggcg gctcaccagg ggcgttttgc actccaaatt ctgggttgtg 660
   gatggacggc acatatacat gggcagtgcc aacatggact ggcggtccct gacgcaggtg 720
   aaggagcttg gcgctgtcat ctataactgc agccacctgg cccaagacct ggagaagacc 780
   ttccagacct actgggtgct gggggtgccc aaggctgtcc tccccaaaac ctggcctcag 840
   aacttctcat ctcacatcaa ccgtttccag cccttccagg gcctctttga tggggtgccc 900
   accactgcct acttctcagc atcgccaccc gcactctgtc cccagggccg cacccctgac 960
   ctggaggcgc tgttggcggt gatggggagc gcccaggagt tcatctatgc ctccgtgatg 1020
   gagtatttcc ctaccacgcg cttcagccac ccccgcaggt actggccggt gctggacaac 1080
   gcgctgcggg cggcagcctt cagcaagggt gtgcgcgtgc gcctgctggt cagctgcgga 1140
   ctcaacacgg accccaccat gttcccctat ctgcggtccc tgcaggcgct cagcaacccc 1200
   gcggccaacg tctctgtgga cgtgaaagtc ttcatcgtgc cggtggggaa tcattccaac 1260
   atcccgttca gcagggtgaa ccacagcaag ttcatggtca cggagaaggc agcctacata 1320
   ggcacctcca actggtcgga ggattacttc agcagcacga cgggggtggg cctggtggtc 1380
   acccagagcc ccggcgcgca gcccgcgggg gccacggtac aggagcagct gcggcagctc 1440
   tttgagcggg actggagttc gcgctacgcc gtcggcctgg acggacaggc tccgggccag 1500
   gactgcgttt ggcagggctg a 1521
<210> 125
   <211> 2142
   <212> DNA
   <213> Homo sapiens
<400> 125
   atggagtttc agacccaggt ctttgtattc gtgttgctct ggttgtctgg tgttgatgga     60
   gattacaagg atgacgacga taaaggatcc cccagagggc ccacaatcaa gccctgtcct    120
   ccatgcaaat gcccagcacc taacctcttg ggtggaccat ccgtcttcat cttccctcca    180
   aagatcaagg atgtactcat gatctccctg agccccatag tcacatgtgt ggtggtggat    240
   gtgagcgagg atgacccaga tgtccagatc agctggtttg tgaacaacgt ggaagtacac    300
   acagctcaga cacaaaccca tagagaggat tacaacagta ctctccgggt ggtcagtgcc    360
   ctccccatcc agcaccagga ctggatgagt ggcaaggagt tcaaatgcaa ggtcaacaac    420
   aaagacctcc cagcgcccat cgagagaacc atctcaaaac ccaaagggtc agtaagagct    480
   ccacaggtat atgtcttgcc tccaccagaa gaagagatga ctaagaaaca ggtcactctg    540
   acctgcatgg tcacagactt catgcctgaa gacatttacg tggagtggac caacaacggg    600
   aaaacagagc taaactacaa gaacactgaa ccagtcctgg actctgatgg ttcttacttc    660
   atgtacagca agctgagagt ggaaaagaag aactgggtgg aaagaaatag ctactcctgt    720
   tcagtggtcc acgagggtct gcacaatcac cacacgacta agagcttctc ccggactccg    780
   ggtaaacgtc ctcccacctg gggccaggtg cagcccaagg acgtgcccag gtcctgggag    840
   catggctcca gcccagcttg ggagcccctg gaagcagagg ccaggcagca gagggactcc    900
   tgccagcttg tccttgtgga aagcatcccc caggacctgc catctgcagc cggcagcccc    960
   tctgcccagc ctctgggcca ggcctggctg cagctgctgg acactgccca ggagagcgtc   1020
   cacgtggctt catactactg gtccctcaca gggcctgaca tcggggtcaa cgactcgtct   1080
   tcccagctgg gagaggctct tctgcagaag ctgcagcagc tgctgggcag gaacatttcc   1140
   ctggctgtgg ccaccagcag cccgacactg gccaggacat ccaccgacct gcaggttctg   1200
   gctgcccgag gtgcccatgt acgacaggtg cccatggggc ggctcaccag ggggtgtttg   1260
   cactccaaat tctgggttgt ggatggacgg cacatataca tgggcagtgc caacatggac   1320
   tggcggtctc tgacgcaggt gaaggagctt ggcgctgtca tctataactg cagccacctg   1380
   gcccaagacc tggagaagac cttccagacc tactgggtac tgggggtgcc caaggctgtc   1440
   ctccccaaaa cctggcctca gaacttctca tctcacttca accgtttcca gcccttccac   1500
   ggcctctttg atggggtgcc caccactgcc tacttctcag cgtcgccacc agcactctgt   1560
   ccccagggcc gcacccggga cctggaggcg ctgctggcgg tgatggggag cgcccaggag   1620
   ttcatctatg cctccgtgat ggagtatttc cccaccacgc gcttcagcca ccccccgagg   1680
   tactggccgg tgctggacaa cgcgctgcgg gcggcagcct tcggcaaggg cgtgcgcgtg   1740
   cgcctgctgg tcggctgcgg actcaacacg gaccccacca tgttccccta cctgcggtcc 1800
   ctgcaggcgc tcagcaaccc cgcggccaac gtctctgtgg acgtgaaagt cttcatcgtg 1860
   ccggtgggga accattccaa catcccattc agcagggtga accacagcaa gttcatggtc 1920
   acggagaagg cagcctacat aggcacctcc aactggtcgg aggattactt cagcagcacg 1980
   gcgggggtgg gcttggtggt cacccagagc cctggcgcgc agcccgcggg ggccacggtg 2040
   caggagcagc tgcggcagct ctttgagcgg gactggagtt cgcgctacgc cgtcggcctg 2100
   gacggacagg ctccgggcca ggactgcgtt tggcagggct ga 2142
<210> 126
   <211> 713
   <212> PRT
   <213> Homo sapiens
<400> 126
   Met Glu Phe Gln Thr Gln Val Phe Val Phe Val Leu Leu Trp Leu Ser
   1 5 10 15
   Gly Val Asp Gly Asp Tyr Lys Asp Asp Asp Asp Lys Gly Ser Pro Arg
   20 25 30
   Gly Pro Thr Ile Lys Pro Cys Pro Pro Cys Lys Cys Pro Ala Pro Asn
   35 40 45
   Leu Leu Gly Gly Pro Ser Val Phe Ile Phe Pro Pro Lys Ile Lys Asp
   50 55 60
   Val Leu Met Ile Ser Leu Ser Pro Ile Val Thr Cys Val Val Val Asp
   65 70 75 80
   Val Ser Glu Asp Asp Pro Asp Val Gln Ile Ser Trp Phe Val Asn Asn
   85 90 95
   Val Glu Val His Thr Ala Gln Thr Gln Thr His Arg Glu Asp Tyr Asn
   100 105 110
   Ser Thr Leu Arg Val Val Ser Ala Leu Pro Ile Gln His Gln Asp Trp
   115 120 125
   Met Ser Gly Lys Glu Phe Lys Cys Lys Val Asn Asn Lys Asp Leu Pro
   130 135 140
   Ala Pro Ile Glu Arg Thr Ile Ser Lys Pro Lys Gly Ser Val Arg Ala
   145 150 155 160
   Pro Gln Val Tyr Val Leu Pro Pro Pro Glu Glu Glu Met Thr Lys Lys
   165 170 175
   Gln Val Thr Leu Thr Cys Met Val Thr Asp Phe Met Pro Glu Asp Ile
   180 185 190
   Tyr Val Glu Trp Thr Asn Asn Gly Lys Thr Glu Leu Asn Tyr Lys Asn
   195 200 205
   Thr Glu Pro Val Leu Asp Ser Asp Gly Ser Tyr Phe Met Tyr Ser Lys
   210 215 220
   Leu Arg Val Glu Lys Lys Asn Trp Val Glu Arg Asn Ser Tyr Ser Cys
   225 230 235 240
   Ser Val Val His Glu Gly Leu His Asn His His Thr Thr Lys Ser Phe
   245 250 255
   Ser Arg Thr Pro Gly Lys Arg Pro Pro Thr Trp Gly Gln Val Gln Pro
   260 265 270
   Lys Asp Val Pro Arg Ser Trp Glu His Gly Ser Ser Pro Ala Trp Glu
   275 280 285
   Pro Leu Glu Ala Glu Ala Arg Gln Gln Arg Asp Ser Cys Gln Leu Val
   290 295 300
   Leu Val Glu Ser Ile Pro Gln Asp Leu Pro Ser Ala Ala Gly Ser Pro
   305 310 315 320
   Ser Ala Gln Pro Leu Gly Gln Ala Trp Leu Gln Leu Leu Asp Thr Ala
   325 330 335
   Gln Glu Ser Val His Val Ala Ser Tyr Tyr Trp Ser Leu Thr Gly Pro
   340 345 350
   Asp Ile Gly Val Asn Asp Ser Ser Ser Gln Leu Gly Glu Ala Leu Leu
   355 360 365
   Gln Lys Leu Gln Gln Leu Leu Gly Arg Asn Ile Ser Leu Ala Val Ala
   370 375 380
   Thr Ser Ser Pro Thr Leu Ala Arg Thr Ser Thr Asp Leu Gln Val Leu
   385 390 395 400
   Ala Ala Arg Gly Ala His Val Arg Gln Val Pro Met Gly Arg Leu Thr
   405 410 415
   Arg Gly Val Leu His Ser Lys Phe Trp Val Val Asp Gly Arg His Ile
   420 425 430
   Tyr Met Gly Ser Ala Asn Met Asp Trp Arg Ser Leu Thr Gln Val Lys
   435 440 445
   Glu Leu Gly Ala Val Ile Tyr Asn Cys Ser His Leu Ala Gln Asp Leu
   450 455 460
   Glu Lys Thr Phe Gln Thr Tyr Trp Val Leu Gly Val Pro Lys Ala Val
   465 470 475 480
   Leu Pro Lys Thr Trp Pro Gln Asn Phe Ser Ser His Phe Asn Arg Phe
   485 490 495
   Gln Pro Phe His Gly Leu Phe Asp Gly Val Pro Thr Thr Ala Tyr Phe
   500 505 510
   Ser Ala Ser Pro Pro Ala Leu Cys Pro Gln Gly Arg Thr Arg Asp Leu
   515 520 525
   Glu Ala Leu Leu Ala Val Met Gly Ser Ala Gln Glu Phe Ile Tyr Ala
   530 535 540
   Ser Val Met Glu Tyr Phe Pro Thr Thr Arg Phe Ser His Pro Pro Arg
   545 550 555 560
   Tyr Trp Pro Val Leu Asp Asn Ala Leu Arg Ala Ala Ala Phe Gly Lys
   565 570 575
   Gly Val Arg Val Arg Leu Leu Val Gly Cys Gly Leu Asn Thr Asp Pro
   580 585 590
   Thr Met Phe Pro Tyr Leu Arg Ser Leu Gln Ala Leu Ser Asn Pro Ala
   595 600 605
   Ala Asn Val Ser Val Asp Val Lys Val Phe Ile Val Pro Val Gly Asn
   610 615 620
   His Ser Asn Ile Pro Phe Ser Arg Val Asn His Ser Lys Phe Met Val
   625 630 635 640
   Thr Glu Lys Ala Ala Tyr Ile Gly Thr Ser Asn Trp Ser Glu Asp Tyr
   645 650 655
   Phe Ser Ser Thr Ala Gly Val Gly Leu Val Val Thr Gln Ser Pro Gly
   660 665 670
   Ala Gln Pro Ala Gly Ala Thr Val Gln Glu Gln Leu Arg Gln Leu Phe
   675 680 685
   Glu Arg Asp Trp Ser Ser Arg Tyr Ala Val Gly Leu Asp Gly Gln Ala
   690 695 700
   Pro Gly Gln Asp Cys Val Trp Gln Gly
   705 710
<210> 127
   <211> 490
   <212> PRT
   <213> Homo sapiens
<400> 127
   Met Lys Pro Lys Leu Met Tyr Gln Glu Leu Lys Val Pro Ala Glu Glu
   1 5 10 15
   Pro Ala Asn Glu Leu Pro Met Asn Glu Ile Glu Ala Trp Lys Ala Ala
   20 25 30
   Glu Lys Lys Ala Arg Trp Val Leu Leu Val Leu Ile Leu Ala Val Val
   35 40 45
   Gly Phe Gly Ala Leu Met Thr Gln Leu Phe Leu Trp Glu Tyr Gly Asp
   50 55 60
   Leu His Leu Phe Gly Pro Asn Gln Arg Pro Ala Pro Cys Tyr Asp Pro
   65 70 75 80
   Cys Glu Ala Val Leu Val Glu Ser Ile Pro Glu Gly Leu Asp Phe Pro
   85 90 95
   Asn Ala Ser Thr Gly Asn Pro Ser Thr Ser Gln Ala Trp Leu Gly Leu
   100 105 110
   Leu Ala Gly Ala His Ser Ser Leu Asp Ile Ala Ser Phe Tyr Trp Thr
   115 120 125
   Leu Thr Asn Asn Asp Thr His Thr Gln Glu Pro Ser Ala Gln Gln Gly
   130 135 140
   Glu Glu Val Leu Arg Gln Leu Gln Thr Leu Ala Pro Lys Gly Val Asn
   145 150 155 160
   Val Arg Ile Ala Val Ser Lys Pro Ser Gly Pro Gln Pro Gln Ala Asp
   165 170 175
   Leu Gln Ala Leu Leu Gln Ser Gly Ala Gln Val Arg Met Val Asp Met
   180 185 190
   Gln Lys Leu Thr His Gly Val Leu His Thr Lys Phe Trp Val Val Asp
   195 200 205
   Gln Thr His Phe Tyr Leu Gly Ser Ala Asn Met Asp Trp Arg Ser Leu
   210 215 220
   Thr Gln Val Lys Glu Leu Gly Val Val Met Tyr Asn Cys Ser Cys Leu
   225 230 235 240
   Ala Arg Asp Leu Thr Lys Ile Phe Glu Ala Tyr Trp Phe Leu Gly Gln
   245 250 255
   Ala Gly Ser Ser Ile Pro Ser Thr Trp Pro Arg Phe Tyr Asp Thr Arg
   260 265 270
   Tyr Asn Gln Glu Thr Pro Met Glu Ile Cys Leu Asn Gly Thr Pro Ala
   275 280 285
   Leu Ala Tyr Leu Ala Ser Ala Pro Pro Pro Leu Cys Pro Ser Gly Arg
   290 295 300
   Thr Pro Asp Leu Lys Ala Leu Leu Asn Val Val Asp Asn Ala Arg Ser
   305 310 315 320
   Phe Ile Tyr Val Ala Val Met Asn Tyr Leu Pro Thr Leu Glu Phe Ser
   325 330 335
   His Pro His Arg Phe Trp Pro Ala Ile Asp Asp Gly Leu Arg Arg Ala
   340 345 350
   Thr Tyr Glu Arg Gly Val Lys Val Arg Leu Leu Ile Ser Cys Trp Gly
   355 360 365
   His Ser Glu Pro Ser Met Arg Ala Phe Leu Leu Ser Leu Ala Ala Leu
   370 375 380
   Arg Asp Asn His Thr His Ser Asp Ile Gln Val Lys Leu Phe Val Val
   385 390 395 400
   Pro Ala Asp Glu Ala Gln Ala Arg Ile Pro Tyr Ala Arg Val Asn His
   405 410 415
   Asn Lys Tyr Met Val Thr Glu Arg Ala Thr Tyr Ile Gly Thr Ser Asn
   420 425 430
   Trp Ser Gly Asn Tyr Phe Thr Glu Thr Ala Gly Thr Ser Leu Leu Val
   435 440 445
   Thr Gln Asn Gly Arg Gly Gly Leu Arg Ser Gln Leu Glu Ala Ile Phe
   450 455 460
   Leu Arg Asp Trp Asp Ser Pro Tyr Ser His Asp Leu Asp Thr Ser Ala
   465 470 475 480
   Asp Ser Val Gly Asn Ala Cys Arg Leu Leu
   485 490
<210> 128
   <211> 445
   <212> PRT
   <213> Homo sapiens
<400> 128
   Met Gly Glu Asp Glu Asp Gly Leu Ser Glu Lys Asn Cys Gln Asn Lys
   1               5                   10                  15
   Cys Arg Ile Ala Leu Val Glu Asn Ile Pro Glu Gly Leu Asn Tyr Ser
               20                  25                  30
   Glu Asn Ala Pro Phe His Leu Ser Leu Phe Gln Gly Trp Met Asn Leu
   35 40 45
   Leu Asn Met Ala Lys Lys Ser Val Asp Ile Val Ser Ser His Trp Asp
   50 55 60
   Leu Asn His Thr His Pro Ser Ala Cys Gln Gly Gln Arg Leu Phe Glu
   65 70 75 80
   Lys Leu Leu Gln Leu Thr Ser Gln Asn Ile Glu Ile Lys Leu Val Ser
   85 90 95
   Asp Val Thr Ala Asp Ser Lys Val Leu Glu Ala Leu Lys Leu Lys Gly
   100 105 110
   Ala Glu Val Thr Tyr Met Asn Met Thr Ala Tyr Asn Lys Gly Arg Leu
   115 120 125
   Gln Ser Ser Phe Trp Ile Val Asp Lys Gln His Val Tyr Ile Gly Ser
   130 135 140
   Ala Gly Leu Asp Trp Gln Ser Leu Gly Gln Met Lys Glu Leu Gly Val
   145 150 155 160
   Ile Phe Tyr Asn Cys Ser Cys Leu Val Leu Asp Leu Gln Arg Ile Phe
   165 170 175
   Ala Leu Tyr Ser Ser Leu Lys Phe Lys Ser Arg Val Pro Gln Thr Trp
   180 185 190
   Ser Lys Arg Leu Tyr Gly Val Tyr Asp Asn Glu Lys Lys Leu Gln Leu
   195 200 205
   Gln Leu Asn Glu Thr Lys Ser Gln Ala Phe Val Ser Asn Ser Pro Lys
   210 215 220
   Leu Phe Cys Pro Lys Asn Arg Ser Phe Asp Ile Asp Ala Ile Tyr Ser
   225 230 235 240
   Val Ile Asp Asp Ala Lys Gln Tyr Val Tyr Ile Ala Val Met Asp Tyr
   245 250 255
   Leu Pro Ile Ser Ser Thr Ser Thr Lys Arg Thr Tyr Trp Pro Asp Leu
   260 265 270
   Asp Ala Lys Ile Arg Glu Ala Leu Val Leu Arg Ser Val Arg Val Arg
   275 280 285
   Leu Leu Leu Ser Phe Trp Lys Glu Thr Asp Pro Leu Thr Phe Asn Phe
   290 295 300
   Ile Ser Ser Leu Lys Ala Ile Cys Thr Glu Ile Ala Asn Cys Ser Leu
   305 310 315 320
   Lys Val Lys Phe Phe Asp Leu Glu Arg Glu Asn Ala Cys Ala Thr Lys
   325 330 335
   Glu Gln Lys Asn His Thr Phe Pro Arg Leu Asn Arg Asn Lys Tyr Met
   340 345 350
   Val Thr Asp Gly Ala Ala Tyr Ile Gly Asn Phe Asp Trp Val Gly Asn
   355 360 365
   Asp Phe Thr Gln Asn Ala Gly Thr Gly Leu Val Ile Asn Gln Ala Asp
   370 375 380
   Val Arg Asn Asn Arg Ser Ile Ile Lys Gln Leu Lys Asp Val Phe Glu
   385 390 395 400
   Arg Asp Trp Tyr Ser Pro Tyr Ala Lys Thr Leu Gln Pro Thr Lys Gln
   405 410 415
   Pro Asn Cys Ser Ser Leu Phe Lys Leu Lys Pro Leu Ser Asn Lys Thr
   420 425 430
   Ala Thr Asp Asp Thr Gly Gly Lys Asp Pro Arg Asn Val
   435 440 445
<210> 129
   <211> 506
   <212> PRT
   <213> Macaca fascicularis
<400> 129
   Met Leu Lys Pro Leu Arg Arg Ala Ala Val Thr Pro Met Trp Pro Cys
   1               5                   10                  15
   Ser Met Leu Pro Arg Arg Leu Trp Asp Arg Glu Ala Gly Thr Leu Gln
   20 25 30
   Val Leu Gly Val Leu Ala Met Leu Trp Leu Gly Ser Met Ala Leu Thr
   35 40 45
   Tyr Leu Leu Trp Gln Val Arg Arg Pro Pro Thr Trp Gly Gln Val Gln
   50 55 60
   Pro Lys Asp Val Pro Arg Ser Trp Gly His Gly Ser Ser Pro Ala Leu
   65 70 75 80
   Glu Pro Leu Glu Ala Glu Val Arg Lys Gln Arg Asp Ser Cys Gln Leu
   85 90 95
   Val Leu Val Glu Ser Ile Pro Gln Asp Leu Pro Phe Ala Ala Gly Ser
   100 105 110
   Leu Ser Ala Gln Pro Leu Gly Gln Ala Trp Leu Gln Leu Leu Asp Thr
   115 120 125
   Ala Gln Glu Ser Val His Val Ala Ser Tyr Tyr Trp Ser Leu Thr Gly
   130 135 140
   Pro Asp Ile Gly Val Asn Asp Ser Ser Ser Gln Leu Gly Glu Ala Leu
   145 150 155 160
   Leu Gln Lys Leu Gln Gln Leu Leu Gly Arg Asn Ile Ser Leu Ala Val
   165 170 175
   Ala Thr Ser Ser Pro Thr Leu Ala Arg Lys Ser Thr Asp Leu Gln Val
   180 185 190
   Leu Ala Ala Arg Gly Ala Gln Val Arg Arg Val Pro Met Gly Arg Leu
   195 200 205
   Thr Arg Gly Val Leu His Ser Lys Phe Trp Val Val Asp Gly Arg His
   210 215 220
   Ile Tyr Met Gly Ser Ala Asn Met Asp Trp Arg Ser Leu Thr Gln Val
   225 230 235 240
   Lys Glu Leu Gly Ala Val Ile Tyr Asn Cys Ser His Leu Ala Gln Asp
   245 250 255
   Leu Glu Lys Thr Phe Gln Thr Tyr Trp Val Leu Gly Val Pro Lys Ala
   260 265 270
   Val Leu Pro Lys Thr Trp Pro Gln Asn Phe Ser Ser His Ile Asn Arg
   275 280 285
   Phe Gln Pro Phe Gln Gly Leu Phe Asp Gly Val Pro Thr Thr Ala Tyr
   290 295 300
   Phe Ser Ala Ser Pro Pro Ala Leu Cys Pro Gln Gly Arg Thr Pro Asp
   305 310 315 320
   Leu Glu Ala Leu Leu Ala Val Met Gly Ser Ala Gln Glu Phe Ile Tyr
   325 330 335
   Ala Ser Val Met Glu Tyr Phe Pro Thr Thr Arg Phe Ser His Pro Arg
   340 345 350
   Arg Tyr Trp Pro Val Leu Asp Asn Ala Leu Arg Ala Ala Ala Phe Ser
   355 360 365
   Lys Gly Val Arg Val Arg Leu Leu Val Ser Cys Gly Leu Asn Thr Asp
   370 375 380
   Pro Thr Met Phe Pro Tyr Leu Arg Ser Leu Gln Ala Leu Ser Asn Pro
   385 390 395 400
   Ala Ala Asn Val Ser Val Asp Val Lys Val Phe Ile Val Pro Val Gly
   405 410 415
   Asn His Ser Asn Ile Pro Phe Ser Arg Val Asn His Ser Lys Phe Met
   420 425 430
   Val Thr Glu Lys Ala Ala Tyr Ile Gly Thr Ser Asn Trp Ser Glu Asp
   435 440 445
   Tyr Phe Ser Ser Thr Thr Gly Val Gly Leu Val Val Thr Gln Ser Pro
   450 455 460
   Gly Ala Gln Pro Ala Gly Ala Thr Val Gln Glu Gln Leu Arg Gln Leu
   465 470 475 480
   Phe Glu Arg Asp Trp Ser Ser Arg Tyr Ala Val Gly Leu Asp Gly Gln
   485 490 495
   Ala Pro Gly Gln Asp Cys Val Trp Gln Gly
   500 505
<210> 130
   <211> 506
   <212> PRT
   <213> Macaca mulatta
<400> 130
   Met Leu Lys Pro Leu Arg Arg Ala Ala Val Thr Pro Met Trp Pro Cys
   1               5                   10                  15
   Ser Met Leu Pro Arg Arg Leu Trp Asp Arg Glu Ala Gly Thr Leu Gln
               20                  25                  30
   Val Leu Gly Val Leu Ala Met Leu Trp Leu Gly Ser Met Ala Leu Thr
           35                  40                  45
   Tyr Leu Leu Trp Gln Val Arg Cys Pro Pro Thr Trp Gly Gln Val Gln
       50                  55                  60
   Pro Arg Asp Val Pro Arg Ser Trp Gly His Gly Ser Ser Leu Ala Leu
   65                  70                  75                  80
   Glu Pro Leu Glu Ala Glu Val Arg Lys Gln Arg Asp Ser Cys Gln Leu
                   85                  90                  95
   Val Leu Val Glu Ser Ile Pro Gln Asp Leu Pro Phe Ala Ala Gly Ser
               100                 105                 110
   Leu Ser Ala Gln Pro Leu Gly Gln Ala Trp Leu Gln Leu Leu Asp Thr
           115                 120                 125
   Ala Gln Glu Ser Val His Val Ala Ser Tyr Tyr Trp Ser Leu Thr Gly
       130                 135                 140
   Pro Asp Ile Gly Val Asn Asp Ser Ser Ser Gln Leu Gly Glu Ala Leu
   145                 150                 155                 160
   Leu Gln Lys Leu Gln Gln Leu Leu Gly Arg Asn Ile Ser Leu Ala Val
                   165                 170                 175
   Ala Thr Ser Ser Pro Thr Leu Ala Arg Lys Ser Thr Asp Leu Gln Val
   180 185 190
   Leu Ala Ala Arg Gly Ala Gln Val Arg Arg Val Pro Met Gly Arg Leu
   195 200 205
   Thr Arg Gly Val Leu His Ser Lys Phe Trp Val Val Asp Gly Arg His
   210 215 220
   Ile Tyr Met Gly Ser Ala Asn Met Asp Trp Arg Ser Leu Thr Gln Val
   225 230 235 240
   Lys Glu Leu Gly Ala Val Ile Tyr Asn Cys Ser His Leu Ala Gln Asp
   245 250 255
   Leu Glu Lys Thr Phe Gln Thr Tyr Trp Val Leu Gly Val Pro Lys Ala
   260 265 270
   Val Leu Pro Lys Thr Trp Pro Gln Asn Phe Ser Ser His Ile Asn Arg
   275 280 285
   Phe Gln Pro Phe Gln Gly Leu Phe Asp Gly Val Pro Thr Thr Ala Tyr
   290 295 300
   Phe Ser Ala Ser Pro Pro Ala Leu Cys Pro Gln Gly Arg Thr Pro Asp
   305 310 315 320
   Leu Glu Ala Leu Leu Ala Val Met Gly Ser Ala Gln Glu Phe Ile Tyr
   325 330 335
   Ala Ser Val Met Glu Tyr Phe Pro Thr Thr Arg Phe Ser His Pro Arg
   340 345 350
   Arg Tyr Trp Pro Val Leu Asp Asn Ala Leu Arg Ala Ala Ala Phe Ser
   355 360 365
   Lys Gly Val Arg Val Arg Leu Leu Val Ser Cys Gly Leu Asn Thr Asp
   370 375 380
   Pro Thr Met Phe Pro Tyr Leu Arg Ser Leu Gln Ala Leu Ser Asn Pro
   385 390 395 400
   Ala Ala Asn Val Ser Val Asp Val Lys Val Phe Ile Val Pro Val Gly
   405 410 415
   Asn His Ser Asn Ile Pro Phe Ser Arg Val Asn His Ser Lys Phe Met
   420 425 430
   Val Thr Glu Lys Ala Ala Tyr Ile Gly Thr Ser Asn Trp Ser Glu Asp
   435 440 445
   Tyr Phe Ser Ser Thr Thr Gly Val Gly Leu Val Val Thr Gln Ser Pro
   450 455 460
   Gly Ala Gln Pro Ala Gly Ala Thr Val Gln Glu Gln Leu Arg Gln Leu
   465 470 475 480
   Phe Glu Arg Asp Trp Ser Ser Arg Tyr Ala Val Gly Leu Asp Gly Gln
   485 490 495
   Ala Pro Gly Gln Asp Cys Val Trp Gln Gly
   500 505
<210> 131
   <211> 1512
   <212> DNA
   <213> Mus musculus
<400> 131
   atggacaaga agaaagagca cccagagatg cggataccac tccagacagc agtggaggtc 60
   tctgattggc cctgctccac atctcatgat ccacatagcg gacttggcat ggtactgggg 120
   atgctagctg tactgggact cagctctgtg actctcatct tgttcctgtg gcaaggggcc 180
   acttctttca ccagtcatcg gatgttccct gaggaagtgc cctcctggtc ctgggagacc 240
   ctgaaaggag acgctgagca gcagaataac tcctgtcagc tcatccttgt ggaaagcatc 300
   cccgaggact tgccatttgc agctggcagc cccactgccc agcccctggc ccaggcttgg 360
   ctgcagcttc ttgacactgc tcgggagagc gtccacattg cctcgtacta ctggtccctc 420
   actggactgg acattggagt caatgactcg tcttctcggc agggagaggc ccttctacag 480
   aagttccaac agcttcttct caggaacatc tctgtggtgg tggccaccca cagcccaaca 540
   ttggccaaga catccactga cctccaggtc ttggctgccc atggtgccca gatacgacaa 600
   gtgcccatga aacagcttac tgggggtgtt ctacactcca aattctgggt tgtggatggg 660
   cgacacgtct acgtgggcag cgccaacatg gactggcggt ccctgactca ggtgaaggaa 720
   cttggtgcaa tcatctacaa ctgcagcaac ctggctcaag accttgagaa aacattccag 780
   acctactggg tgctagggac tccccaagct gttctcccta aaacctggcc tcggaacttc 840
   tcatcccaca tcaaccgctt ccatcccttg cggggtccct ttgatggggt tcccaccacg 900
   gcctatttct cggcctcccc tccctccctc tgcccgcatg gccggacccg ggatctggac 960
   gcagtgttgg gagtgatgga gggtgctcgc cagttcatct atgtctcggt gatggagtat 1020
   ttccctacca cgcgcttcac ccaccatgcc aggtactggc ccgtgctgga caatgcgcta 1080
   cgggcagcgg ccctcaataa gggtgtgcat gtgcgcttac tggtcagctg ctggttcaac 1140
   acagacccca ccatgttcgc ttatctgagg tccctgcagg ctttcagtaa cccctcggct 1200
   ggcatctcag tggatgtgaa agtcttcatc gtgcctgtgg gaaatcattc caacatcccg 1260
   ttcagccgcg tgaaccacag caagttcatg gtcacagaca agacagccta tgtaggcacc 1320
   tctaactggt cagaagacta cttcagccac accgctggtg tgggcctgat tgtcagccag 1380
   aagaccccca gagcccagcc aggcgcaacc accgtgcagg agcagctgag gcaactcttt 1440
   gaacgagact ggagttccca ctatgctatg gacctagaca gacaagtccc gagccaggac 1500
   tgtgtctggt ag 1512
<210> 132
   <211> 503
   <212> PRT
   <213> Mus musculus
<400> 132
   Met Asp Lys Lys Lys Glu His Pro Glu Met Arg Ile Pro Leu Gln Thr
   1               5                   10                  15
   Ala Val Glu Val Ser Asp Trp Pro Cys Ser Thr Ser His Asp Pro His
               20                  25                  30
   Ser Gly Leu Gly Met Val Leu Gly Met Leu Ala Val Leu Gly Leu Ser
           35                  40                  45
   Ser Val Thr Leu Ile Leu Phe Leu Trp Gln Gly Ala Thr Ser Phe Thr
       50                  55                  60
   Ser His Arg Met Phe Pro Glu Glu Val Pro Ser Trp Ser Trp Glu Thr
   65                  70                  75                  80
   Leu Lys Gly Asp Ala Glu Gln Gln Asn Asn Ser Cys Gln Leu Ile Leu
                   85                  90                  95
   Val Glu Ser Ile Pro Glu Asp Leu Pro Phe Ala Ala Gly Ser Pro Thr
   100 105 110
   Ala Gln Pro Leu Ala Gln Ala Trp Leu Gln Leu Leu Asp Thr Ala Arg
   115 120 125
   Glu Ser Val His Ile Ala Ser Tyr Tyr Trp Ser Leu Thr Gly Leu Asp
   130 135 140
   Ile Gly Val Asn Asp Ser Ser Ser Arg Gln Gly Glu Ala Leu Leu Gln
   145 150 155 160
   Lys Phe Gln Gln Leu Leu Leu Arg Asn Ile Ser Val Val Val Ala Thr
   165 170 175
   His Ser Pro Thr Leu Ala Lys Thr Ser Thr Asp Leu Gln Val Leu Ala
   180 185 190
   Ala His Gly Ala Gln Ile Arg Gln Val Pro Met Lys Gln Leu Thr Gly
   195 200 205
   Gly Val Leu His Ser Lys Phe Trp Val Val Asp Gly Arg His Val Tyr
   210 215 220
   Val Gly Ser Ala Asn Met Asp Trp Arg Ser Leu Thr Gln Val Lys Glu
   225 230 235 240
   Leu Gly Ala Ile Ile Tyr Asn Cys Ser Asn Leu Ala Gln Asp Leu Glu
   245 250 255
   Lys Thr Phe Gln Thr Tyr Trp Val Leu Gly Thr Pro Gln Ala Val Leu
   260 265 270
   Pro Lys Thr Trp Pro Arg Asn Phe Ser Ser His Ile Asn Arg Phe His
   275 280 285
   Pro Leu Arg Gly Pro Phe Asp Gly Val Pro Thr Thr Ala Tyr Phe Ser
   290 295 300
   Ala Ser Pro Pro Ser Leu Cys Pro His Gly Arg Thr Arg Asp Leu Asp
   305 310 315 320
   Ala Val Leu Gly Val Met Glu Gly Ala Arg Gln Phe Ile Tyr Val Ser
   325 330 335
   Val Met Glu Tyr Phe Pro Thr Thr Arg Phe Thr His His Ala Arg Tyr
   340 345 350
   Trp Pro Val Leu Asp Asn Ala Leu Arg Ala Ala Ala Leu Asn Lys Gly
   355 360 365
   Val His Val Arg Leu Leu Val Ser Cys Trp Phe Asn Thr Asp Pro Thr
   370 375 380
   Met Phe Ala Tyr Leu Arg Ser Leu Gln Ala Phe Ser Asn Pro Ser Ala
   385 390 395 400
   Gly Ile Ser Val Asp Val Lys Val Phe Ile Val Pro Val Gly Asn His
   405 410 415
   Ser Asn Ile Pro Phe Ser Arg Val Asn His Ser Lys Phe Met Val Thr
   420 425 430
   Asp Lys Thr Ala Tyr Val Gly Thr Ser Asn Trp Ser Glu Asp Tyr Phe
   435 440 445
   Ser His Thr Ala Gly Val Gly Leu Ile Val Ser Gln Lys Thr Pro Arg
   450 455 460
   Ala Gln Pro Gly Ala Thr Thr Val Gln Glu Gln Leu Arg Gln Leu Phe
   465 470 475 480
   Glu Arg Asp Trp Ser Ser His Tyr Ala Met Asp Leu Asp Arg Gln Val
   485 490 495
   Pro Ser Gln Asp Cys Val Trp
   500

## Description

### [Technical Field]

The present invention relates to a use of an antibody binding to phospholipase D4. Hereinafter, "phospholipase D" may be abbreviated as PLD and "phospholipase D4" and the like may be abbreviated as PLD4 and the like.

### [Background Art]

PLD is an enzyme which catalyzes a reaction to produce phosphatidic acid and choline by hydrolyzing phosphatidyl choline and causes various intracellular signaling. It has been believed that the produced phosphatidic acid functions as a lipid signal molecule.

PLD1 and PLD2 have been known as two types of mammal PLD, which have been previously known, and contain a phosphatidyl inositide-binding Phox homology domain (PX domain) and a phosphatidyl inositide-binding pleckstrin homology domain (PH domain) in the N-terminal region thereof. Both domains are involved in membrane localization of PLD.

PLD1 and PLD2 further contain two His-x-Lys-x-x-x-x-Asp sequences (HKD motifs). The HKD motifs are essential domains for PLD activity.

Phosphatidic acid produced by PLD1 and PLD2 has been suggested to be involved in cytoskeleton reconstruction, exocytosis, phagocytosis, canceration, cell adhesion, chemotaxis and the like, and mainly acts on nervous systems, immune systems and the like.

Human Hu-K4 and mouse SAM9, which are now officially named PLD3, lack the PX and PH domains and do not show PLD activity despite having two HKD motifs . Although there are further three PLD family members, PLD4, PLD5 and PLD6, little has been known about these non-classical PLDs.

As a result of searching a gene expression pattern in mouse cerebellar development in Cerebellar Development Transcriptome Database (CDT-DB), a transcription product, PLD4, controlled during the development was identified (see Non Patent Literature 1). Basic characteristics of PLD4 have not been reported. Enzymatic activity of PLD4 with or without glycosylation needs to be determined.

PLD4 has a 506 amino acid sequence shown in SEQ ID NO: 1 and is encoded by a cDNA base sequence of SEQ ID NO: 44 (Non Patent Literatures 1 and 2). The PLD4 protein has two tentative PDE regions (phosphodiesterase motifs) constituted of two HKD motifs (His-x-Lys-x-x-x-x-Asp amino acid sequence, x represents other amino acids) conserved in the C-terminal region, and a putative phosphorylation site (Thr 472). The structure of the PLD4 protein is estimated as a type II transmembrane protein. In addition, PLD4 does not have PX and PH domains, which PLD1 and PLD2 in a classical PLD family have them, in the N-terminal region.

On the other hand, PLD4 belongs to the PLD family because of having two HKD motifs, but lacks the PX domain and the PH domain and has a putative transmembrane domain instead (Non Patent Literature 3).

The expression of PLD4 mRNA has been found at low to medium levels in small cell clusters preferentially localized around white matter regions including corpus callosum and cerebellar white matter of 1 week old mice. These PLD4 mRNA-expressing cells have been identified as Iba1-positive microglia (Non Patent Literature 3). However, the PLD4-positive cells in mouse cerebellum is dispersed 10-day-old mice. It suggested that PLD4 expression is temporarily restricted during early postnatal development in mouse cerebellum.

Myelin formation in mouse begins in the corpora callosa and the cerebellar white matter at one week after birth. At this time, PLD4 is highly expressed in amoeboid (an activated state) microglia existing in the white matter, and thus it has been also believed that there is a possibility that PLD4-expressing cells in the white matter in this time are involved in myelin formation. In particular, it has also been revealed that PLD4 accumulates in food vacuoles, and it has been suggested that there is a possibility that PLD4 is involved in phagocytosis. In amoeboid microglia which is in an activated state, various cytokines and growth factors are secreted and simultaneously phagocytosis is activated. It has been believed that in the brain white matter of mouse in a developmental period, surplus oligodendrocytes (central nervous system glial cells, which form myelin by wrapping around axons) undergo apoptosis. There is a possibility that the oligodendrocytes are decomposed and removed in amoeboid microglia to secrete signal molecules and thereby adjust a myelin-forming environment in the white matter. It has been suggested that PLD4 is involved in these processes including the myelin formation.

Expression of mouse PLD4 mRNA is also observed in non-neuronal tissues and mainly distributed in the spleen. Strong expression of PLD4 protein is detected around a marginal zone of the splenic red pulp, and splenic PLD4 protein collected from subcellular membrane fractions is highly N-glycosylated. When PLD4 was expressed in a heterologous cell system, PLD4 was localized in the endoplasmic reticulum and Golgi apparatus. The heterologously expressed PLD4 did not show PLD enzyme activity (Non Patent Literature 3).

From the expression pattern of PLD4, which is spatiotemporally restricted, it has been suggested that PLD4 may play a role in common functions among the microglia and splenic marginal zone cells during early postnatal brain development.

On the other hand, the present inventors have found that PLD4 is specifically highly expressed in pDC (plasmacytoid Dendritic Cell) in a resting period (resting pDC) (Patent Literature 1). The present inventors further have reported that a PLD4-specific antibody can be utilized for suppression of pDC activity.

Further, PLD4 has been reported as one of novel susceptibility genes of Systemic Sclerosis in Japanese (Non Patent Literature 4). As a result of the same analysis in Europe, however, significant correlation with PLD4 has not been found and strong results showing a relationship between PLD4 and autoimmune diseases such as Systemic Sclerosis have not been obtained.

An immune mechanism is roughly classified into two groups. One is "natural immunity (innate immunity)" which detects foreign substances such as pathogens and carries out an initial attack, and the other is "acquired immunity" through information exchange which is presentation of antigen peptides and the like derived from foreign substances. Neutrophils, macrophage, dendritic cells (DC), NK (Natural Killer) cells and the like are mainly involved in the "natural immunity", and T cells and B cells to which information of antigen peptides and the like presented by the above dendritic cells and the like is transmitted are involved in the "acquired immunity" . T cells activated by transmission of information of antigen peptides are capable of specifically recognizing and attacking pathogens in a direct manner as the cell-mediated immunity, and B cells activated in the same manner as above are capable of specific recognition and attack against pathogens in an indirect manner by producing antibodies (hormonal immunity).

In the "natural immunity", pathogen-associated molecular patterns (PAMPs) universally existing in pathogens (LPS, CpG DNA, lipoproteins, RNA etc.) are recognized through Toll-like receptors (TLR), and secretion of inflammatory cytokines is promoted via NF-kB, or secretion of interferon (IFN) is promoted via IRF (Interferon regulatory factor). TLR is roughly classified into two groups by subcellular localization sites: a group expressed on cell surfaces and a group expressed in endosomes and endoplasmic reticula (ER). In pDC, IRF7 is activated via TLR7 and TLR9 localized in endosomes and endoplasmic reticula to induce IFN-α production. The reason why these TLRs are not expressed on cell surfaces but in cells has been suggested to decrease a risk of onset of autoimmune diseases. TLR7 and TLR9 recognize single-stranded RNA and DNA respectively as a ligand. Not only foreign pathogenic bacteria but also hosts hold these nucleic acids, and thus it has been suggested that receptors, which recognize nucleic acids and activate immune cells, always induce the autoimmune diseases.

On the other hand, B cells (B lymphocytes) showing an important role in the "acquired immunity" are lymphocytes which express immunoglobulin Ig receptors on the surface thereof. B cells are produced from hematopoietic stem cells in the bone marrow, and are differentiated into pre-B cells and immature B cells, and then mature into naive B cells (mature, unprimed B cells). The naive B cells are activated by not only the stimulation through the above T cells but also the direct antigen stimulation, and further become antibody-producing cells by differentiation and proliferation to produce and secrete antibodies such as IgM, IgD, IgA, IgE, IgG (including subclasses such as IgG1, IgG2, IgG2b, IgG3 and the like). It has been known that in addition to B cell receptors (BCR) recognizing specific foreign antigens, the above TLRs are expressed in B cells . It has been previously known, for example, that LPS which has been known to cause the proliferation and antibody production of B cells is a ligand of TLR4 and the above TLR7 and TLR9 are also expressed in B cells. Such B cells have been suggested to have a possibility to induce not only the above autoimmune diseases but also allergic diseases due to the overreaction of the antibody-producing ability thereof.

IgG, immunoglobulin G, is an antibody isotype consisting of four peptide chains-two identical heavy chains and two identical light chains. IgG is produced by B cells and plays a critical role for adaptive immunity. Naive B cells which do not produce IgG, differentiate into plasmablasts, and eventually into plasma cells . Plasmablasts and plasma cells can produce a large amount of antibodies. Conventionally, myeloid dendritic cells (DCs) have been shown to trigger B cell growth and differentiation by stimulating with IL-12 and IL-6 and/or membrane molecules such as BAFF/APRIL (Non Patent Literatures 5, 6 and 7). In addition, plasmacytoid DCs (pDCs) induce maturation and differentiation of naive B cells into antibody-secreting plasmablasts and plasma cells producing IFN-α and IL-6 (Non Patent Literature 8). The variable region of IgG captures various pathogens such as viruses, bacteria, and fungi, resulting in protection of the body from such infections.

SLE is regarded as a classic immune complex-mediated autoimmune disease. Immune complexes (ICs) are formed in circulation or in situ as a result of produced auto-antibodies against nucleic acids and their associated proteins, such as dsDNA, ribonucleoprotein, and histone. Such ICs cause inflammation with disease-characteristic clinical symptoms such as nephritis, arthritis, skin rashes, and vasculitis. Blood from SLE patient is characterized by reduction of naive B cells and increased memory B cells, plasmablasts and plasma cells (Non Patent Literatures 9, 10 and 11). Therefore, suppression of differentiation into plasma cells and antibody production through manipulation of auto-reactive antibody-secreting plasmablasts would result in a promising strategy to cure autoimmune diseases.

In PBMCs, there are various subsets of B cells, such as naive B cells, memory B cells, and plasmablasts. Most of B cell subset in PBMCs is naive B cells. Naive B cells are the one who are not exposed by foreign antigen. Memory B cells are the one who are formed by primary infection and are critical in quick antibody-mediated immune response by differentiation into plasmablasts. Plasmablasts are the one who secrete a large amount of antibody and marked by CD19+CD27+IgD-CD38+.

Once exposed by foreign antigen, naive B cells become activated B cells. The activated B cells are further differentiated in to memory B cell and/ or also plasmablasts that secrete antibodies. This change is called "maturation".

B cell maturation occurs in multiple phases. The initial, antigen-independent phase induces mature B cells that can bind to a unique antigen. This stage of maturation happens in the bone marrow and the spleen in living body. The antigen-dependent phase of B cell maturation happens following B cell activation by antigen binding and co-stimulation. These signals promote B cell maturation into either memory B cells or antibody-secreting plasmablasts. The antigen-dependent phase of B cell maturation involves activated B cell proliferation, antibody affinity maturation, and antibody class switching. Those maturations occur in the germinal centers of secondary lymphoid tissues.

It has been reported that, in vitro experimental condition, pDCs induce the maturation of activated B cells into Ig-secreting plasmablasts through release of IFN-α and IL-6. CpG2216 activates pDCs to induce IFN-α production and B cells to initiate maturation. IFN-α from pDCs further supports maturation of activated B cells into plasmablasts in the presence of IL-6.

Patent Literature 2 discloses novel proteins and polynucleotides, and speculates as to the biological activities and uses of the proteins. The amino acid sequence of PLD4 and is disclosed.

### [Citation List]

### [Patent Literature]

[PTL 1] PCT/JP2013/052781 (published after the priority date of the present application as WO2013/115410, and citable under Article 54(3) EPC)
[PTL 2] WO 00/11015

### [Non Patent Literature]

[NPL 1] Tao et al., Nat. Methods 2(8), pp 591-598 (2005)
[NPL 2] Clark et al., Genome Res. 13(10), pp 2265-2270 (2003)
[NPL 3] Plos ONE www.plosone.org, November 2010, Volume 5, Issue 11, e13932
[NPL 4] ARTHRITIS & RHEUMATISM Vol. 65, No. 2, February 2013, pp 472-480
[NPL 5] Balazs et al., 2002, Immunity, 17, 341-352
[NPL 6] Litinskiy et al., 2002, Nat Immunol, 3, 822-829
[NPL 7] MacLennan and Vinuesa, 2002, Immunity, 17, 235-238
[NPL 8] Jego et al, 2003, Immunity, 19, 225-234
[NPL 9] Odendahl et al., 2000, JI, 165, 5970-5979
[NPL 10] Arce et al., 2001, JI, 167, 2361-2369
[NPL 11] Wei et al., 2007, JI, 178, 6624-6633

### [Summary]

### [Technical Problem]

A problem to be solved by the present invention is to regulate activated B cells using an antibody binding to PLD4 and to improve symptoms of diseases caused thereby.

### [Solution to Problem]

Through research on PLD4, the present inventors verified that in addition to pDC cells in a resting period which have been previously reported, PLD4 expression was also induced in activated B cells. The present inventors therefore examined influence of PLD4 antibodies on activated B cells. A method for producing and purifying anti-PLD4 antibodies is carried out by a method in Patent Literature 1.

That is, the present invention relates to a second use using anti-PLD4 antibodies described below.
(1) A monoclonal antibody binding or antibody fragment containing an antigen-binding region thereof that binds to phospholipase D4 (PLD4) protein for use as an activated B cell-suppressing medicament in preventing or treating an autoimmune disease caused by activated B cells or in preventing or treating an allergic disease.
(2) The monoclonal antibody or antibody fragment containing the antigen-binding region thereof for use according to (1) above, comprising
   (a) a sequence SYWMH (SEQ ID NO: 2) as CDR1, a sequence DIYPGSDSTNYNEKFKS (SEQ ID NO: 3) as CDR2 and GGWLDAMDY (SEQ ID NO: 4) as a sequence CDR3 in a variable region of a heavy chain and a sequence RASQDISNYLN (SEQ ID NO: 5) as CDR1, a sequence YTSRLHS (SEQ ID NO: 6) as CDR2 and a sequence QQGNTLPW (SEQ ID NO: 7) as CDR3 in a variable region of a light chain;
   (b) a sequence TYWMH (SEQ ID NO: 8) as CDR1, a sequence AIYPGNSETSYNQKFKG (SEQ ID NO: 9) as CDR2 and GYSDFDY (SEQ ID NO: 10) as a sequence CDR3 in the variable region of the heavy chain and a sequence HASQGIRSNIG (SEQ ID NO: 11) as CDR1, a sequence HGTNLED (SEQ ID NO: 12) as CDR2 and a sequence VQYVQFP (SEQ ID NO: 13) as CDR3 in the variable region of the light chain;
   (c) a sequence DYNLH (SEQ ID NO: 14) as CDR1, a sequence YIYPYNGNTGYNQKFKR (SEQ ID NO: 15) as CDR2 and GGIYDDYYDYAIDY (SEQ ID NO: 16) as a sequence CDR3 in the variable region of the heavy chain and a sequence RASENIYSHIA (SEQ ID NO: 17) as CDR1, a sequence GATNLAH (SEQ ID NO: 18) as CDR2 and a sequence QHFWGTP (SEQ ID NO: 19) as CDR3 in the variable region of the light chain;
   (d) a sequence SYYLY (SEQ ID NO: 20) as CDR1, a sequence LINPTNSDTIFNEKFKS (SEQ ID NO: 21) as CDR2 and EGGYGYGPFAY (SEQ ID NO: 22) as a sequence CDR3 in the variable region of the heavy chain and a sequence TSSQTLVHSNGNTYLH (SEQ ID NO: 23) as CDR1, a sequence KVSNRFS (SEQ ID NO: 24) as CDR2 and a sequence HSTHVP (SEQ ID NO: 25) as CDR3 in the variable region of the light chain;
   (e) a sequence SYGMS (SEQ ID NO: 26) as CDR1, a sequence TISSGGSYIYYPESVKG (SEQ ID NO: 27) as CDR2 and LYGGRRGYGLDY (SEQ ID NO: 28) as a sequence CDR3 in the variable region of the heavy chain and a sequence RSSKSLLHSDGITYLY (SEQ ID NO: 29) as CDR1, a sequence QMSNLAS (SEQ ID NO: 30) as CDR2 and a sequence AQNLEL (SEQ ID NO: 31) as CDR3 in the variable region of the light chain;
   (f) a sequence SHYYWT (SEQ ID NO: 32) as CDR1, a sequence YISYDGSNNYNPSLKN (SEQ ID NO: 33) as CDR2 and EGPLYYGNPYWYFDV (SEQ ID NO: 34) as a sequence CDR3 in the variable region of the heavy chain and a sequence RASQDIDNYLN (SEQ ID NO: 35) as CDR1, a sequence YTSRLHS (SEQ ID NO: 36) as CDR2 and a sequence QQFNTLP (SEQ ID NO: 37) as CDR3 in the variable region of the light chain;
   (g) a sequence SHYYWS (SEQ ID NO: 38) as CDR1, a sequence YISYDGSNNYNPSLKN (SEQ ID NO: 39) as CDR2 and EGPLYYGNPYWYFDV (SEQ ID NO: 40) as a sequence CDR3 in the variable region of the heavy chain and a sequence RASQDIDNYLN (SEQ ID NO: 41) as CDR1, a sequence YTSRLHS (SEQ ID NO: 42) as CDR2 and a sequence QQFNTLP (SEQ ID NO: 43) as CDR3 in the variable region of the light chain.
(3) The monoclonal antibody or antibody fragment containing the antigen-binding region thereof for use according to (1) above, comprising
   (a) a heavy chain variable region set forth in amino acids 36 to 153 of SEQ ID: 75 and a light chain variable region set forth in amino acids 21 to 127 of SEQ ID: 95;
   (b) a heavy chain variable region set forth in amino acids 20 to 135 of SEQ ID: 77 and a light chain variable region set forth in amino acids 21 to 127 of SEQ ID: 97;
   (c) a heavy chain variable region set forth in amino acids 20 to 142 of SEQ ID: 79 and a light chain variable region set forth in amino acids 21 to 127 of SEQ ID: 99;
   (d) a heavy chain variable region set forth in amino acids 20 to 142 of SEQ ID: 81 and a light chain variable region set forth in amino acids 21 to 127 of SEQ ID: 101;
   (e) a heavy chain variable region set forth in amino acids 20 to 139 of SEQ ID: 83 and a light chain variable region set forth in amino acids 20 to 131 of SEQ ID: 103;
   (f) a heavy chain variable region set forth in amino acids 20 to 140 of SEQ ID: 85 and a light chain variable region set forth in amino acids 21 to 131 of SEQ ID: 105;
   (g) a heavy chain variable region set forth in amino acids 20 to 140 of SEQ ID: 87 and a light chain variable region set forth in amino acids 21 to 131 of SEQ ID: 107;
   (h) a heavy chain variable region set forth in amino acids 19 to 142 of SEQ ID: 89 and a light chain variable region set forth in amino acids 21 to 127 of SEQ ID: 109;
   (i) a heavy chain variable region set forth in amino acids 19 to 142 of SEQ ID: 91 and a light chain variable region set forth in amino acids 21 to 127 of SEQ ID: 111; or
   (j) a heavy chain variable region set forth in amino acids 19 to 142 of SEQ ID: 93 and a light chain variable region set forth in amino acids 21 to 127 of SEQ ID: 113.
(4) A monoclonal antibody or antibody fragment containing the antigen-binding region thereof for use according to (1) above, wherein the antibody is produced by any of hybridomas mp5B7, mp7B4, mp13D4 and mp13H11 of Deposit Nos. NITE BP-1211, NITE BP-1212, NITE BP-1213 and NITE BP-1214, or a fragment containing an antigen-binding region thereof as an active ingredient.
(5) The monoclonal antibody or antibody fragment containing the antigen-binding region thereof for use according to (1) above, which is chimerized or humanized.
(6) The monoclonal antibody or antibody fragment containing the antigen-binding region thereof for use according to (1) above, comprising a heavy chain set forth in SEQ ID: 121 and a light chain set forth in SEQ ID: 123.
(7) An *in vitro* method for detecting activated B cells, the method including (a) a step of bringing a monoclonal antibody binding to an extracellular domain of PLD4 or an antibody fragment containing an antigen-binding region thereof into contact with cells to be tested; and (b) a step of detecting the monoclonal antibody or the antibody fragment containing the antigen-binding region thereof which binds to the cells.
(8) Use of a monoclonal antibody binding to an extracellular domain of PLD4 or an antibody fragment containing an antibody-binding region thereof to detect activated B cells *in vitro.*
(9) An *in vitro* method for suppressing an activity of activated B cells, the method including a step of bringing either of the following components into contact with activated B cells:
   (a) a monoclonal antibody or an antibody fragment containing an antigen-binding region thereof which binds to PLD4 and suppresses the activity of activated B cells or has cytotoxic activity against activated B cells, and
   (b) immunoglobulin into which a complementarity-determining regions of the monoclonal antibody in (a) are grafted, or an antibody fragment containing an antigen-binding region thereof, wherein the activity of the activated B cells is antibody production or maturation into antibody-secreting cells.
(10) A monoclonal antibody or antibody fragment containing an antigen-binding region thereof or an immunoglobulin for use as an activated B cell-suppressing medicament in preventing or treating an autoimmune disease caused by activated B cells or in preventing or treating an allergic disease, wherein:
   (a) the monoclonal antibody or antibody fragment containing an antigen-binding region thereof binds to PLD4 and suppresses an activity of activated B cells or has cytotoxic activity against activated B cells, and
   (b) the immunoglobulin is an immunoglobulin into which a complementarity-determining regions of the monoclonal antibody in (a) are grafted, or an antibody fragment containing an antigen-binding region thereof, wherein the activity of the activated B cells is an antibody production or maturation into antibody-secreting cells.

The "activated B cells" may include B cells possessing the activity of proliferation and antibody production and secretion by not only direct stimulation through BCR and TLR but also stimulation through T cells.

The "fragment containing an antigen-binding region" may include Fab, Fab', F(ab')₂ fragments and the like obtained by partial digestion with papain or pepsin, but is not limited thereto. In addition, the fragment containing an antigen-binding region also may include a fragment of immunoglobulin containing a variable region into which CDR (complementarily-determining region) of a monoclonal antibody is grafted. It is well known that these antibody fragments can be used as antibody molecules having binding affinity to antigens. Alternatively, insofar as required antigen-binding activity is maintained, antibodies constructed by gene recombination can be used. Examples of antibodies constructed by gene recombination can include chimeric antibodies, CDR-grafted antibodies, single chain Fv (scFv), diabody (diabodies), linear antibodies, and polyspecific antibodies formed from antibody fragments and the like. A method for obtaining these antibodies based on monoclonal antibodies or antibody-producing cells producing the monoclonal antibodies is known.

The "autoimmune diseases" are diseases which are caused by attacks of immune functions by misunderstanding one's own body tissues as foreign substances. Organ-specific autoimmune diseases include Guillain-Barre syndrome, myasthenia gravis, chronic gastritis (chronic atrophic gastritis), autoimmune hepatitis, primary biliary cirrhosis, primary sclerosing cholangitis, autoimmune pancreatitis, aortitis syndrome, Goodpasture syndrome, rapidly progressive glomerulonephritis, megaloblastic anemia, autoimmune hemolytic anemia, autoimmune neutropenia, idiopathic thrombocytopenic purpura, Basedow disease, Hashimoto thyroiditis, primary hypothyroidism, idiopathic Addison's disease, type 1 diabetes, ulcerative colitis, Crohn's disease, celiac disease and the like; and systemic autoimmune diseases include articular rheumatism, systemic lupus erythematosus, anti-phospholipid antibody syndrome, polymyositis, scleroderma, Sjogren's syndrome, vasculitis syndrome, autoimmune lymphoproliferative syndrome (ALPS) and the like, but are not limited thereto.

The "allergic diseases" are diseases caused by abnormal immune reactions against foreign substances, and include atopic dermatitis, bronchial asthma, pollinosis, allergic rhinitis, urticaria, infantile asthma, allergic gastroenteritis, contact dermatitis, serum sickness, vascular purpura and the like but are not limited thereto.

### [Advantageous Effects of Invention]

The present invention provides a therapeutic method attributable to suppression of activated B cells using an antibody specifically recognizing PLD4 and a fragment thereof, and a medicament having its therapeutic effect.

The present invention can be further expected to have preventive and therapeutic effects on patients with autoimmune diseases or allergic diseases by using the activated B cell-suppressing activity.

### [Brief Description of Drawings]

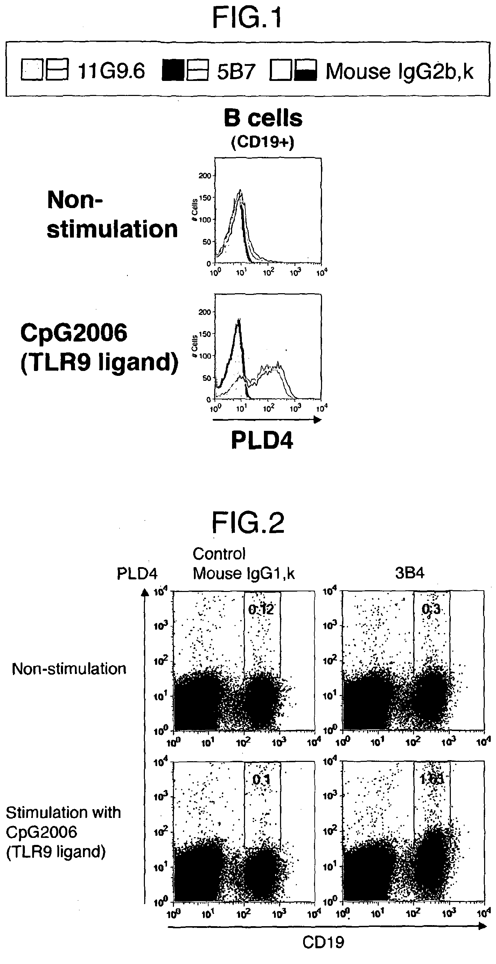

Fig. 1 is a FACS analysis diagram which shows staining of human B cells (CD19+) with anti-PLD4 antibodies. PLD4 protein was induced on CD19+ B cells by stimulation with TLR9 ligand, CpG2006. Induction of PLD4 in activated B cells (CD19+) could be detected by a TLR9 ligand (CpG2006). Monoclonal antibodies 11G9.6 and 5B7 were used to detect PLD4. Mouse IgG2b, κ was used as a negative control.
Fig. 2 is a FACS analysis diagram which shows staining of human PBMC with an anti-PLD4 antibody and an anti-CD19 antibody. PLD4+ cells were increased in activated B cells (CD19+) by stimulation with TLR9 ligand. Mouse IgG1, κ was used as a negative control.

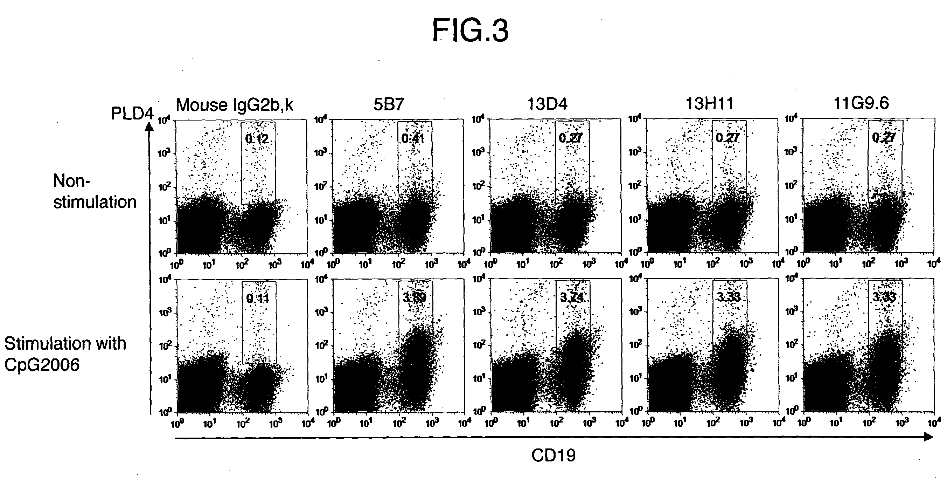

Fig. 3 is a FACS analysis diagram which shows staining of human PBMC with anti-PLD4 antibodies and an anti-CD19 antibody in the presence or absence of TLR9 ligand stimulation. A significant increase of PLD4+ TLR9 ligand-stimulated B cells (CD19+) could be detected with anti-PLD4 antibodies (5B7, 13D4, 13H11 and 11G9.6). Mouse IgG2b, κ was used as a negative control.

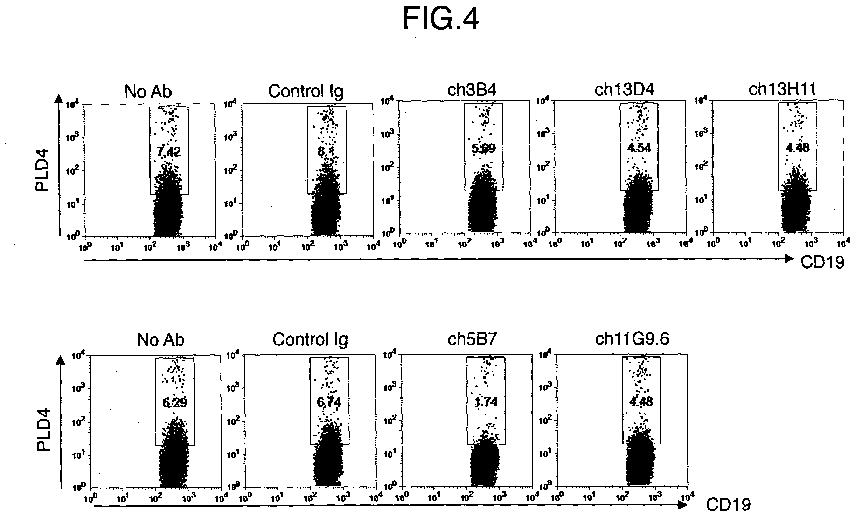

Fig. 4 is a FACS analysis diagram which shows reduction of PLD4+ activated B cells by the indicated each anti-PLD4 chimeric antibody. Co-culture of PBMCs with the anti-PLD4 chimeric antibodies (ch3B4, ch13D4, ch13H11, ch5B7 and ch11G9.6) reduced PLD4+ activated B cells in the presence of TLR9 ligand. In a case in which an antibody was not added (NoAb) and a case in which a non-specific antibody was used (Control Ig), however, the activation of B cells by adding CpG2006 could not be suppressed.

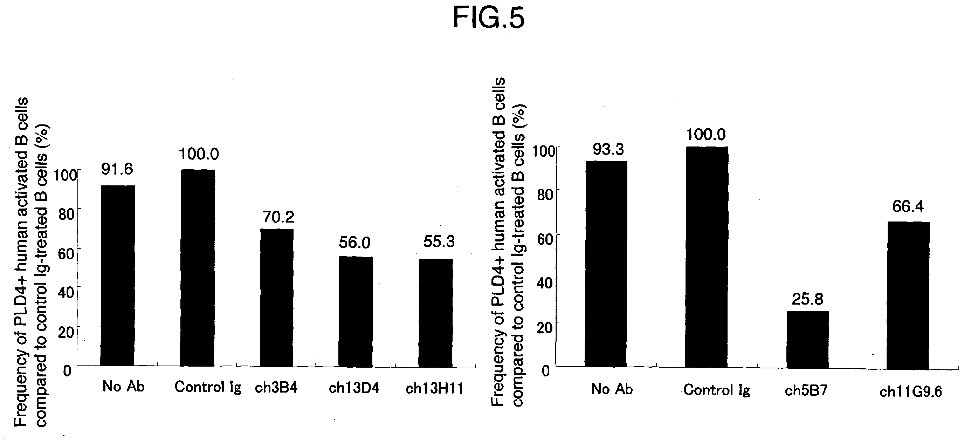

Fig. 5 is a diagram in which suppressive effect in Fig. 4 is expressed in numbers. An activated B cell group which expresses PLD4 and was treated with control Ig is considered as 100% and changes in an activated B cell group which expresses PLD4 and was treated with each anti-PLD4 chimeric antibody are shown.

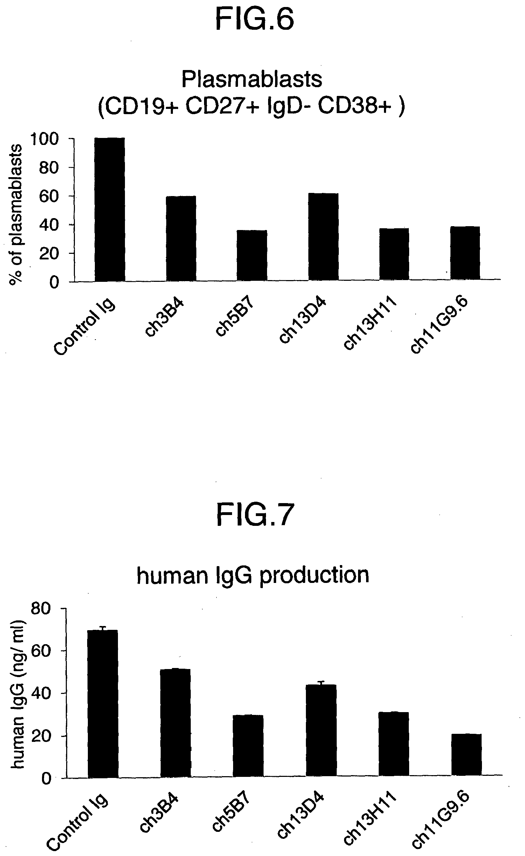

Fig.6 is a result of flow cytometry. PBMCs were cultured with the indicated chimeric PLD4 antibodies in the presence of TLR9 ligand and recombinant human IL-6. Plasmablast population (CD19+CD27+IgD-CD38+) was reduced by the treatment with ch3B4, ch5B7, ch13D4, ch13H11, or ch11G9.6 compared with control Ig treatment.
Fig. 7 is a result of ELISA assay of the culture supernatant of Fig. 6. Human IgG production from plasmablasts was reduced by the treatment with ch3B4, ch5B7, ch13D4, ch13H11, or ch11G9.6 compared with control Ig treatment.

### [Description of Embodiments]

The present inventors newly found that PLD4 was a molecule whose expression is induced with activation of B cells.

The present inventors have previously reported expression, subcellular localization, structure and function of human PLD4 (Patent Literature 1). In the present invention, it further turned out that the expression of PLD4 is induced in not only pDC but also activated B cells . It was further newly found that anti-PLD4 antibodies suppressed activated B cells. Such findings not only strengthen a possibility that anti-PLD4 antibodies have a therapeutic effect on autoimmune diseases by suppression of pDC activity, which has been previously reported, but also B cell activity.

Proteins such as CD19, CD20, CD22 and BAFF-R are expressed on the surface of B cells. CD19 is expressed on B cells from an early stage such as pro-B cells to antibody-secreting plasma cells, and functions as an auxiliary receptor controlling activation in mature B cells. CD20 is expressed from pre B cells to activated B cells, CD22 is expressed on the cell surface of mature B cells, and the expression of BAFF-R is observed in the extensive differentiation stage of B cells. Therefore, there is concern that antibodies recognizing these proteins suppress not only activated B cells but also unprimed naive B cells . The anti-PLD4 antibodies of the present invention are however characterized by suppressing activated B cells without influence on naive B cells.

The anti-PLD4 antibodies used in the present invention are the same as those reported previously (Patent Literature 1). In short, using as an immunogen a recombinant PLD4-Ig fusion protein encoding an amino acid sequence containing an extracellular domain of PLD4 (the amino acid sequence corresponding to from position 54 to 506 in the amino acid sequence shown in SEQ ID NO: 1), an antibody against PLD4 was obtained as follows.

### <Creation of anti-human PLD4 monoclonal antibodies>

### 1) Immunization

As an immunogen, the above recombinant PLD4-Ig fusion protein was used. The PLD4-Ig fusion protein was administered to the dorsal hypodermis of three BALB/c mice. As adjuvants, Freund's Adjuvants, Complete and Incomplete (SIGMA), were used. The volume of first administration was 200 µg/mouse, and the volume of second to fourth administration was 50 µg/mouse.

### 2) Confirmation of anti-serum titer

Blood was collected after third and fourth immunization and anti-serum titer was evaluated by ELISA.

The PLD4-Ig fusion protein was transformed into a solid phase on a 96 well microtiter plate. An antiserum was serially diluted in 3-fold increments from 1000-fold and a dilution series up to 729000-fold was prepared. To the antigen-coated plate, each 50 µl of each sample was added and a first-order reaction was carried out. After washing, a second-order reaction was carried out with the HRP-labeled anti-mouse IgG (κ, λ) antibody and color development was detected with OPD (orthophenylene diamine) (490 nm).

### 3) Cell fusion

Splenic cells were extracted from mice in which an increase in anti-serum titer was observed. The extracted splenic cells and mouse myeloma cells (P3U1) were fused by the PEG method and the fused splenic cells were selectively cultured in an HAT medium.

### <FACS screening of hybridomas using CAL-1 cells>

An antibody produced from each clone of the fused splenic cells obtained by HAT selective culture was evaluated by FACS. Consequently, 3B4, 5B7, 7B4, 8C11, 10C3, 11D10, 13D4, 13H11, 14C1 and 11G9.6 in hybridoma culture supernatant well reacted to human PLD4.

In each monoclonal antibody produced from the above hybridomas, CDR regions (CDRs; CDR1, CDR2 and CDR3) and FW regions (Frame work regions) in a variable region and a sequence of the variable region were determined according to an analytical method of Kabat numbering system (Kabat et al, 1991, Sequences of Proteins of Immunological Interest, National Institutes of Health Publication No. 91-3242, 5th ed., United States Department of Health and Human Services, Bethesda, MD) .

The nucleic acid sequence of the heavy chain variable region of the obtained mouse 11G9.6 antibody is SEQ ID NO: 74, and the amino acid sequence is SEQ ID NO: 75. The amino acid sequences of CDR1, CDR2 and CDR3 within the heavy chain variable region of the mouse 11G9.6 antibody are SEQ ID NO: 2, SEQ ID NO: 3 and SEQ ID NO: 4, respectively.

The nucleic acid sequence of the heavy chain variable region of the obtained mouse 3B4 antibody is SEQ ID NO: 76, and the amino acid sequence is SEQ ID NO: 77. The amino acid sequences of CDR1, CDR2 and CDR3 within the heavy chain variable region of the mouse 3B4 antibody are SEQ ID NO: 8, SEQ ID NO: 9 and SEQ ID NO: 10, respectively.

The nucleic acid sequence of the heavy chain variable region of the obtained mouse 5B7 antibody is SEQ ID NO: 78, and the amino acid sequence is SEQ ID NO: 79. The amino acid sequences of CDR1, CDR2 and CDR3 within the heavy chain variable region of the mouse 5B7 antibody are SEQ ID NO: 14, SEQ ID NO: 15 and SEQ ID NO: 16, respectively.

The nucleic acid sequence of the heavy chain variable region of the obtained mouse 7B4 antibody is SEQ ID NO: 80, and the amino acid sequence is SEQ ID NO: 81. The amino acid sequences of CDR1, CDR2 and CDR3 within the heavy chain variable region of the mouse 7B4 antibody are SEQ ID NO: 14, SEQ ID NO: 15 and SEQ ID NO: 16, respectively. The 7B4 antibody is an antibody which has the same CDR sequences in the variable regions of the heavy and light chains as of the 5B7 antibody.

The nucleic acid sequence of the heavy chain variable region of the obtained mouse 8C11 antibody is SEQ ID NO: 82, and the amino acid sequence is SEQ ID NO: 83. The amino acid sequences of CDR1, CDR2 and CDR3 within the heavy chain variable region of the mouse 8C11 antibody are SEQ ID NO: 20, SEQ ID NO: 21 and SEQ ID NO: 22, respectively.

The nucleic acid sequence of the heavy chain variable region of the obtained mouse 10C3 antibody is SEQ ID NO: 84, and the amino acid sequence is SEQ ID NO: 85. The amino acid sequences of CDR1, CDR2 and CDR3 within the heavy chain variable region of the mouse 10C3 antibody are SEQ ID NO: 26, SEQ ID NO: 27 and SEQ ID NO: 28, respectively.

The nucleic acid sequence of the heavy chain variable region of the obtained mouse 11D10 antibody is SEQ ID NO: 86, and the amino acid sequence is SEQ ID NO: 87. The amino acid sequences of CDR1, CDR2 and CDR3 within the heavy chain variable region of the mouse 11D10 antibody are SEQ ID NO: 26, SEQ ID NO: 27 and SEQ ID NO: 28, respectively. The 11D10 antibody is an antibody which has the same CDR sequences in the variable regions of the heavy and light chains as of the 10C3 antibody. Their heavy chain isotypes are, however, different (10C3 has the constant region of mouse IgG2a and 11D10 has the constant region of mouse IgG2b).

The nucleic acid sequence of the heavy chain variable region of the obtained mouse 13D4 antibody is SEQ ID NO: 88, and the amino acid sequence is SEQ ID NO: 89. The amino acid sequences of CDR1, CDR2 and CDR3 within the heavy chain variable region of the mouse 13D4 antibody are SEQ ID NO: 32, SEQ ID NO: 33 and SEQ ID NO: 34, respectively.

The nucleic acid sequence of the heavy chain variable region of the obtained mouse 13H11 antibody is SEQ ID NO: 90, and the amino acid sequence is SEQ ID NO: 91. The amino acid sequences of CDR1, CDR2 and CDR3 within the heavy chain variable region of the mouse 13H11 antibody are SEQ ID NO: 38, SEQ ID NO: 39 and SEQ ID NO: 40, respectively.

The nucleic acid sequence of the heavy chain variable region of the obtained mouse 14C1 antibody is SEQ ID NO: 92, and the amino acid sequence is SEQ ID NO: 93. The amino acid sequences of CDR1, CDR2 and CDR3 within the heavy chain variable region of the mouse 14C1 antibody are SEQ ID NO: 38, SEQ ID NO: 39 and SEQ ID NO: 40, respectively. The 14C1 antibody is an antibody which has the same CDR sequences in the variable regions of the heavy and light chains as of the 13H11 antibody. Their heavy chain isotypes are, however, different (13H11 has the constant region of mouse IgG2b and 14C1 has the constant region of mouse IgG1).

The nucleic acid sequence of the light chain variable region of the mouse 11G9.6 antibody is SEQ ID NO: 94, and the amino acid sequence is SEQ ID NO: 95. The amino acid sequences of CDR1, CDR2 and CDR3 within the light chain variable region of the mouse 11G9.6 antibody are SEQ ID NO: 5, SEQ ID NO: 6 and SEQ ID NO: 7, respectively.

The nucleic acid sequence of the light chain variable region of the mouse 3B4 antibody is SEQ ID NO: 96, and the amino acid sequence is SEQ ID NO: 97. The amino acid sequences of CDR1, CDR2 and CDR3 within the light chain variable region of the mouse 3B4 antibody are SEQ ID NO: 11, SEQ ID NO: 12 and SEQ ID NO: 13, respectively.

The nucleic acid sequence of the light chain variable region of the mouse 5B7 antibody is SEQ ID NO: 98, and the amino acid sequence is SEQ ID NO: 99. The amino acid sequences of CDR1, CDR2 and CDR3 within the light chain variable region of the mouse 5B7 antibody are SEQ ID NO: 17, SEQ ID NO: 18 and SEQ ID NO: 19, respectively.

The nucleic acid sequence of the light chain variable region of the mouse 7B4 antibody is SEQ ID NO: 100, and the amino acid sequence is SEQ ID NO: 101. The amino acid sequences of CDR1, CDR2 and CDR3 within the light chain variable region of the mouse 7B4 antibody are SEQ ID NO: 17, SEQ ID NO: 18 and SEQ ID NO: 19, respectively.

The nucleic acid sequence of the light chain variable region of the mouse 8C11 antibody is SEQ ID NO: 102, and the amino acid sequence is SEQ ID NO: 103. The amino acid sequences of CDR1, CDR2 and CDR3 within the light chain variable region of the mouse 8C11 antibody are SEQ ID NO: 23, SEQ ID NO: 24 and SEQ ID NO: 25, respectively.

The nucleic acid sequence of the light chain variable region of the mouse 10C3 antibody is SEQ ID NO: 104, and the amino acid sequence is SEQ ID NO: 105. The amino acid sequences of CDR1, CDR2 and CDR3 within the light chain variable region of the mouse 10C3 antibody are SEQ ID NO: 29, SEQ ID NO: 30 and SEQ ID NO: 31, respectively.

The nucleic acid sequence of the light chain variable region of the mouse 11D10 antibody is SEQ ID NO: 106, and the amino acid sequence is SEQ ID NO: 107. The amino acid sequences of CDR1, CDR2 and CDR3 within the light chain variable region of the mouse 11D10 antibody are SEQ ID NO: 29, SEQ ID NO: 30 and SEQ ID NO: 31, respectively.

The nucleic acid sequence of the light chain variable region of the mouse 13D4 antibody is SEQ ID NO: 108, and the amino acid sequence is SEQ ID NO: 109. The amino acid sequences of CDR1, CDR2 and CDR3 within the light chain variable region of the mouse 13D4 antibody are SEQ ID NO: 35, SEQ ID NO: 36 and SEQ ID NO: 37, respectively.

The nucleic acid sequence of the light chain variable region of the mouse 13H11 antibody is SEQ ID NO: 100, and the amino acid sequence is SEQ ID NO: 111. The amino acid sequences of CDR1, CDR2 and CDR3 within the light chain variable region of the mouse 13H11 antibody are SEQ ID NO: 41, SEQ ID NO: 42 and SEQ ID NO: 43, respectively.

The nucleic acid sequence of the light chain variable region of the mouse 14C1 antibody is SEQ ID NO: 112, and the amino acid sequence is SEQ ID NO: 113. The amino acid sequences of CDR1, CDR2 and CDR3 within the light chain variable region of the mouse 14C1 antibody are SEQ ID NO: 41, SEQ ID NO: 42 and SEQ ID NO: 43, respectively.

Examples of more preferred monoclonal antibodies in the present invention can include monoclonal antibodies produced by
hybridomas mp5B7, mp7B4, mp13D4 and mp13H11.
Hybridomas mp5B7, mp7B4, mp13D4 and mp13H11 were accepted by National Institute of Technology and Evaluation, International Patent Organism Depositary,
under accession No. NITE ABP-1211, NITE ABP-1212, NITE ABP-1213 and NITE ABP-1214
as of January 27, 2012. The details specifying the deposition will be described as follows.
(1) Name and Address of Depositary Authority
   Name: National Institute of Technology and Evaluation, Advanced Industrial Science and Technology, International Patent Organism Depositary
   Address: 2-5-8 Kazusa Kamatari Kisarazu-shi, Chiba Ibaraki, 292-0818, Japan
(2) Deposit Date: January 27, 2012
(3) Deposit number NITE BP-1211 (hybridoma mp5B7)
   NITE BP-1212 (hybridoma mp7B4)
   NITE BP-1213 (hybridoma mp13D4)
   NITE BP-1214 (hybridoma mp13H11)

In particular, more preferred antibodies are an antibody having a combination of
the heavy chain CDR1: DYNLH, CDR2: YIYPYNGNTGYNQKFKR, and CDR3: GGIYDDYYDYAIDY, and
the light chain CDR1: RASENIYSHIA, CDR2: GATNLAH, and CDR3: QHFWGTP
as the sequences of CDRs constituting its variable regions; an antibody having a combination of
the heavy chain CDR1: SHYYWT, CDR2: YISYDGSNNYNPSLKN, and CDR3: EGPLYYGNPYWYFDV, and
the light chain CDR1: RASQDIDNYLN, CDR2: YTSRLHS, and CDR3: QQFNTLP
as the sequences of CDRs constituting its variable regions; and an antibody having a combination of
the heavy chain CDR1: SHYYWS, CDR2: YISYDGSNNYNPSLKN, and CDR3: EGPLYYGNPYWYFDV, and
the light chain CDR1: RASQDIDNYLN, CDR2: YTSRLHS, and CDR3: QQFNTLP,
as the sequences of CDRs constituting its variable regions.

A chimeric antibody or a humanized antibody recognizing PLD4 can be produced by genetic engineering using a polynucleotide encoding it. As described in Patent Document 1, for example, each active chimeric antibody (ch3B4Ab, ch5B7Ab, ch7B4Ab, ch8C11Ab, ch10C3Ab, ch11D10Ab, ch13D4Ab, ch13H11Ab, ch14C1Ab, ch11G9.6Ab etc.) can be easily produced using each CDR region of the above mouse monoclonal antibodies (3B4, 5B7, 7B4, 8C11, 10C3, 11D10, 13D4, 13H11, 14C1, 11G9.6etc.) by those of skill in the art.

The present inventors have verified that monoclonal antibodies against PLD4 have CDC (Complement Dependent Cytotoxicity) activity and ADCC (Antibody-dependent cellular cytotoxicity) activity against the PLD4-expressing cells. Therefore, the anti-PLD4 monoclonal antibodies according to the present invention have cytotoxicity action against PLD4-expressing cells.

That is, the present invention relates to an agent for suppressing activated B cells, wherein the agent comprises an antibody binding to an extracellular domain of PLD4 as an active component. Alternatively, the present invention provides a method for suppressing antibody production, the method including a step of administering an antibody binding to an extracellular domain of PLD4. The present invention further relates to use of an antibody binding to an extracellular domain of PLD4 in production of a pharmaceutical composition for suppressing activated B cells.

In the present invention, an antibody modified as needed can be used. According to the present invention, an antibody recognizing the extracellular domain of PLD4 has the activated B cell-suppressing action. That is, it has been believed that there is a possibility that an antibody itself have cytotoxicity action against activated B cells. The subclass of an antibody showing intense effector action is known. Alternatively, suppressive effect on activated B cells can be further increased by modifying an antibody with a cytotoxic agent. As the cytotoxic agents, the following substances can be mentioned. Toxins: Pseudomonas Endotoxin (PE), diphtheria toxin, lysine Radioisotopes: Tc99m, Sr89, 1131, Y90
Anticancer agents: calicheamicin, mitomycin, paclitaxel

The toxins containing proteins can be bound to an antibody or a fragment thereof or the like by a bifunctional reagent. Alternatively, by conjugating a gene encoding an antibody with a gene encoding a toxin, a fusion protein of the two can be also obtained. A method for binding a radioisotope to an antibody is also known. A method for labeling an antibody with a radioisotope, for example, using a chelating agent is known. Further, an anticancer agent can be bound to an antibody, using glycan or a bifunctional reagent or the like.

In the present invention, an antibody whose structure is artificially modified can be used as an active component. For example, various modification methods for improving the cytotoxicity action and stability of antibodies are known. Concretely, immunoglobulin in which the glycan of its heavy chain is modified is known (Shinkawa, T. et al. J. Biol. Chem. 278:3466-3473. 2003). By modification of glycan, the ADCC (Antibody Dependent Cell-mediated Cytotoxicity) activity of immunoglobulin was increased.

In the present invention, one or more monoclonal antibodies can be used. For example, several types of monoclonal antibodies recognizing the extracellular domain of PLD4 can be combined and used for the present invention.

As described below, it can be verified that anti-PLD4 antibodies have suppressive action on the acquired immune antibody-producing activity of activated B cells. B cells produce a large amount of antibodies by stimulation of a BCR ligand or a TLR ligand (preferably TLR4 ligand, TLR7 ligand or TLR9 ligand). An anti-PLD4 antibody is provided before and after stimulation of the above ligand on B cells or simultaneously with stimulation of the ligand, and using B cells for which an anti-PLD4 antibody is not provided as a control, ability to produce acquired immune antibodies derived from B cells is compared. The antibody-producing ability can be evaluated by measuring secretory immunoglobulin contained in a culture supernatant of B cells. As a result of the comparison, when the amount of the acquired immune antibody derived from B cells in the supernatant significantly declines by adding an anti-PLD4 antibody, it can be verified that the tested anti-PLD4 antibody has suppressive action on the antibody-producing ability of B cells. A method for measuring the antibodies is known. B cells are cells which produce hormonal immunity (secretory antibody) in a living body. Therefore, hormonal immunity can be adjusted by suppressing the antibody-producing ability of B cells.

When an antibody recognizing the extracellular domain of PLD4 is administered to a host different from an organism species from which the antibody is derived, it is desired to process into a form which is difficult to be recognized as a foreign substance by such a host. By processing into molecules described below, for example, it can be difficult that immunoglobulin is recognized as a foreign substance. Techniques for processing immunoglobulin molecules as described below are known:
- a fragment containing an antigen-binding region which lacks a constant region (Monoclonal Antibodies: Principles and Practice. third edition, Academic Press Limited. 1995; Antibody Engineering, A Practical Approach, IRL PRESS, 1996);
- a chimeric antibody constituted of an antigen-binding region of a monoclonal antibody and a constant region of host immunoglobulin (Experimental manual for genetic expression, Kodansha Ltd. 1994 (edited by Isao Ishida and Tamie Ando)) ; and
- a CDR-substituted antibody in which a complementarity-determining region (CDR) in host immunoglobulin is substituted by the CDR of a monoclonal antibody (Experimental manual for genetic expression, Kodansha Ltd. 1994 (edited by Isao Ishida and Tamie Ando)).

Alternatively, a variable region gene of human immunoglobulin can be also obtained by the phage display method (McCafferty J. et al., Nature 348:552-554, 1990; Kretzschmar T et. al., Curr Opin Biotechnol. 2002 Dec; 13(6):598-602.). In the phage display method, a gene encoding a variable region of human immunoglobulin is incorporated into a phage gene. Using various types of immunoglobulin genes as sources, a phage library can be also created. A phage expresses such a variable region as a fusion protein of a protein constructing the phage itself. The variable region expressed by the phage on the phage surface maintains binding activity to antigens. Therefore, by selecting a phage binding to an antigen or cells expressing the antigen or the like, a phage expressing a variable region having target binding activity can be screened from a phage library. Further, a gene encoding a variable region having target binding activity is maintained in the phage particle selected as above. That is, in the phage display method, using the binding activity of a variable region as an index, a gene encoding a variable region having target binding activity can be obtained.

In the agent for suppressing B cell activity or the method for suppressing B cell activity according to the present invention, an antibody recognizing the extracellular domain of PLD4 or an antibody fragment containing at least the antigen-binding region thereof can be administered as a protein or a polynucleotide encoding it. In order to administer a polynucleotide, it is desired that a vector in which a polynucleotide encoding a target protein is arranged be used under control of a proper promoter so that the target protein can be expressed. In a vector, an enhancer and a terminator can be also arranged. Vectors which maintain the genes of heavy and light chains constituting immunoglobulin and in which an immunoglobulin molecule can be expressed are known. A vector in which immunoglobulin can be expressed can be administered by introduction into cells . For administration to a living body, a vector which can infect cells by administration to the living body can be directly administered. Alternatively, a vector is introduced into a lymphocyte separated from a living body and then the vector can be returned into the living body (ex vivo) .

In the agent for suppressing B cell activity or the method for suppressing B cell activity based on the present invention, the amount of monoclonal antibody to be administered to a living body is normally 0.5 mg to 10 mg, for example 1 mg to 50 mg, preferably 2 mg to 10 mg as immunoglobulin per kg of body weight. An interval of administration of an antibody to a living body can be properly adjusted in order that an effective concentration of immunoglobulin in the living body during treatment period can be maintained. Concretely, for example, an antibody can be administered at intervals of 1 to 2 weeks. Any administration route can be used. Those of skill in the art can properly select an effective administration route for treatment. Concretely, oral or parenteral administration can be mentioned. By an intravenous injection, an intramuscular injection, an intraperitoneal injection or a subcutaneous injection or the like, for example, an antibody can be systemically or locally administered. The formulations suitable for parenteral administration in the present invention include injections, suppositories, sprays and the like. In addition, when provided to cells, immunoglobulin is provided in a culture fluid in an amount of normally 1 µg/ml, preferably 10 µg/mL or more, more preferably 50 µg/mL or more, and further preferably 0.5 mg/mL or more.

In the agent for suppressing B cell activity or the method for suppressing B cell activity based on the present invention, a monoclonal antibody can be administered to a living body by any method. A monoclonal antibody is normally combined with a pharmaceutically acceptable carrier. A monoclonal antibody can be combined with additives as needed, such as a thickener, a stabilizer, an antiseptic and a solubilizing agent. Such carriers or additives include lactose, a citric acid, a stearic acid, magnesium stearate, sucrose, starch, talc, gelatin, agar, plant oil, ethylene glycol and the like. The term "pharmaceutically acceptable" means to be approved by government authorities of various countries, or that its use for animals, mammals and, in particular, human is listed in pharmacopoeias of various countries or pharmacopoeias commonly acknowledged. The agent for suppressing B cell activity in the present invention can be also supplied in the form of freeze-drying powders or tablets at one or more doses . Further, sterilized water for injections, a physiological salt solution or a buffer solution, which are used for dissolution, can be combined with freeze-drying powders or tablets in order that the composition will obtain a desired concentration before administration.

Further, for administration as a vector expressing immunoglobulin, a heavy chain and a light chain are cotransfected as different plasmids and each plasmid can be administered at 0.1 to 10 mg, for example 1 to 5 mg per kg of body weight. In addition, 1 to 5 µg vectors/10⁶ cells are used to introduce into cells in vitro. The present invention will be now described in more detail by way of examples.

The present invention will be now described in more detail by way of examples. It should be noted, however, that the present invention is not limited to the examples.

### [Examples]

### Example 1

Human PBMC (1 × 10⁷ cells/ml) was stimulated by CpG2006, a ligand of TLR9, (a final concentration of 1 µM) and incubated in a 24 well plate in a CO₂ incubator (37°C, 5% CO₂) for about 20 hours. In parallel, human PBMC (1 × 10⁷ cells/ml) which was not stimulated was also cultured in a CO₂ incubator (37°C, 5% CO₂) for about 20 hours.

Human PBMC was treated with FcR Blocking Reagent (Miltenyi), which was diluted 5-fold with FACS buffer (1% FBS/PBS), at 4°C for 20 minutes. After washing, staining was carried out with 5B7, 11G9.6 or mouse IgG2b, κ, a primary antibody, (each 10 µg/ml) at 4°C for 15 minutes. A secondary antibody and subsequent antibodies were diluted with FACS buffer so that FcR Blocking Reagent would be diluted 25-fold. PE-labeled anti-mouse Ig (BD), a secondary antibody, was diluted 100-fold and the solution was added thereto and mixed. Besides, to fractionate B cells on FACS, an APC-labeled anti-human CD 19 antibody (Biolegend) was diluted 30-fold with FACS buffer containing FcR Blocking Reagent and staining was carried out at 4°C for 15 minutes. Using FACS Calibur (BD), data was incorporated. Living cells were gated on a dot plot of the X axis: FSC and the Y axis: SSC. Data was incorporated until the number of cells in the living cell gate became 100,000 counts. B cells: anti-marker molecule antibody-positive cells were gated. The gated cells were analyzed on the histogram with the X axis: PLD4, and the results of staining with mouse IgG2b, κ were overlaid thereon. Consequently, anti-PLD4 antibodies were hardly bound to non-stimulated, but were selectively bound to activated B cells by stimulation with TLR9 ligand (Fig. 1) . This shows that PLD4 is expressed on activated B cells.

### Example 2

### <Binding test to B cells by each monoclonal antibody>

Human PBMC was stimulated with CpG2006 with a final concentration of 1 µM for about 20 hours. Cells were collected and treated with FcR Blocking Reagent at 4°C for 20 minutes. After washing, staining was carried out with each 10 µg/ml of 3B4, 5B7, 13D4, 13H11, 11G9.6, mouse IgG1, κ or mouse IgG2b, κ, a primary antibody, at 4°C for 15 minutes. Staining was carried out with PE-labeled anti-mouse Ig, a secondary antibody, at 4°C for 15 minutes. For gating of a B cell group, double staining was carried out with an APC-labeled anti-human CD19 antibody at 4°C for 15 minutes. A living cell group on the dot plot of the X axis: FSC and the Y axis: SSC was analyzed by binding of anti-PLD4 antibody to CD19+ B cells (Fig. 2 and Fig.3). Consequently, all of the tested anti-PLD4 monoclonal antibodies were bound to B cells stimulated by TLR9. That is, it was verified that by all anti-PLD4 monoclonal antibodies, expression of PLD4 was induced in B cells in an activation-dependent manner.

### Example 3

### <Cytotoxic activity of anti-PLD4 chimeric antibodies against activated B cells>

Frequency of PLD4+ activated B cells induced by stimulation with TLR9 ligand (1 µM) was used as an index. Human PBMC was cultured with CpG2006 and each anti-PLD4 chimeric antibody or control Ig for about 16 hours. As a medium, RPM11640 (SIGMA) was used (including 10% FBS (Equitech-bio), 5 ml of 200 mML-Glutamine (GIBCO), 5 ml of Pen-Strep (GIBCO), 5 ml of Sodium Pyruvate (GIBCO), and 0.5 ml of 50 mM 2-ME (SIGMA)). The cells were collected and treated with FcR Blocking Reagent at 4°C for 20 minutes. After washing, the cells were further stained by 5B7 or 13D4, 3B4 or mouse IgG2b, κ, a primary antibody, at 4°C for 15 minutes (each 10 µg/ml). A sample in which PBMC was treated with a chimeric 3B4 antibody (ch3B4), a chimeric 3D4 antibody (ch3D4), or a chimeric 13H11 antibody (ch13H11) was stained with 5B7, and a sample in which PBMC was treated with a chimeric 5B7 antibody (ch5B7) or a chimeric 11G9.6 antibody (ch11G9.6) was stained with 13D4. It has been verified that an anti-PLD4 antibody clone treated for ADCC and an anti-PLD4 antibody clone used for staining do not compete with each other. The binding of the anti-PLD4 was found by PE-labeled anti-mouse Ig, a secondary antibody, at 4°C for 15 minutes. For gating of B cells, double staining was carried out with an APC-labeled anti-human CD19 antibody at 4°C for 15 minutes (Fig. 4). The population of PLD4 + activated B cells treated with each chimeric anti-PLD4 antibody was compared with that of PLD4 + activated B cells treated with the control antibody (Fig. 5). Consequently, all of the chimeric anti-PLD4 antibodies reduced activated PLD4 + B cells compared to the treatment with control Ig (when a case of treating with control Ig was considered as 100%, ch3B4: 70.2%, ch13D4: 56.0%, ch13H11: 55.3%, ch5B7: 25.8%, ch11G9.6: 66.4%).

### Example 4

### <Inhibitory effects of the chimeric anti-PLD4 antibodies against activated B cells>

To determine the effect of anti-human PLD4 antibody on B cells maturation and Ig production through B cell activation, whole human PBMCs were treated with ch3B4, ch5B7, ch13D4, ch13H11, ch11G9.6, or control Ig for 24 h. Then, the PBMCs were further cultured in the presence of CpG2216 (1 µM) and recombinant human IL-6 to induce B cell activation, resulting in B cell maturation. In the result of culture of activated B cells for 7 days, Plasmablasts, CD19+CD27+IgD-CD38+, in the activated B cells was analyzed by flow cytometry with a PE-labeled anti-human CD19 antibody. In order to measure human IgG production, the cultured activated B cells were re-stimulated with 50 ng/ ml of PMA (Phorbol myristate acetate) after washed with PBS 2 times. Two days later, human IgG production was measured in the culture supernatants by ELISA. Plasmablasts in the activated B cells were reduced by the treatment with ch3B4, ch5B7, ch13D4, ch13H11, or ch11G9.6 compared with control Ig treatment (Figure 6) . Also, human IgG production was reduced by the treatment with ch3B4, ch5B7, ch13D4, ch13H11, or ch11G9.6 compared to control Ig treatment (Figure 7) . These results indicated that the treatment with the chimeric anti-human PLD4 Abs reduced Ab-secreting activated human B cells.

### [Industrial Applicability]

As shown in the above examples, anti-PLD4 antibodies recognize and suppress activated B cells. Therefore, the antibodies are useful for prevention and treatment of diseases involved in immune function (autoimmune diseases and allergic diseases).

### <Explanation of sequence information of anti-PLD4 monoclonal antibodies according to the present invention>

### 1. Anti-PLD4 mouse 11G9.6 antibody

The nucleic acid sequence of the heavy chain variable region of the obtained anti-PLD4 mouse 11G9.6 antibody is SEQ ID NO: 74, and the amino acid sequence is SEQ ID NO: 75. The amino acid sequences of CDR1, CDR2 and CDR3 within the heavy chain variable region of the mouse 11G9.6 antibody are SEQ ID NO: 2, SEQ ID NO: 3 and SEQ ID NO: 4, respectively.

The nucleic acid sequence of the heavy chain variable region of the anti-PLD4 mouse 11G9.6 antibody (504 bp) [capital letters: mouse 11G9.6 VH variable region, small letters: mouse IgG2b heavy chain constant region] (SEQ ID NO: 74)

The amino acid sequence of the heavy chain variable region of the mouse 11G9.6 antibody (168 a. a.) [capital letters: mouse 11G9.6 VH variable region, small letters: mouse IgG2b heavy chain constant region] The underlined sequence shows its signal sequence and the double underline shows its CDR regions (CDR1, CDR2 and CDR3) (SEQ ID NO: 75).
CDR1 in the heavy chain variable region of the 11G9.6 antibody (SEQ ID NO: 2)
CDR2 in the heavy chain variable region of the 11G9.6 antibody (SEQ ID NO: 3)
CDR3 in the heavy chain variable region of the 11G9.6 antibody (SEQ ID NO: 4)

The nucleic acid sequence of the light chain variable region of the obtained anti-PLD4 mouse 11G9.6 antibody is SEQ ID NO: 94, and the amino acid sequence is SEQ ID NO: 95. The amino acid sequences of CDR1, CDR2 and CDR3 within the light chain variable region of the mouse 11G9.6 antibody are SEQ ID NO: 5, SEQ ID NO: 6 and SEQ ID NO: 7, respectively.

The nucleic acid sequence of the light chain variable region of the anti-PLD4 mouse 11G9.6 antibody (421 bp) [capital letters: mouse 11G9.6 VL variable region, small letters: mouse Ig κ light chain constant region] (SEQ ID NO: 94)

The amino acid sequence of the light chain variable region of the mouse 11G9.6 antibody (140 a.a.) [capital letters: mouse 11G9.6 VL variable region, small letters: mouse Ig κ light chain constant region] The underlined sequence shows its signal sequence and the double underline shows its CDR regions (CDR1, CDR2 and CDR3) (SEQ ID NO: 95).
CDR1 in the light chain variable region of the 11G9.6 antibody (SEQ ID NO: 5)
CDR2 in the light chain variable region of the 11G9.6 antibody (SEQ ID NO: 6)
CDR3 in the light chain variable region of the 11G9.6 antibody (SEQ ID NO: 7)

### 2. Anti-PLD4 mouse 3B4 antibody

The nucleic acid sequence of the heavy chain variable region of the obtained anti-PLD4 mouse 3B4 antibody is SEQ ID NO: 76, and the amino acid sequence is SEQ ID NO: 77. The amino acid sequences of CDR1, CDR2 and CDR3 within the heavy chain variable region of the mouse 3B4 antibody are SEQ ID NO: 8, SEQ ID NO: 9 and SEQ ID NO: 10, respectively.

The nucleic acid sequence of the heavy chain variable region of the anti-PLD4 mouse 3B4 antibody (437 bp) [capital letters : mouse 3B4 VH variable region, small letters : mouse IgG1 heavy chain constant region]

The amino acid sequence of the heavy chain variable region of the mouse 3B4 antibody (145 a. a.) [capital letters: mouse 3B4 VH variable region, small letters: mouse IgG1 heavy chain constant region] The underlined sequence shows its signal sequence and the double underline shows its CDR regions (CDR1, CDR2 and CDR3).
CDR1 in the heavy chain variable region of the 3B4 antibody TYWMH
CDR2 in the heavy chain variable region of the 3B4 antibody AIYPGNSETSYNQKFKG
CDR3 in the heavy chain variable region of the 3B4 antibody GYSDFDY

The nucleic acid sequence of the light chain variable region of the obtained anti-PLD4 mouse 3B4 antibody is SEQ ID NO: 96, and the amino acid sequence is SEQ ID NO: 97. The amino acid sequences of CDR1, CDR2 and CDR3 within the light chain variable region of the mouse 3B4 antibody are SEQ ID NO: 11, SEQ ID NO: 12 and SEQ ID NO: 13, respectively.

The nucleic acid sequence of the light chain variable region of the anti-PLD4 mouse 3B4 antibody (459 bp) [capital letters: mouse 3B4 VL variable region, small letters: mouse Ig κ light chain constant region]

The amino acid sequence of the light chain variable region of the mouse 3B4 antibody (153 a. a.) [capital letters: mouse 3B4 VL variable region, small letters: mouse Ig κ light chain constant region] The underlined sequence shows its signal sequence and the double underline shows its CDR regions (CDR1, CDR2 and CDR3).
CDR1 in the light chain variable region of the 3B4 antibody HASQGIRSNIG
CDR2 in the light chain variable region of the 3B4 antibody HGTNLED
CDR3 in the light chain variable region of the 3B4 antibody VQYVQFP

### 3. Anti-PLD4 mouse 5B7 antibody

The nucleic acid sequence of the heavy chain variable region of the obtained anti-PLD4 mouse 5B7 antibody is SEQ ID NO: 78, and the amino acid sequence is SEQ ID NO: 79. The amino acid sequences of CDR1, CDR2 and CDR3 within the heavy chain variable region of the mouse 5B7 antibody are SEQ ID NO: 14, SEQ ID NO: 15 and SEQ ID NO: 16, respectively.

The nucleic acid sequence of the heavy chain variable region of the anti-PLD4 mouse 5B7 antibody (475 bp) [capital letters: mouse 5B7 VH variable region, small letters: mouse IgG2b heavy chain constant region]

The amino acid sequence of the heavy chain variable region of the mouse 5B7 antibody (158 a. a.) [capital letters: mouse 5B7 VH variable region, small letters: mouse IgG2b heavy chain constant region] The underlined sequence shows its signal sequence and the double underline shows its CDR regions (CDR1, CDR2 and CDR3).
CDR1 in the heavy chain variable region of the 5B7 antibody DYNLH
CDR2 in the heavy chain variable region of the 5B7 antibody YIYPYNGNTGYNQKFKR
CDR3 in the heavy chain variable region of the 5B7 antibody GGIYDDYYDYAIDY

The nucleic acid sequence of the light chain variable region of the obtained anti-PLD4 mouse 5B7 antibody is SEQ ID NO: 98, and the amino acid sequence is SEQ ID NO: 99. The amino acid sequences of CDR1, CDR2 and CDR3 within the light chain variable region of the mouse 5B7 antibody are SEQ ID NO: 17, SEQ ID NO: 18 and SEQ ID NO: 19, respectively.

The nucleic acid sequence of the light chain variable region of the anti-PLD4 mouse 5B7 antibody (467 bp) [capital letters: mouse 5B7 VL variable region, small letters: mouse Ig κ light chain constant region]

The amino acid sequence of the light chain variable region of the mouse 5B7 antibody (155 a. a.) [capital letters: mouse 5B7 VL variable region, small letters: mouse Ig κ light chain constant region] The underlined sequence shows its signal sequence and the double underline shows its CDR regions (CDR1, CDR2 and CDR3).
CDR1 in the light chain variable region of the 5B7 antibody RASENIYSHIA
CDR2 in the light chain variable region of the 5B7 antibody GATNLAH
CDR3 in the light chain variable region of the 5B7 antibody QHFWGTP

### 4. Anti-PLD4 mouse 7B4 antibody

The nucleic acid sequence of the heavy chain variable region of the obtained anti-PLD4 mouse 7B4 antibody is SEQ ID NO: 80, and the amino acid sequence is SEQ ID NO: 81. The amino acid sequences of CDR1, CDR2 and CDR3 within the heavy chain variable region of the mouse 7B4 antibody are SEQ ID NO: 14, SEQ ID NO: 15 and SEQ ID NO: 16, respectively.

The nucleic acid sequence of the heavy chain variable region of the anti-PLD4 mouse 7B4 antibody (470 bp) [capital letters: mouse 7B4 VH variable region, small letters: mouse IgG2b heavy chain constant region]

The amino acid sequence of the heavy chain variable region of the mouse 7B4 antibody (156 a. a.) [capital letters: mouse 7B4 VH variable region, small letters: mouse IgG2b heavy chain constant region] The underlined sequence shows its signal sequence and the double underline shows its CDR regions (CDR1, CDR2 and CDR3).
CDR1 in the heavy chain variable region of the 7B4 antibody DYNLH
CDR2 in the heavy chain variable region of the 7B4 antibody YIYPYNGNTGYNQKFKR
CDR3 in the heavy chain variable region of the 7B4 antibody GGIYDDYYDYAIDY

The nucleic acid sequence of the light chain variable region of the obtained anti-PLD4 mouse 7B4 antibody is SEQ ID NO: 100, and the amino acid sequence is SEQ ID NO: 101. The amino acid sequences of CDR1, CDR2 and CDR3 within the light chain variable region of the mouse 7B4 antibody are SEQ ID NO: 17, SEQ ID NO: 18 and SEQ ID NO: 19, respectively.

The nucleic acid sequence of the light chain variable region of the anti-PLD4 mouse 7B4 antibody (454 bp) [capital letters: mouse 7B4 VL variable region, small letters: mouse Ig κ light chain constant region]

The amino acid sequence of the light chain variable region of the mouse 7B4 antibody (151 a. a.) [capital letters: mouse 7B4 VL variable region, small letters: mouse Ig κ light chain constant region] The underlined sequence shows its signal sequence and the double underline shows its CDR regions (CDR1, CDR2 and CDR3).
CDR1 in the light chain variable region of the 7B4 antibody RASENIYSHIA
CDR2 in the light chain variable region of the 7B4 antibody GATNLAH
CDR3 in the light chain variable region of the 7B4 antibody QHFWGTP

### 5. Anti-PLD4 mouse 8C11 antibody

The nucleic acid sequence of the heavy chain variable region of the obtained anti-PLD4 mouse 8C11 antibody is SEQ ID NO: 82, and the amino acid sequence is SEQ ID NO: 83. The amino acid sequences of CDR1, CDR2 and CDR3 within the heavy chain variable region of the mouse 8C11 antibody are SEQ ID NO: 20, SEQ ID NO: 21 and SEQ ID NO: 22, respectively.

The nucleic acid sequence of the heavy chain variable region of the anti-PLD4 mouse 8C11 antibody (462 bp) [capital letters: mouse 8C11 VH variable region, small letters: mouse IgG2b heavy chain constant region]

The amino acid sequence of the heavy chain variable region of the mouse 8C11 antibody (154 a. a.) [capital letters: mouse 8C11 VH variable region, small letters: mouse IgG2b heavy chain constant region] The underlined sequence shows its signal sequence and the double underline shows its CDR regions (CDR1, CDR2 and CDR3).
CDR1 in the heavy chain variable region of the 8C11 antibody SYYLY
CDR2 in the heavy chain variable region of the 8C11 antibody LINPTNSDTIFNEKFKS
CDR3 in the heavy chain variable region of the 8C11 antibody EGGYGYGPFAY

The nucleic acid sequence of the light chain variable region of the obtained anti-PLD4 mouse 8C11 antibody is SEQ ID NO: 102, and the amino acid sequence is SEQ ID NO: 103. The amino acid sequences of CDR1, CDR2 and CDR3 within the light chain variable region of the mouse 8C11 antibody are SEQ ID NO: 23, SEQ ID NO: 24 and SEQ ID NO: 25, respectively.

The nucleic acid sequence of the light chain variable region of the anti-PLD4 mouse 8C11 antibody (457 bp) [capital letters: mouse 8C11 VL variable region, small letters: mouse Ig κ light chain constant region]

The amino acid sequence of the light chain variable region of the mouse 8C11 antibody (152 a. a.) [capital letters: mouse 8C11 VL variable region, small letters: mouse Ig κ light chain constant region] The underlined sequence shows its signal sequence and the double underline shows its CDR regions (CDR1, CDR2 and CDR3).
CDR1 in the light chain variable region of the 8C11 antibody TSSQTLVHSNGNTYLH
CDR2 in the light chain variable region of the 8C11 antibody KVSNRFS
CDR3 in the light chain variable region of the 8C11 antibody HSTHVP

### 6. Anti-PLD4 mouse 10C3 antibody

The nucleic acid sequence of the heavy chain variable region of the obtained anti-PLD4 mouse 10C3 antibody is SEQ ID NO: 84, and the amino acid sequence is SEQ ID NO: 85. The amino acid sequences of CDR1, CDR2 and CDR3 within the heavy chain variable region of the mouse 10C3 antibody are SEQ ID NO: 26, SEQ ID NO: 27 and SEQ ID NO: 28, respectively.

The nucleic acid sequence of the heavy chain variable region of the anti-PLD4 mouse 10C3 antibody (450 bp) [capital letters: mouse 10C3 VH variable region, small letters: mouse IgG2a heavy chain constant region]

The amino acid sequence of the heavy chain variable region of the mouse 10C3 antibody (150 a. a.) [capital letters: mouse 10C3 VH variable region, small letters: mouse IgG2a heavy chain constant region] The underlined sequence shows its signal sequence and the double underline shows its CDR regions (CDR1, CDR2 and CDR3).
CDR1 in the heavy chain variable region of the 10C3 antibody SYGMS
CDR2 in the heavy chain variable region of the 10C3 antibody TISSGGSYIYYPESVKG
CDR3 in the heavy chain variable region of the 10C3 antibody LYGGRRGYGLDY

The nucleic acid sequence of the light chain variable region of the obtained anti-PLD4 mouse 10C3 antibody is SEQ ID NO: 104, and the amino acid sequence is SEQ ID NO: 105. The amino acid sequences of CDR1, CDR2 and CDR3 within the light chain variable region of the mouse 10C3 antibody are SEQ ID NO: 29, SEQ ID NO: 30 and SEQ ID NO: 31, respectively.

The nucleic acid sequence of the light chain variable region of the anti-PLD4 mouse 10C3 antibody (423 bp) [capital letters: mouse 10C3 VL variable region, small letters: mouse Ig κ light chain constant region]

The amino acid sequence of the light chain variable region of the mouse 10C3 antibody (141 a. a.) [capital letters: mouse 10C3 VL variable region, small letters: mouse Ig κ light chain constant region] The underlined sequence shows its signal sequence and the double underline shows its CDR regions (CDR1, CDR2 and CDR3).
CDR1 in the light chain variable region of the 10C3 antibody RSSKSLLHSDGITYLY
CDR2 in the light chain variable region of the 10C3 antibody QMSNLAS
CDR3 in the light chain variable region of the 10C3 antibody AQNLEL

### 7. Anti-PLD4 mouse 11D10 antibody

The nucleic acid sequence of the heavy chain variable region of the obtained anti-PLD4 mouse 11D10 antibody is SEQ ID NO: 86, and the amino acid sequence is SEQ ID NO: 87. The amino acid sequences of CDR1, CDR2 and CDR3 within the heavy chain variable region of the mouse 11D10 antibody are SEQ ID NO: 26, SEQ ID NO: 27 and SEQ ID NO: 28, respectively.

The nucleic acid sequence of the heavy chain variable region of the anti-PLD4 mouse 11D10 antibody (450 bp) [capital letters: mouse 11D10 VH variable region, small letters: mouse IgG2b heavy chain constant region]

The amino acid sequence of the heavy chain variable region of the mouse 11D10 antibody (150 a. a.) [capital letters: mouse 11D10 VH variable region, small letters : mouse IgG2b heavy chain constant region] The underlined sequence shows its signal sequence and the double underline shows its CDR regions (CDR1, CDR2 and CDR3).
CDR1 in the heavy chain variable region of the 11D10 antibody SYGMS
CDR2 in the heavy chain variable region of the 11D10 antibody TISSGGSYIYYPESVKG
CDR3 in the heavy chain variable region of the 11D10 antibody LYGGRRGYGLDY

The nucleic acid sequence of the light chain variable region of the obtained anti-PLD4 mouse 11D10 antibody is SEQ ID NO: 106, and the amino acid sequence is SEQ ID NO: 107. The amino acid sequences of CDR1, CDR2 and CDR3 within the light chain variable region of the mouse 11D10 antibody are SEQ ID NO: 29, SEQ ID NO: 30 and SEQ ID NO: 31, respectively.

The nucleic acid sequence of the light chain variable region of the anti-PLD4 mouse 11D10 antibody (423 bp) [capital letters: mouse 11D10 VL variable region, small letters: mouse Ig κ light chain constant region]

The amino acid sequence of the light chain variable region of the mouse 11D10 antibody (141 a. a.) [capital letters: mouse 11D10 VL variable region, small letters: mouse Ig κ light chain constant region] The underlined sequence shows its signal sequence and the double underline shows its CDR regions (CDR1, CDR2 and CDR3).
CDR1 in the light chain variable region of the 11D10 antibody RSSKSLLHSDGITYLY
CDR2 in the light chain variable region of the 11D10 antibody QMSNLAS
CDR3 in the light chain variable region of the 11D10 antibody AQNLEL

### 8. Anti-PLD4 mouse 13D4 antibody

The nucleic acid sequence of the heavy chain variable region of the obtained anti-PLD4 mouse 13D4 antibody is SEQ ID NO: 88, and the amino acid sequence is SEQ ID NO: 89. The amino acid sequences of CDR1, CDR2 and CDR3 within the heavy chain variable region of the mouse 13D4 antibody are SEQ ID NO: 32, SEQ ID NO: 33 and SEQ ID NO: 34, respectively.

The nucleic acid sequence of the heavy chain variable region of the anti-PLD4 mouse 13D4 antibody (472 bp) [capital letters: mouse 13D4 VH variable region, small letters: mouse IgG2b heavy chain constant region]

The amino acid sequence of the heavy chain variable region of the mouse 13D4 antibody (157 a. a.) [capital letters: mouse 13D4 VH variable region, small letters: mouse IgG2b heavy chain constant region] The underlined sequence shows its signal sequence and the double underline shows its CDR regions (CDR1, CDR2 and CDR3).
CDR1 in the heavy chain variable region of the 13D4 antibody SHYYWT
CDR2 in the heavy chain variable region of the 13D4 antibody YISYDGSNNYNPSLKN
CDR3 in the heavy chain variable region of the 13D4 antibody EGPLYYGNPYWYFDV

The nucleic acid sequence of the light chain variable region of the obtained anti-PLD4 mouse 13D4 antibody is SEQ ID NO: 108, and the amino acid sequence is SEQ ID NO: 109. The amino acid sequences of CDR1, CDR2 and CDR3 within the light chain variable region of the mouse 13D4 antibody are SEQ ID NO: 35, SEQ ID NO: 36 and SEQ ID NO: 37, respectively.

The nucleic acid sequence of the light chain variable region of the anti-PLD4 mouse 13D4 antibody (404 bp) [capital letters: mouse 13D4 VL variable region, small letters: mouse Ig κ light chain constant region]

The amino acid sequence of the light chain variable region of the mouse 13D4 antibody (134 a. a.) [capital letters: mouse 13D4 VL variable region, small letters: mouse Ig κ light chain constant region] The underlined sequence shows its signal sequence and the double underline shows its CDR regions (CDR1, CDR2 and CDR3).
CDR1 in the light chain variable region of the 13D4 antibody RASQDIDNYLN
CDR2 in the light chain variable region of the 13D4 antibody YTSRLHS
CDR3 in the light chain variable region of the 13D4 antibody QQFNTLP

### 9. Anti-PLD4 mouse 13H11 antibody

The nucleic acid sequence of the heavy chain variable region of the obtained anti-PLD4 mouse 13H11 antibody is SEQ ID NO: 90, and the amino acid sequence is SEQ ID NO: 91. The amino acid sequences of CDR1, CDR2 and CDR3 within the heavy chain variable region of the mouse 13H11 antibody are SEQ ID NO: 38, SEQ ID NO: 39 and SEQ ID NO: 40, respectively.

The nucleic acid sequence of the heavy chain variable region of the anti-PLD4 mouse 13H11 antibody (471 bp) [capital letters: mouse 13H11 VH variable region, small letters: mouse IgG2b heavy chain constant region]

The amino acid sequence of the heavy chain variable region of the mouse 13H11 antibody (157 a. a.) [capital letters: mouse 13H11 VH variable region, small letters : mouse IgG2b heavy chain constant region] The underlined sequence shows its signal sequence and the double underline shows its CDR regions (CDR1, CDR2 and CDR3).
CDR1 in the heavy chain variable region of the 13H11 antibody SHYYWS
CDR2 in the heavy chain variable region of the 13H11 antibody YISYDGSNNYNPSLKN
CDR3 in the heavy chain variable region of the 13H11 antibody EGPLYYGNPYWYFDV

The nucleic acid sequence of the light chain variable region of the obtained anti-PLD4 mouse 13H11 antibody is SEQ ID NO: 110, and the amino acid sequence is SEQ ID NO: 111. The amino acid sequences of CDR1, CDR2 and CDR3 within the light chain variable region of the mouse 13H11 antibody are SEQ ID NO: 41, SEQ ID NO: 42 and SEQ ID NO: 43, respectively.

The nucleic acid sequence of the light chain variable region of the anti-PLD4 mouse 13H11 antibody (414 bp) [capital letters: mouse 13H11 VL variable region, small letters: mouse Ig κ light chain constant region]

The amino acid sequence of the light chain variable region of the mouse 13H11 antibody (138 a. a.) [capital letters: mouse 13H11 VL variable region, small letters: mouse Ig κ light chain constant region] The underlined sequence shows its signal sequence and the double underline shows its CDR regions (CDR1, CDR2 and CDR3).
CDR1 in the light chain variable region of the 13H11 antibody RASQDIDNYLN
CDR2 in the light chain variable region of the 13H11 antibody YTSRLHS
CDR3 in the light chain variable region of the 13H11 antibody QQFNTLP

### 10. Anti-PLD4 mouse 14C1 antibody

The nucleic acid sequence of the heavy chain variable region of the obtained anti-PLD4 mouse 14C1 antibody is SEQ ID NO: 92, and the amino acid sequence is SEQ ID NO: 93. The amino acid sequences of CDR1, CDR2 and CDR3 within the heavy chain variable region of the mouse 14C1 antibody are SEQ ID NO: 38, SEQ ID NO: 39 and SEQ ID NO: 40, respectively.

The nucleic acid sequence of the heavy chain variable region of the anti-PLD4 mouse 14C1 antibody (470 bp) [capital letters: mouse 14C1 VH variable region, small letters: mouse IgG1 heavy chain constant region]

The amino acid sequence of the heavy chain variable region of the mouse 14C1 antibody (156 a. a.) [capital letters: mouse 14C1 VH variable region, small letters: mouse IgG1 heavy chain constant region] The underlined sequence shows its signal sequence and the double underline shows its CDR regions (CDR1, CDR2 and CDR3).
CDR1 in the heavy chain variable region of the 14C1 antibody SHYYWS
CDR2 in the heavy chain variable region of the 14C1 antibody YISYDGSNNYNPSLKN
CDR3 in the heavy chain variable region of the 14C1 antibody EGPLYYGNPYWYFDV

The nucleic acid sequence of the light chain variable region of the obtained anti-PLD4 mouse 14C1 antibody is SEQ ID NO: 112, and the amino acid sequence is SEQ ID NO: 113. The amino acid sequences of CDR1, CDR2 and CDR3 within the light chain variable region of the mouse 14C1 antibody are SEQ ID NO: 41, SEQ ID NO: 42 and SEQ ID NO: 43, respectively.

The nucleic acid sequence of the light chain variable region of the anti-PLD4 mouse 14C1 antibody (465 bp) [capital letters: mouse 14C1 VL variable region, small letters: mouse Ig κ light chain constant region]

The amino acid sequence of the light chain variable region of the mouse 14C1 antibody (155 a. a.) [capital letters: mouse 14C1 VL variable region, small letters: mouse Ig κ light chain constant region] The underlined sequence shows its signal sequence and the double underline shows its CDR regions (CDR1, CDR2 and CDR3).
CDR1 in the light chain variable region of the 14C1 antibody RASQDIDNYLN
CDR2 in the light chain variable region of the 14C1 antibody YTSRLHS
CDR3 in the light chain variable region of the 14C1 antibody QQFNTLP

The base sequences and the amino acid sequences of the heavy chain and the light chain of the created chimeric 11G9.6 antibody are as the sequence numbers given below.

### Heavy chain

SEQ ID NO: 120 (base sequence)
SEQ ID NO: 121 (amino acid sequence)

### Light chain

SEQ ID NO: 122 (base sequence)
SEQ ID NO: 123 (amino acid sequence)

11. The nucleic acid sequence of the heavy chain of the anti-PLD4 chimeric 11G9.6 antibody (1401 bp) [capital letters: chimeric 11G9 VH variable region, small letters: human IgG1 heavy chain constant region] (SEQ ID NO: 120)
12. The amino acid sequence of the heavy chain of the anti-PLD4 chimeric 11G9.6 antibody (466 a. a.) [capital letters: chimeric 11G9 VH variable region, small letters: human IgG1 heavy chain constant region] (SEQ ID NO: 121)
13. The nucleic acid sequence of the light chain of the anti-PLD4 chimeric 11G9.6 antibody (705 bp) [capital letters: chimeric 11G9 VL variable region, small letters: human Ig κ light chain constant region] (SEQ ID NO: 122)
14. The amino acid sequence of the light chain of the anti-PLD4 chimeric 11G9.6 antibody (234 a. a.) [capital letters: chimeric 11G9 VL variable region, small letters: human Ig κ light chain constant region] (SEQ ID NO: 123)

### <cDNA and protein sequences of PLD4-related molecules>

> Human PLD4 cDNA (1521 bp) (SEQ ID NO: 44)
> Human PLD4 protein (506 amino acids) (SEQ ID NO: 1)
> Cynomolgus monkey PLD4 cDNA (1521 bp) (SEQ ID NO: 63)
> Cynomolgus monkey PLD4 protein (506 amino acids) (SEQ ID NO: 129)
> Rhesus monkey PLD4 cDNA (1521 bp) (SEQ ID NO: 124)
> Rhesus monkey PLD4 protein (506 amino acids) (SEQ ID NO: 130)
> Mouse PLD4 cDNA (1512 base pairs) (SEQ ID NO: 131)
> Mouse PLD4 protein (503 amino acids) (SEQ ID NO: 132)
> Human PLD3 cDNA sequence (SEQ ID NO: 55)
> Human PLD3 protein (490 amino acids) (SEQ ID NO: 127)
> Human PLD5 cDNA (1338 base pairs) (SEQ ID NO: 56)
> Human PLD5 protein (445 amino acids) (SEQ ID NO: 128)
> Human PLD4-Ig fusion protein cDNA (2142 bp) (SEQ ID NO: 125)
> Human PLD4-Ig fusion protein (713 amino acids) (SEQ ID NO: 126)

### ACCESSION NUMBERS

NITE ABP-1211
NITE ABP-1212
NITE ABP-1213
NITE ABP-1214

### SEQUENCE LISTING FREE TEXT

SEQ ID NO: 45: Forward primer
SEQ ID NO: 46: Reverse primer
SEQ ID NO: 47: Forward primer
SEQ ID NO: 48: Reverse primer
SEQ ID NO: 49: Forward primer
SEQ ID NO: 50: Reverse primer
SEQ ID NO: 51: Forward primer
SEQ ID NO: 52: Reverse primer
SEQ ID NO: 53: Forward primer
SEQ ID NO: 54: Reverse primer
SEQ ID NO: 70: Anchor primer
SEQ ID NO: 70: n is deoxyinosine
SEQ ID NO: 71: AUAP primer
SEQ ID NO: 72: Primer
SEQ ID NO: 73: Primer
SEQ ID NO: 114: Primer
SEQ ID NO: 115: Primer
SEQ ID NO: 116: Primer
SEQ ID NO: 117: Primer
SEQ ID NO: 118: Primer
SEQ ID NO: 119: Primer

## Claims

1. A monoclonal antibody or antibody fragment containing an antigen-binding region thereof that binds to phospholipase D4 (PLD4) protein for use as an activated B cell-suppressing medicament in preventing or treating an autoimmune disease caused by activated B cells or in preventing or treating an allergic disease.

2. The monoclonal antibody or antibody fragment containing the antigen-binding region thereof for use according to claim 1, comprising
(a) a heavy chain CDR1 set forth in SEQ ID NO: 2, a heavy chain CDR2 set forth in SEQ ID NO: 3, a heavy chain CDR3 set forth in SEQ ID NO: 4, a light chain CDR1 set forth in SEQ ID NO: 5, a light chain CDR2 as set forth in SEQ ID NO: 6, and a light chain CDR3 set forth in SEQ ID NO: 7;
(b) a heavy chain CDR1 set forth in SEQ ID NO: 8, a heavy chain CDR2 set forth in SEQ ID NO: 9, a heavy chain CDR3 set forth in SEQ ID NO: 10, a light chain CDR1 set forth in SEQ ID NO: 11, a light chain CDR2 set forth in SEQ ID NO: 12 and a light chain CDR3 set forth in SEQ ID NO: 13;
(c) a heavy chain CDR1 set forth in SEQ ID NO: 14, a heavy chain CDR2 set forth in SEQ ID NO: 15, a heavy chain CDR3 set forth in SEQ ID NO: 16, a light chain CDR1 set forth in SEQ ID NO: 17, a light chain CDR2 set forth in SEQ ID NO: 18 and a light chain CDR3 set forth in SEQ ID NO: 19;
(d) a heavy chain CDR1 set forth in SEQ ID NO: 20, a heavy chain CDR2 set forth in SEQ ID NO: 21, a heavy chain CDR3 set forth in SEQ ID NO: 22, a light chain CDR1 set forth in SEQ ID NO: 23, a light chain CDR2 set forth in SEQ ID NO: 24 and a light chain CDR3 set forth in SEQ ID NO: 25;
(e) a heavy chain CDR1 set forth in SEQ ID NO: 26, a heavy chain CDR2 set forth in SEQ ID NO: 27, a heavy chain CDR3 set forth in SEQ ID NO: 28, a light chain CDR1 set forth in SEQ ID NO: 29, a light chain CDR2 set forth in SEQ ID NO: 30 and a light chain CDR3 set forth in SEQ ID NO: 31;
(f) a heavy chain CDR1 set forth in SEQ ID NO: 32, a heavy chain CDR2 set forth in SEQ ID NO: 33, a heavy chain CDR3 set forth in SEQ ID NO: 34, a light chain CDR1 set forth in SEQ ID NO: 35, a light chain CDR2 set forth in SEQ ID NO: 36 and a light chain CDR3 set forth in SEQ ID NO: 37; or
(g) a heavy chain CDR1 set forth in SEQ ID NO: 38, a heavy chain CDR2 set forth in SEQ ID NO: 39, a heavy chain CDR3 set forth in SEQ ID NO: 40, a light chain CDR1 set forth in SEQ ID NO: 41, a light chain CDR2 set forth in SEQ ID NO: 42 and a light chain CDR3 set forth in SEQ ID NO: 43.

3. The monoclonal antibody or antibody fragment containing the antigen-binding region thereof for use according to claim 1, comprising
(a) a heavy chain variable region set forth in amino acids 36 to 153 of SEQ ID: 75 and a light chain variable region set forth in amino acids 21 to 127 of SEQ ID: 95;
(b) a heavy chain variable region set forth in amino acids 20 to 135 of SEQ ID: 77 and a light chain variable region set forth in amino acids 21 to 127 of SEQ ID: 97;
(c) a heavy chain variable region set forth in amino acids 20 to 142 of SEQ ID: 79 and a light chain variable region set forth in amino acids 21 to 127 of SEQ ID: 99;
(d) a heavy chain variable region set forth in amino acids 20 to 142 of SEQ ID: 81 and a light chain variable region set forth in amino acids 21 to 127 of SEQ ID: 101;
(e) a heavy chain variable region set forth in amino acids 20 to 139 of SEQ ID: 83 and a light chain variable region set forth in amino acids 20 to 131 of SEQ ID: 103;
(f) a heavy chain variable region set forth in amino acids 20 to 140 of SEQ ID: 85 and a light chain variable region set forth in amino acids 21 to 131 of SEQ ID: 105;
(g) a heavy chain variable region set forth in amino acids 20 to 140 of SEQ ID: 87 and a light chain variable region set forth in amino acids 21 to 131 of SEQ ID: 107;
(h) a heavy chain variable region set forth in amino acids 19 to 142 of SEQ ID: 89 and a light chain variable region set forth in amino acids 21 to 127 of SEQ ID: 109;
(i) a heavy chain variable region set forth in amino acids 19 to 142 of SEQ ID: 91 and a light chain variable region set forth in amino acids 21 to 127 of SEQ ID: 111; or
(j) a heavy chain variable region set forth in amino acids 19 to 142 of SEQ ID: 93 and a light chain variable region set forth in amino acids 21 to 127 of SEQ ID: 113.

4. A monoclonal antibody or antibody fragment containing an antigen-binding region thereof for use according to claim 1, wherein the antibody is produced by any one of hybridomas mp5B7, mp7B4, mp13D4 and mp13H11 of Deposit Nos. NITE BP-1211, NITE BP-1212, NITE BP-1213 and NITE BP-1214, or antibody fragment containing an antigen-binding region thereof for use as an activated B cell-suppressing medicament.

5. The monoclonal antibody or antibody fragment containing the antigen-binding region thereof for use according to claim 1, which is chimerized or humanized.

6. The monoclonal antibody or antibody fragment containing the antigen-binding region thereof for use according to claim 1 comprising a heavy chain set forth in SEQ ID: 121 and a light chain set forth in SEQ ID: 123.

7. An *in vitro* method for detecting activated B cells, the method including
a) a step of bringing a monoclonal antibody binding to an extracellular domain of PLD4 or an antibody fragment containing an antigen-binding region thereof into contact with cells to be tested; and
b) a step of detecting the monoclonal antibody or the antibody fragment containing the antigen-binding region thereof which binds to the cells.

8. Use of a monoclonal antibody binding to an extracellular domain of human PLD4 or an antibody fragment containing an antigen-binding region thereof to detect activated B cells *in vitro.*

9. An *in vitro* method for suppressing an activity of activated B cells, the method including a step of bringing either of the following components into contact with activated B cells:
(a) a monoclonal antibody or an antibody fragment containing an antigen-binding region thereof which binds to PLD4 and suppresses the activity of activated B cells or has cytotoxic activity against activated B cells, and
(b) immunoglobulin into which the complementarity-determining regions of the monoclonal antibody in (a) are grafted, or an antibody fragment containing an antigen-binding region thereof, wherein the activity of the activated B cells is antibody production or maturation into antibody-secreting cells.

10. A monoclonal antibody or antibody fragment containing an antigen-binding region thereof or an immunoglobulin for use as an activated B cell-suppressing medicament in preventing or treating an autoimmune disease caused by activated B cells or in preventing or treating an allergic disease, wherein:
(a) the monoclonal antibody or antibody fragment containing an antigen-binding region thereof binds to PLD4 and suppresses an activity of activated B cells or has cytotoxic activity against activated B cells, or
(b) the immunoglobulin is an immunoglobulin into which the complementarity-determining regions of the monoclonal antibody in (a) are grafted, or an antibody fragment containing an antigen-binding region thereof, wherein the activity of the activated B cells is antibody production or maturation into antibody-secreting cells.

## Patentansprüche

1. Monoklonaler Antikörper oder Antikörperfragment, das eine antigenbindende Region davon enthält, die an ein Phospholipase-D4-(PLD4)-Protein bindet, zur Verwendung als ein aktivierte B-Zellen unterdrückendes Medikament beim Vorbeugen oder Behandeln einer Autoimmunkrankheit, die durch aktivierte B-Zellen verursacht wird, oder beim Vorbeugen oder Behandeln einer allergischen Krankheit.

2. Monoklonaler Antikörper oder Antikörperfragment, das die antigenbindende Region davon enthält, zur Verwendung nach Anspruch 1, umfassend
a) eine schwere Kette CDR1, die in SEQ ID NO: 2 dargestellt ist, eine schwere Kette CDR2, die in SEQ ID NO: 3 dargestellt ist, eine schwere Kette CDR3, die in SEQ ID NO: 4 dargestellt ist, eine leichte Kette CDR1, die in SEQ ID NO: 5 dargestellt ist, eine leichte Kette CDR2, die in SEQ ID NO: 6 dargestellt ist, und eine leichte Kette CDR3, die in SEQ ID NO: 7 dargestellt ist;
b) eine schwere Kette CDR1, die in SEQ ID NO: 8 dargestellt ist, eine schwere Kette CDR2, die in SEQ ID NO: 9 dargestellt ist, eine schwere Kette CDR3, die in SEQ ID NO: 10 dargestellt ist, eine leichte Kette CDR1, die in SEQ ID NO: 11 dargestellt ist, eine leichte Kette CDR2, die in SEQ ID NO: 12 dargestellt ist, und eine leichte Kette CDR3, die in SEQ ID NO: 13 dargestellt ist;
c) eine schwere Kette CDR1, die in SEQ ID NO: 14 dargestellt ist, eine schwere Kette CDR2, die in SEQ ID NO: 15 dargestellt ist, eine schwere Kette CDR3, die in SEQ ID NO: 16 dargestellt ist, eine leichte Kette CDR1, die in SEQ ID NO: 17 dargestellt ist, eine leichte Kette CDR2, die in SEQ ID NO: 18 dargestellt ist, und eine leichte Kette CDR3, die in SEQ ID NO: 19 dargestellt ist;
d) eine schwere Kette CDR1, die in SEQ ID NO: 20 dargestellt ist, eine schwere Kette CDR2, die in SEQ ID NO: 21 dargestellt ist, eine schwere Kette CDR3, die in SEQ ID NO: 22 dargestellt ist, eine leichte Kette CDR1, die in SEQ ID NO: 23 dargestellt ist, eine leichte Kette CDR2, die in SEQ ID NO: 24 dargestellt ist, und eine leichte Kette CDR3, die in SEQ ID NO: 25 dargestellt ist;
e) eine schwere Kette CDR1, die in SEQ ID NO: 26 dargestellt ist, eine schwere Kette CDR2, die in SEQ ID NO: 27 dargestellt ist, eine schwere Kette CDR3, die in SEQ ID NO: 28 dargestellt ist, eine leichte Kette CDR1, die in SEQ ID NO: 29 dargestellt ist, eine leichte Kette CDR2, die in SEQ ID NO: 30 dargestellt ist, und eine leichte Kette CDR3, die in SEQ ID NO: 31 dargestellt ist;
f) eine schwere Kette CDR1, die in SEQ ID NO: 32 dargestellt ist, eine schwere Kette CDR2, die in SEQ ID NO: 33 dargestellt ist, eine schwere Kette CDR3, die in SEQ ID NO: 34 dargestellt ist, eine leichte Kette CDR1, die in SEQ ID NO: 35 dargestellt ist, eine leichte Kette CDR2, die in SEQ ID NO: 36 dargestellt ist, und eine leichte Kette CDR3, die in SEQ ID NO: 37 dargestellt ist; oder
g) eine schwere Kette CDR1, die in SEQ ID NO: 38 dargestellt ist, eine schwere Kette CDR2, die in SEQ ID NO: 39 dargestellt ist, eine schwere Kette CDR3, die in SEQ ID NO: 40 dargestellt ist, eine leichte Kette CDR1, die in SEQ ID NO: 41 dargestellt ist, eine leichte Kette CDR2, die in SEQ ID NO: 42 dargestellt ist, und eine leichte Kette CDR3, die in SEQ ID NO: 43 dargestellt ist.

3. Monoklonaler Antikörper oder Antikörperfragment, das die antigenbindende Region davon enthält, zur Verwendung nach Anspruch 1, umfassend
a) eine variable Region der schweren Kette, die in Aminosäuren 36 bis 153 von SEQ ID: 75 dargestellt ist, und eine variable Region der leichten Kette, die in Aminosäuren 21 bis 127 von SEQ ID: 95 dargestellt ist;
b) eine variable Region der schweren Kette, die in Aminosäuren 20 bis 135 von SEQ ID: 77 dargestellt ist, und eine variable Region der leichten Kette, die in Aminosäuren 21 bis 127 von SEQ ID: 97 dargestellt ist;
c) eine variable Region der schweren Kette, die in Aminosäuren 20 bis 142 von SEQ ID: 79 dargestellt ist, und eine variable Region der leichten Kette, die in Aminosäuren 21 bis 127 von SEQ ID: 99 dargestellt ist;
d) eine variable Region der schweren Kette, die in Aminosäuren 20 bis 142 von SEQ ID: 81 dargestellt ist, und eine variable Region der leichten Kette, die in Aminosäuren 21 bis 127 von SEQ ID: 101 dargestellt ist;
e) eine variable Region der schweren Kette, die in Aminosäuren 20 bis 139 von SEQ ID: 83 dargestellt ist, und eine variable Region der leichten Kette, die in Aminosäuren 20 bis 131 von SEQ ID: 103 dargestellt ist;
f) eine variable Region der schweren Kette, die in Aminosäuren 20 bis 140 von SEQ ID: 85 dargestellt ist, und eine variable Region der leichten Kette, die in Aminosäuren 21 bis 131 von SEQ ID: 105 dargestellt ist;
g) eine variable Region der schweren Kette, die in Aminosäuren 20 bis 140 von SEQ ID: 87 dargestellt ist, und eine variable Region der leichten Kette, die in Aminosäuren 21 bis 131 von SEQ ID: 107 dargestellt ist;
h) eine variable Region der schweren Kette, die in Aminosäuren 19 bis 142 von SEQ ID: 89 dargestellt ist, und eine variable Region der leichten Kette, die in Aminosäuren 21 bis 127 von SEQ ID: 109 dargestellt ist;
i) eine variable Region der schweren Kette, die in Aminosäuren 19 bis 142 von SEQ ID: 91 dargestellt ist, und eine variable Region der leichten Kette, die in Aminosäuren 21 bis 127 von SEQ ID: 111 dargestellt ist; oder
j) eine variable Region der schweren Kette, die in Aminosäuren 19 bis 142 von SEQ ID: 93 dargestellt ist, und eine variable Region der leichten Kette, die in Aminosäuren 21 bis 127 von SEQ ID: 113 dargestellt ist.

4. Monoklonaler Antikörper oder Antikörperfragment, das eine antigenbindende Region davon enthält, zur Verwendung nach Anspruch 1, wobei der Antikörper von einem von Hybridomen mp5B7, mp7B4, mp13D4 und mp13H11 von Hinterlegungsnummern NITE BP-1211, NITE BP-1212, NITE BP-1213 und NITE BP-1214 hergestellt ist, oder ein Antikörperfragment, das eine antigenbindende Region davon enthält, zur Verwendung als ein aktivierte B-Zellen unterdrückendes Medikament.

5. Monoklonaler Antikörper oder Antikörperfragment, das die antigenbindende Region davon enthält, zur Verwendung nach Anspruch 1, der/das chimärisiert oder humanisiert ist.

6. Monoklonaler Antikörper oder Antikörperfragment, das die antigenbindende Region davon enthält, zur Verwendung nach Anspruch 1, umfassend eine schwere Kette, die in SEQ ID: 121 dargestellt ist, und eine leichte Kette, die in SEQ ID: 123 dargestellt ist.

7. In-vitro-Verfahren zum Erfassen von aktivierten B-Zellen, wobei das Verfahren Folgendes enthält
a) einen Schritt des Inkontaktbringens eines monoklonalen Antikörpers, der an eine extrazelluläre Domäne von PLD4 bindet, oder eines Antikörperfragments, das eine antigenbindende Region davon enthält, mit zu testenden Zellen; und
b) einen Schritt des Erfassens des monoklonalen Antikörpers oder des Antikörperfragments, das die antigenbindende Region davon enthält, der/das an die Zellen bindet.

8. Verwendung eines monoklonalen Antikörpers, der an eine extrazelluläre Domäne von menschlichem PLD4 bindet, oder eines Antikörperfragments, das eine antigenbindende Region davon enthält, um aktivierte B-Zellen in vitro zu erfassen.

9. In-vitro-Verfahren zum Unterdrücken einer Aktivität von aktivierten B-Zellen, wobei das Verfahren einen Schritt des Inkontaktbringens von einer der folgenden Komponenten mit aktivierten B-Zellen enthält:
a) einem monoklonalen Antikörper oder einem Antikörperfragment, das eine antigenbindende Region davon enthält, der/das an PLD4 bindet und die Aktivität von aktivierten B-Zellen unterdrückt oder zytotoxische Aktivität gegen aktivierte B-Zellen aufweist, und
b) Immunglobulin, in das die komplementaritätsbestimmenden Regionen des monoklonalen Antikörpers in (a) transplantiert sind, oder einem Antikörperfragment, das eine antigenbindende Region davon enthält, wobei die Aktivität der aktivierten B-Zellen Antikörperproduktion oder Reifung zu antikörpersezernierenden Zellen ist.

10. Monoklonaler Antikörper oder Antikörperfragment, das eine antigenbindende Region davon enthält, oder Immunglobulin zur Verwendung als ein aktivierte B-Zellen unterdrückendes Medikament beim Vorbeugen oder Behandeln einer Autoimmunkrankheit, die durch aktivierte B-Zellen verursacht wird, oder beim Vorbeugen oder Behandeln einer allergischen Krankheit, wobei:
(a) der monoklonale Antikörper oder das Antikörperfragment, das eine antigenbindende Region davon enthält, an PLD4 bindet und eine Aktivität von aktivierten B-Zellen unterdrückt oder zytotoxische Aktivität gegen aktivierte B-Zellen aufweist oder
(b) das Immunglobulin ein Immunglobulin ist, in das die komplementaritätsbestimmenden Regionen des monoklonalen Antikörpers in (a) transplantiert sind, oder ein Antikörperfragment, das eine antigenbindende Region davon enthält, wobei die Aktivität der aktivierten B-Zellen Antikörperproduktion oder Reifung zu antikörpersezernierenden Zellen ist.

## Revendications

1. Anticorps monoclonal ou fragment d'anticorps contenant une région de celui-ci de liaison à un antigène qui se lie à une protéine de phospholipase D4 (PLD4) en vue d'une utilisation en tant que médicament de suppression de cellules B activées dans la prévention ou le traitement d'une maladie auto-immune causée par des cellules B activées ou dans la prévention ou le traitement d'une maladie allergique.

2. Anticorps monoclonal ou fragment d'anticorps contenant la région de celui-ci de liaison à un antigène en vue d'une utilisation selon la revendication 1, comprenant :
(a) une CDR1 de chaîne lourde indiquée dans la SEQ ID n° : 2, une CDR2 de chaîne lourde indiquée dans la SEQ ID n° : 3, une CDR3 de chaîne lourde indiquée dans la SEQ ID n° : 4, une CDR1 de chaîne légère indiquée dans la SEQ ID n° : 5, une CDR2 de chaîne légère indiquée dans la SEQ ID n° : 6 et une CDR3 de chaîne légère indiquée dans la SEQ ID n° : 7 ;
(b) une CDR1 de chaîne lourde indiquée dans la SEQ ID n° : 8, une CDR2 de chaîne lourde indiquée dans la SEQ ID n° : 9, une CDR3 de chaîne lourde indiquée dans la SEQ ID n° : 10, une CDR1 de chaîne légère indiquée dans la SEQ ID n° : 11, une CDR2 de chaîne légère indiquée dans la SEQ ID n° : 12 et une CDR3 de chaîne légère indiquée dans la SEQ ID n° : 13 ;
(c) une CDR1 de chaîne lourde indiquée dans la SEQ ID n° : 14, une CDR2 de chaîne lourde indiquée dans la SEQ ID n° : 15, une CDR3 de chaîne lourde indiquée dans la SEQ ID n° : 16, une CDR1 de chaîne légère indiquée dans la SEQ ID n° : 17, une CDR2 de chaîne légère indiquée dans la SEQ ID n° : 18 et une CDR3 de chaîne légère indiquée dans la SEQ ID n° : 19 ;
(d) une CDR1 de chaîne lourde indiquée dans la SEQ ID n° : 20, une CDR2 de chaîne lourde indiquée dans la SEQ ID n° : 21, une CDR3 de chaîne lourde indiquée dans la SEQ ID n° : 22, une CDR1 de chaîne légère indiquée dans la SEQ ID n° : 23, une CDR2 de chaîne légère indiquée dans la SEQ ID n° : 24 et une CDR3 de chaîne légère indiquée dans la SEQ ID n° : 25 ;
(e) une CDR1 de chaîne lourde indiquée dans la SEQ ID n° : 26, une CDR2 de chaîne lourde indiquée dans la SEQ ID n° : 27, une CDR3 de chaîne lourde indiquée dans la SEQ ID n° : 28, une CDR1 de chaîne légère indiquée dans la SEQ ID n° : 29, une CDR2 de chaîne légère indiquée dans la SEQ ID n° : 30 et une CDR3 de chaîne légère indiquée dans la SEQ ID n° : 31 ;
(f) une CDR1 de chaîne lourde indiquée dans la SEQ ID n° : 32, une CDR2 de chaîne lourde indiquée dans la SEQ ID n° : 33, une CDR3 de chaîne lourde indiquée dans la SEQ ID n° : 34, une CDR1 de chaîne légère indiquée dans la SEQ ID n° : 35, une CDR2 de chaîne légère indiquée dans la SEQ ID n° : 36 et une CDR3 de chaîne légère indiquée dans la SEQ ID n° : 37 ; ou
(g) une CDR1 de chaîne lourde indiquée dans la SEQ ID n° : 38, une CDR2 de chaîne lourde indiquée dans la SEQ ID n° : 39, une CDR3 de chaîne lourde indiquée dans la SEQ ID n° : 40, une CDR1 de chaîne légère indiquée dans la SEQ ID n° : 41, une CDR2 de chaîne légère indiquée dans la SEQ ID n° : 42 et une CDR3 de chaîne légère indiquée dans la SEQ ID n° : 43.

3. Anticorps monoclonal ou fragment d'anticorps contenant la région de celui-ci de liaison à un antigène en vue d'une utilisation selon la revendication 1, comprenant :
(a) une région variable de chaîne lourde indiquée dans les acides aminés 36 à 153 de la SEQ ID : 75 et une région variable de chaîne légère indiquée dans les acides aminés 21 à 127 de la SEQ ID : 95 ;
(b) une région variable de chaîne lourde indiquée dans les acides aminés 20 à 135 de la SEQ ID : 77 et une région variable de chaîne légère indiquée dans les acides aminés 21 à 127 de la SEQ ID : 97 ;
(c) une région variable de chaîne lourde indiquée dans les acides aminés 20 à 142 de la SEQ ID : 79 et une région variable de chaîne légère indiquée dans les acides aminés 21 à 127 de la SEQ ID : 99 ;
(d) une région variable de chaîne lourde indiquée dans les acides aminés 20 à 142 de la SEQ ID : 81 et une région variable de chaîne légère indiquée dans les acides aminés 21 à 127 de la SEQ ID : 101 ;
(e) une région variable de chaîne lourde indiquée dans les acides aminés 20 à 139 de la SEQ ID : 83 et une région variable de chaîne légère indiquée dans les acides aminés 20 à 131 de la SEQ ID : 103 ;
(f) une région variable de chaîne lourde indiquée dans les acides aminés 20 à 140 de la SEQ ID : 85 et une région variable de chaîne légère indiquée dans les acides aminés 21 à 131 de la SEQ ID : 105 ;
(g) une région variable de chaîne lourde indiquée dans les acides aminés 20 à 140 de la SEQ ID : 87 et une région variable de chaîne légère indiquée dans les acides aminés 21 à 131 de la SEQ ID : 107 ;
(h) une région variable de chaîne lourde indiquée dans les acides aminés 19 à 142 de la SEQ ID : 89 et une région variable de chaîne légère indiquée dans les acides aminés 21 à 127 de la SEQ ID : 109 ;
(i) une région variable de chaîne lourde indiquée dans les acides aminés 19 à 142 de la SEQ ID : 91 et une région variable de chaîne légère indiquée dans les acides aminés 21 à 127 de la SEQ ID : 111 ; ou
(i) une région variable de chaîne lourde indiquée dans les acides aminés 19 à 142 de la SEQ ID : 93 et une région variable de chaîne légère indiquée dans les acides aminés 21 à 127 de la SEQ ID : 113.

4. Anticorps monoclonal ou fragment d'anticorps contenant une région de celui-ci de liaison à un antigène en vue d'une utilisation selon la revendication 1, l'anticorps étant produit par l'un quelconque des hybridomes mp5B7, mp7B4, mp13D4 et mp13H11 des numéros de dépôt NITE BP-1211, NITE BP-1212, NITE BP-1213 et NITE BP-1214, ou le fragment d'anticorps contenant une région de celui-ci de liaison à un antigène en vue d'une utilisation en tant que médicament de suppression de cellules B activées.

5. Anticorps monoclonal ou fragment d'anticorps contenant la région de celui-ci de liaison à un antigène en vue d'une utilisation selon la revendication 1, qui est chimérisé ou humanisé.

6. Anticorps monoclonal ou fragment d'anticorps contenant la région de celui-ci de liaison à un antigène en vue d'une utilisation selon la revendication 1, comprenant une chaîne lourde indiquée dans la SEQ ID : 121 et une chaîne légère indiquée dans la SEQ ID : 123.

7. Procédé *in vitro* de détection de cellules B activées, le procédé comprenant
a) une étape de mise en contact d'un anticorps monoclonal se liant à un domaine extracellulaire de PLD4 ou d'un fragment d'anticorps contenant une région de celui-ci de liaison à un antigène avec des cellules à tester ; et
b) une étape de détection de l'anticorps monoclonal ou du fragment d'anticorps contenant la région de celui-ci de liaison à un antigène qui se lie aux cellules.

8. Utilisation d'un anticorps monoclonal se liant à un domaine extracellulaire de PLD4 humain ou d'un fragment d'anticorps contenant une région de celui-ci de liaison à un antigène pour détecter des cellules B activées *in vitro.*

9. Procédé *in vitro* de suppression d'une activité de cellules B activées, le procédé comprenant une étape de mise en contact d'un des composants suivants avec des cellules B activées :
(a) un anticorps monoclonal ou un fragment d'anticorps contenant une région de celui-ci de liaison à un antigène qui se lie à PLD4 et supprime l'activité de cellules B activées ou présente une activité cytotoxique contre des cellules B activées, et
(b) une immunoglobuline dans laquelle les régions de détermination de complémentarité de l'anticorps monoclonal dans (a) sont greffées, ou un fragment d'anticorps contenant une région de celui-ci de liaison à un antigène, l'activité des cellules B activées étant une production d'anticorps ou une maturation en cellules sécrétant des anticorps.

10. Anticorps monoclonal ou fragment d'anticorps contenant une région de celui-ci de liaison à un antigène ou une immunoglobuline en vue d'une utilisation en tant que médicament de suppression de cellules B activées dans la prévention ou le traitement d'une maladie auto-immune causée par des cellules B activées ou dans la prévention ou le traitement d'une maladie allergique,
(a) l'anticorps monoclonal ou le fragment d'anticorps contenant une région de celui-ci de liaison à un antigène se lie à PLD4 et supprime une activité de cellules B activées ou présente une activité cytotoxique contre des cellules B activées, ou
(b) l'immunoglobuline est une immunoglobuline dans laquelle les régions de détermination de complémentarité de l'anticorps monoclonal dans (a) sont greffées, ou un fragment d'anticorps contenant une région de celui-ci de liaison à un antigène, l'activité des cellules B activées étant une production d'anticorps ou une maturation en cellules sécrétant des anticorps.
